# EUROPEAN PATENT SPECIFICATION

(11) **EP 2 024 514 B1**
(45) Date of publication and mention of the grant of the patent: **27.02.2013**
(21) Application number: 07795008.7
(22) Date of filing: 16.05.2007
(51) Int. Cl.: C12Q 1/68

(54) **ASSESSMENT OF EFFECT OF AN AGENT ON A HUMAN BIOLOGICAL CONDITION USING RODENT GENE EXPRESSION PANELS**
BEURTEILUNG DER WIRKUNG EINES WIRKSTOFFES AUF EIN MENSCHLICHES BIOLOGISCHES LEIDEN MITTELS GENEXPRESSIONSPANELS AUS NAGERN
EVALUATION DES EFFETS D'UN AGENT SUR L'ETAT BIOLOGIQUE DE L'HOMME A L'AIDE DE PANNEAUX D'EXPRESSION GENIQUE DE RONGEURS

(30) Priority: 16.05.2006 US 800802 P
(43) Date of publication of application: 18.02.2009
(73) Proprietor: DxTerity Diagnostics Inc., Rancho Dominguez, CA 90220 (US)
(72) Inventor: BEVILACQUA, Michael, Boulder, CO 80301 (US); TRYON, Victor, Woodinville, WA 98072 (US); CHERONIS, John, Conifer, CO 80433 (US); BANKAITIS-DAVIS, Danute, Longmont, CO 80503 (US); STORM, Kathleen, Longmont, CO 80503 (US); WASSMAN, Karl, Dover, MA 02030 (US)
(74) Representative: Finnie, Isobel Lara
(86) International application number: PCT/US2007/011873
(87) International publication number: WO 2007/136728

(56) References cited:
- WO-A-01/25473
- WO-A-03/040404
- WO-A-03/072827
- WO-A-2004/057034
- WO-A-2004/110244
- OVERALL CHRISTOPHER M ET AL: "Tumour microenvironment - Opinion - Validating matrix metalloproteinases as drug targets and anti-targets for cancer therapy" NATURE REVIEWS CANCER, vol. 6, no. 3, March 2006 (2006-03), pages 227-239, XP002457279 ISSN: 1474-175X
- VELDE ANJE TE ET AL: "Overlapping gene expression profiles of experimental colitis and IBD." GASTROENTEROLOGY, vol. 130, no. 4, Suppl. 2, April 2006 (2006-04), pages A112-A113, XP002457280 & DIGESTIVE DISEASE WEEK MEETING/107TH ANNUAL MEETING OF THE AMERICAN-GASTROENTEROLOGICAL-ASSOCIATION; LOS ANGELES, CA, USA; MAY 19 24, 2006 ISSN: 0016-5085
- BOVIN L F ET AL: "Blood cell gene expression profiling in rheumatoid arthritis - Discriminative genes and effect of rheumatoid factor" IMMUNOLOGY LETTERS, AMSTERDAM, NL, vol. 93, no. 2-3, 15 May 2004 (2004-05-15), pages 217-226, XP002419834 ISSN: 0165-2478
- FUJIKADO NORIYUKI ET AL: "Identification of arthritis-related gene clusters by microarray analysis of two independent mouse models for rheumatoid arthritis." ARTHRITIS RESEARCH & THERAPY 2006, vol. 8, no. 4, 2006, page R100, XP002457281 ISSN: 1478-6362

## Description

### FIELD OF THE INVENTION

The present invention relates to use of gene expression data, and in particular to use of gene expression data in assessing the effect of an agent on a human biological condition using rodent Gene Expression Panels.

### BACKGROUND OF THE INVENTION

The prior art has utilized gene expression data to determine the presence or absence of particular markers as diagnostic of a particular condition, and in some circumstances have described the cumulative addition of scores for over-expression of particular disease markers to achieve increased accuracy or sensitivity of diagnosis. Information on any condition of a particular patient and a patient's response to different types and dosages of therapeutic or nutritional agents has become an important issue in clinical medicine today, not only from the aspect of efficiency of medical practice for the health care industry, but for improved outcomes and benefits for patients.

Animal models that simulate biological conditions in humans are often used to test the efficacy of new therapeutic agents. However, many agents fail animal testing for unknown reasons, or may only treat and/or mask the symptoms in such animal models, rather than the underlying biological condition. Thus, an improved method of using animal models to predict human response to a therapeutic agent, or dosage thereof, at the molecular level, would be beneficial. This invention meets these needs and other needs.

WO 03/072827 describes a method for diagnosis and treatment of rheumatoid arthritis by analysing the expressions cDNAs during various stages of mouse collagen induced arthritis.

Overall and Kleifeld (March 2006) Nature Reviews Cancer, Volume 6, pages 227 to 239 describes validating matrix metalloproteinases as drug targets and anti-targets for anti-cancer therapy.

### SUMMARY OF THE INVENTION

An embodiment of the present invention are directed to a method of identifying a rodent Signature gene expression panel for use in assessment of an agent on a human biological condition of interest. In this embodiment, the method includes identifying a Gene Expression Panel for humans with respect to which constituent expression levels are indicative of the biological condition of interest. The embodiment also includes thereafter assessing in a rodent population the constituent genes of the identified Gene Expression Panel to determine which constituents are indicative of the biological condition of interest in both humans and rodents wherein a set of constituents thus determined to be indicative constitutes the Signature panel. In some embodiments, the Signature gene expression panel identified comprises a plurality of constituents from any of Tables 1-9, described below.

Another embodiment of the invention provides a method for assessing the effect of an agent on a human biological condition of interest, based on a sample from a rodent subject to which the agent has been administered. In this embodiment, the sample provides a source of RNAs, and the embodiment includes determining a Signature Panel for rodents. The constituents of the Signature Panel correspond to constituents of a human gene expression panel, wherein measurement of the constituents of the Signature Panel enables measurement of the biological condition of the rodent subject, and measurement of the constituents of the human panel enables measurement of the human biological condition. The embodiment also includes deriving from the rodent sample a first profile data set including a plurality of members, each member being a quantitative measure of the amount of a distinct RNA constituent in the Signature Panel. Finally the embodiment includes producing a calibrated profile data set for the Signature Panel, wherein each member of the calibrated profile data set is a function of a corresponding member of the first profile data set and a corresponding member of a rodent baseline profile data set for the Signature Panel, wherein each member of the rodent baseline data set is a normative measure, determined with respect to a relevant population of rodents, of the amount of one of the constituents in the Signature Panel, the calibrated profile data set providing an assessment of the effect of the agent on the human biological condition, wherein the measures for each constituent are performed under measurement conditions that are substantially repeatable. The measurement conditions are repeatable such that measure for each constituent has a coefficient of variation, on repeated derivation of such measure from the sample, that is less than approximately 10%, preferably less than approximately 5%, more preferably less than approximately 2%.

In one embodiment of the invention, the human biological condition to be assessed is a form of arthritis, including without limitation, rheumatoid arthritis.

In a preferred embodiment the method for assessing the effect of an agent on a human biological condition of interest is performed using amplification to measure the amount of RNA of all of the constituents of the Signature Panel, and the efficiencies of amplification (expressed as a percent) for all constituents are substantially similar. In a preferred embodiment, the efficiencies of amplification for all constituents are substantially similar if they differ by no more than 10%, preferably no more than 5%, more preferably no more than 3%, even more preferably no more than 1%.

In one embodiment, the Signature Panel used in the method for assessing the effect of an agent on a human biological condition comprises a plurality of constituents from any of Tables 1-9, described below. In other embodiments, the Signature Panel used in the method for assessing the effect of an agent on a human biological condition comprises a plurality of constituents selected from the group consisting of CASP3, CD14, CSPG2, HSPA1A, ICAM1, IL1B, IL1RN, MEF2C, MMP9, SERPINE1, TGFB1, and TLR2.

In further related embodiments, the invention provides a rodent Signature Gene Expression Panel (Signature Panel) comprising the constituents CASP3, CD14, CSPG2, HSPA1A, ICAM1, IL1B, IL1RN, MEF2C, MMP9, SERPINE1, TGFB1, and TLR2. Another embodiment of the invention provides a rodent Signature Gene Expression Panel (Signature Panel) comprising a plurality of constituents from any of Tables 1-9, described below, or from any specific one of Tables 1-9.

### BRIEF DESCRIPTION OF THE DRAWINGS

The foregoing features of the invention will be more readily understood by reference to the following detailed description, taken with reference to the accompanying drawings, in which:
Figures 1A-1C show LPS-stimulated whole blood response at 1.5 hr (Group 1), 4 hr (group 2) and 24 hr (Group 3), respectively, in three groups of male Swiss Webster mice for a 24-gene panel.
Figures 2A-2C show LPS + Dexamethasone-stimulated whole blood response at 1.5 hr (Group 4), 4 hr (Group 5) and 24 hr (Group 6), respectively, in three groups of male Swiss Webster mice for a 24-gene panel.
Figure 3 shows LPS-stimulated whole blood response at 1.5 hr, 4 hr and 24 hr, respectively, as an average for groups 1, 2 and 3, of male Swiss Webster mice for a 24-gene panel.
Figure 4 shows LPS-stimulated whole blood response at 1.5 hr, 4 hr and 24 hr, respectively, as an average for groups 4, 5 and 6, of male Swiss Webster mice for a 24-gene panel.
Figures 5A - 5C show a comparison of LPS-stimulated whole blood response in human and murine subjects *in vivo* at 2 and 1.5 hr, respectively, for 17 genes.
Figures 6A - 6C show a comparison of LPS-stimulated whole blood response in human subjects *in vitro* and *in vivo* at 2 and 1.5 hr, for 38 genes.
Figures 7A - 7C show a comparison of LPS-stimulated whole blood response in human (*in vitro*) and murine (*in vivo*) subjects at 2 and 1.5 hr, respectively, for the same 17 genes in Figs. 5A - 5C.
Figures 8A - 8C show a comparison of Dexamethasone Response in LPS-stimulated whole blood response in human (*in vitro*) and murine (in vivo) subjects at 2 and 1.5 hr, respectively, for the same 17 genes in Figs. 5A - 5C and 7A-7C.
Figures 9A- 9C show a comparison of the gene expression responses of individual naïve murine ("normal") subjects at day 60 relative to averaged responses at Day 0 (Baseline animals 1-6) in a CIA study using male DBA/1 mice. Gene expression analysis was performed by QPCR using a custom murine 40-gene panel (Precision Profile^{™}) for Rheumatoid Arthritis.
Figures 10A-10C show a comparison of the gene expression responses of individual naïve murine ("normal") subjects at day 21 relative to averaged responses at Day 0 (Naive animals 1-6) in a KRN study using female BALB/c mice. Gene expression analysis was performed using a custom murine 40-gene panel (Precision Profile^{™}) for Rheumatoid Arthritis.

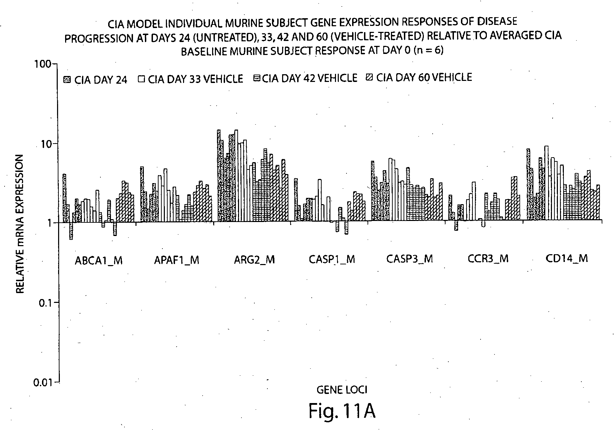

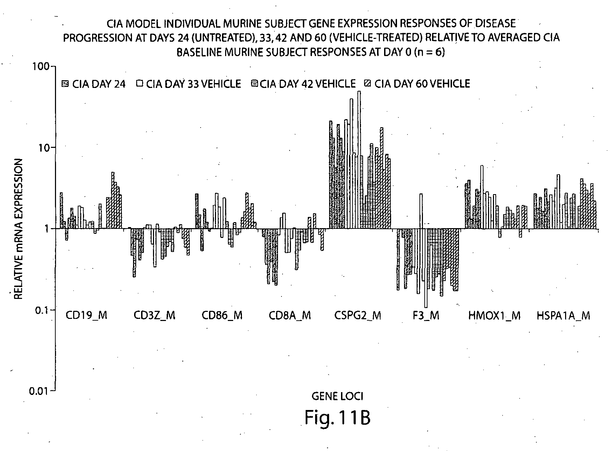

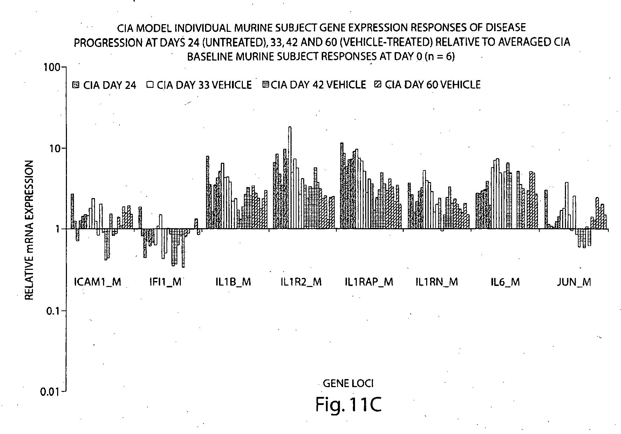

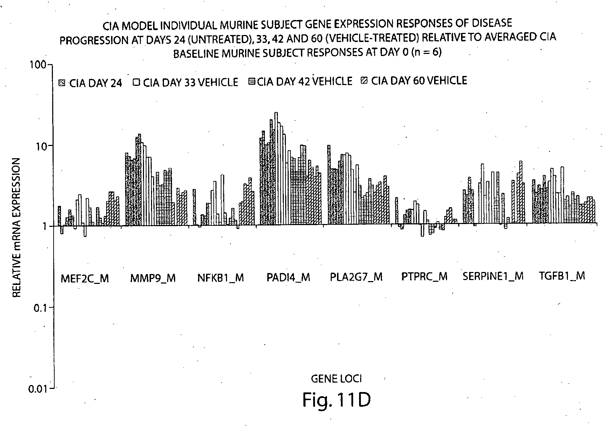

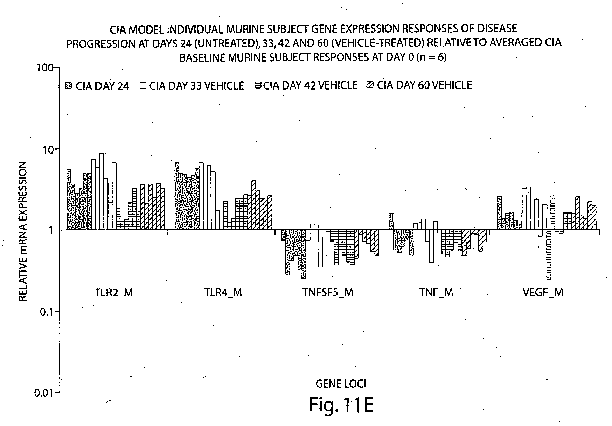

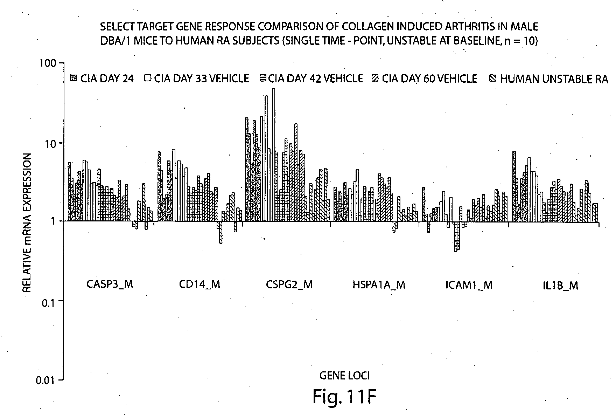

Figures 11A-11E show a comparison of individual murine subject gene expression responses of disease progression at days 24 (untreated), and days 33, 42 and 60 (vehicle-treated), relative to averaged baseline naïve murine subject response at day 0 (n=6) in a CIA study using male DBA/1 mice. Gene expression analysis was performed using a custom murine 40-gene panel (Precision Profile^{™}) for Rheumatoid Arthritis; Figures 11F-11G show a comparison of select target gene responses in Collagen Induced Arthritis in Male DBA/1 Mice to Human RA Subjects (single time-point, unstable at baseline, n=10).

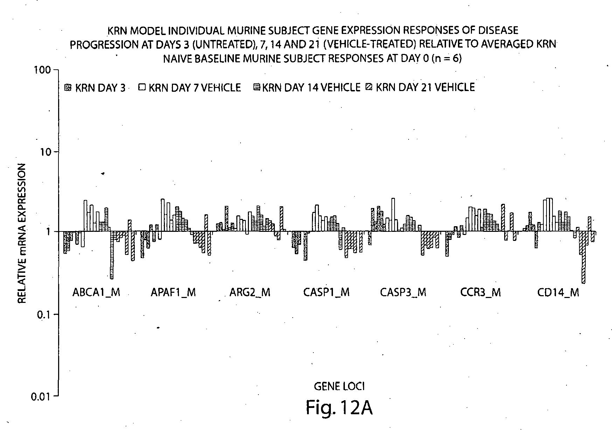

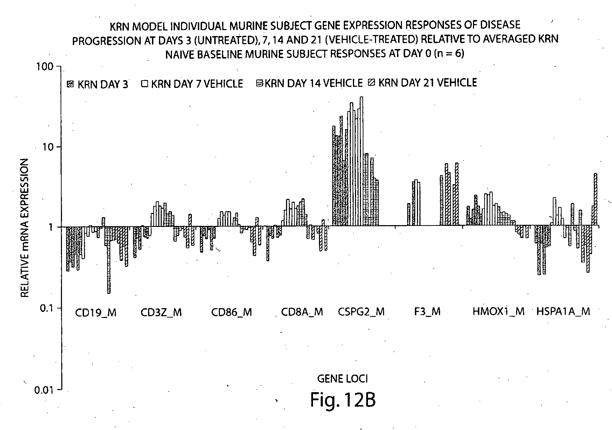

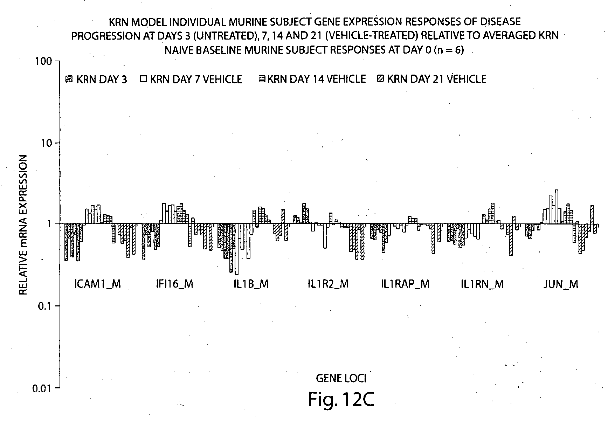

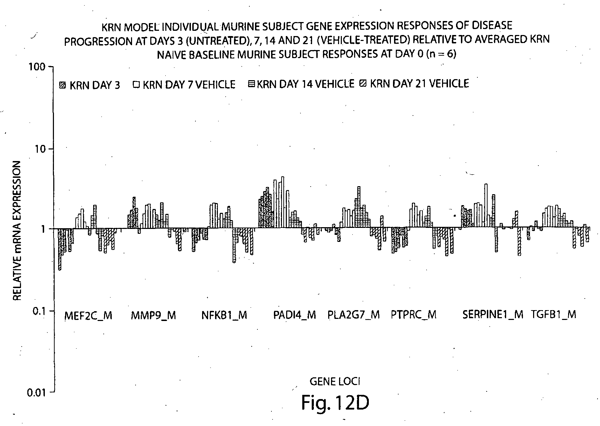

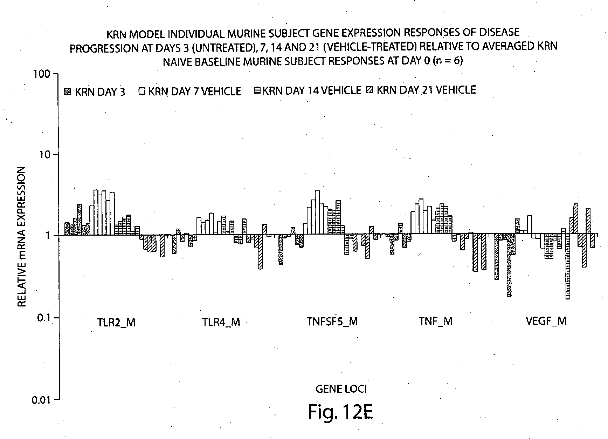

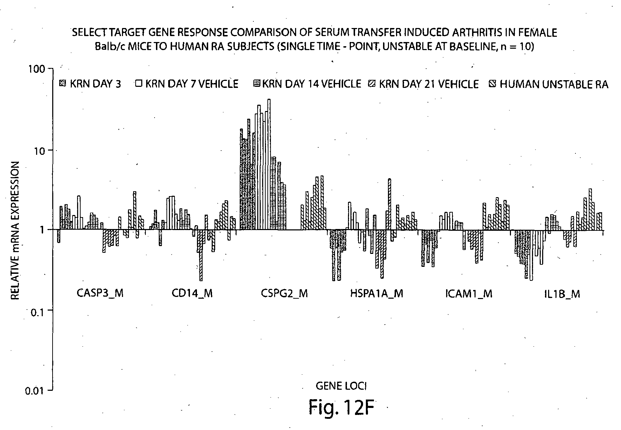

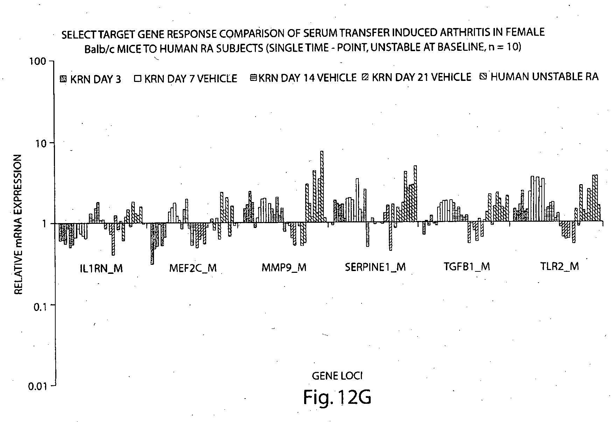

Figures 12A-12E show a comparison of individual murine subject gene expression responses of disease progression at days 3 (untreated), and days 7, 14 and 21 (vehicle-treated) relative to averaged baseline naive murine subject response at day 0 (n=6) in a KRN study using female BALB/c mice. Gene expression analysis was performed using a custom murine 40-gene panel (Precision Profile^{™}) for Rheumatoid Arthritis; Figures 12F and 12G show a comparison of select target gene responses in serum transfer induced arthritis model (KRN) using female BALB/c mice to Human RA Subjects (single time-point, unstable at baseline, n=10).
Figures 13A-13E show a comparison of individual murine subject gene expression responses to dexamethasone treatment at days 33, 42 and 60, relative to respective averaged vehicle-treated murine subject responses at days 33, 42 and 60 in a CIA study using male DBA/1 mice. Gene expression analysis was performed using a custom murine 40-gene panel (Precision Profile^{™}) for Rheumatoid Arthritis.
Figures 14A-14E show a comparison of individual murine subject gene expression responses to vehicle or dexamethasone treatment at day 60 relative to averaged naïve, untreated murine subject responses at day 60 in a CIA study using male DBA/1 mice. Gene expression analysis was performed using a custom murine 40-gene panel (Precision Profile^{™}) for Rheumatoid Arthritis.
Figures 15A-15E show a comparison of individual murine subject gene expression responses to dexamethasone treatment at days 7, 14 and 21, relative to respective averaged vehicle-treated murine subject responses at Days 7, 14 and 21 in a serum transfer induced arthritis model (KRN) using female BALB/c mice. Gene expression analysis was performed using a custom murine 40-gene panel (Precision Profile^{™}) for Rheumatoid Arthritis.
Figures 16A-16E show a comparison of individual murine subject gene expression responses to vehicle or dexamethasone treatment at day 21 relative to averaged naïve, untreated murine subject responses at day 21 in a serum transfer induced arthritis model (KRN) using female BALB/c mice. Gene expression analysis was performed using a custom murine 40-gene panel (Precision Profile^{™}) for Rheumatoid Arthritis.

### DETAILED DESCRIPTION OF THE INVENTION

### Definitions

The following terms shall have the meanings indicated unless the context otherwise requires:
"*Algorithm*" is a set of rules for describing a biological condition. The rule set may be defined exclusively algebraically but may also include alternative or multiple decision points requiring domain-specific knowledge, expert interpretation or other clinical indicators.
An "*agent*" is a "*composition*" or a "*stimulus*", as those terms are defined herein, or a combination of a *composition* and a *stimulus.*
"*Amplification*" in the context of a quantitative RT-PCR assay is a function of the number of DNA replications that are tracked to provide a quantitative determination of its concentration. "*Amplification*" here refers to a degree of sensitivity and specificity of a quantitative assay technique. Accordingly, amplification provides a measurement of concentrations of constituents that is evaluated under conditions wherein the efficiency of amplification and therefore the degree of sensitivity and reproducibility for measuring all constituents is substantially similar.
A "*baseline profile data set*" is a set of values associated with constituents of a *Gene Expression Panel* resulting from evaluation of a biological sample (or population or set of samples) under a desired *biological condition* that is used for mathematically normative purposes. The desired biological condition may be, for example, the condition of a subject (or population or set of subjects) before exposure to an agent or in the presence of an untreated disease or in the absence of a disease. Alternatively, or in addition, the desired biological condition may be health of a subject or a population or set of subjects. Alternatively, or in addition, the desired biological condition may be that associated with a population or set of subjects selected on the basis of at least one of age group, gender, ethnicity, geographic location, nutritional history, medical condition, clinical indicator, medication, physical activity, body mass, and environmental exposure.
A "*biological condition*" of a subject is the condition of the subject in a pertinent realm that is under observation, and such realm may include any aspect of the subject capable of being monitored for change in condition, such as health; disease including inflammation and cancer; trauma; aging; infection; tissue degeneration; developmental steps; physical fitness; obesity, and mood. As can be seen, a condition in this context may be chronic or acute or simply transient. Moreover, a targeted biological condition may be manifest throughout the organism or population of cells or may be restricted to a specific organ (such as skin, heart, eye or blood), but in either case, the condition may be monitored directly by a sample of the affected population of cells or indirectly by a sample derived elsewhere from the subject. The term "*biological condition*" includes a "*physiological condition*".
"*Body fluid*" of a subject includes blood, urine, spinal fluid, lymph, mucosal secretions, prostatic fluid, semen, haemolymph or any other body fluid known in the art for a subject.
"*Calibrated profile data set*"*.* is a function of a member of a first *profile data set* and a corresponding member of a *baseline profile data set* for a given constituent in a panel.
A "*clinical indicator*" is any physiological datum used alone or in conjunction with other data in evaluating the *physiological condition* of a collection of cells or of an organism. This term includes pre-clinical indicators.
A "*composition*". includes a chemical compound, a nutriceutical, a pharmaceutical, a homeopathic formulation, an allopathic formulation, a naturopathic formulation, a combination of compounds, a toxin, a food, a food supplement, a mineral, and a complex mixture of substances, in any physical state or in a combination of physical states. Examples of a "composition" include, without limitation, an aptamer, siRNA, a small molecule agent, an antisense oligo-deoxynucleotide, a monoclonal antibody, a steroidal agent, a non-steroidal anti-inflammatory agent, an alkylating agent, an anti-metabolite, a vinca alkaloid, a taxane, an anthracycline, a topoisomerase inhibitor, a photosensitizer, a tyrosine kinase inhibitor, an epidermal growth factor receptor inhibitor, an FPTase inhibitor, a proteosome inhibitor, a TS/DNA synthesis inhibitor, an S-adenosyl-methionine decarboxylase inhibitor, a DNA methylating agent, a DNA binding agent, and tumor immunotherapy.
To "*derive*" a profile data set from a *sample* includes determining a set of values associated with constituents of a *Gene Expression Panel* either (i) by direct measurement of such constituents in a biological *sample* or (ii) by measurement of such constituents in a second biological *sample* that has been exposed to the original sample or to matter derived from the original sample.
"*Distinct RNA or protein constituent*" in a panel of constituents is a distinct expressed product of a gene, whether RNA or protein. An "*expression*" product of a gene includes the gene product whether RNA or protein resulting from translation of the messenger RNA.
A "*Gene Expression Panel*" (Precision Profile^{™}) is an experimentally verified set of constituents, each constituent being a distinct expressed product of a gene, whether RNA or protein, wherein constituents of the set are selected so that their measurement provides a measurement of a targeted *biological condition*.
A "*Gene Expression Profile*" is a set of values associated with constituents of a *Gene Expression Panel* resulting from evaluation of a biological sample (or population or set of samples).
A "*Gene Expression Profile Inflammatory Index*" is the value of an index function that provides a mapping from an instance of a *Gene Expression Profile* into a single-valued measure of inflammatory condition.
The "*health*" of a subject includes mental, emotional, physical, spiritual, allopathic, naturopathic and homeopathic condition of the subject.
"*Index*" is an arithmetically or mathematically derived numerical characteristic developed for aid in simplifying or disclosing or informing the analysis of more complex quantitative information. A disease or population index may be determined by the application of a specific algorithm to a plurality of subjects or samples with a common biological condition.
"*Inflammation*" is used herein in the general medical sense of the word and may be an acute or chronic; simple or suppurative; localized or disseminated; cellular and tissue response initiated or sustained by any number of chemical, physical or biological agents or combination of agents.
"*Inflammatory state*" is used to indicate the relative biological condition of a subject resulting from inflammation, or characterizing the degree of inflammation.
A "*large number*" of data sets based on a common panel of genes is a number of data sets sufficiently large to permit a statistically significant conclusion to be drawn with respect to an instance of a data set based on the same panel.
A "*normal*" subject is a subject who has not been diagnosed with a biological condition, such as a disease, or one who is not suffering from a biological condition, such as a disease.
A "*normative*" condition of a subject to whom a composition is to be administered means the condition of a subject before administration, even if the subject happens to be suffering from a disease.
A "*panel*" of genes is a set of genes including at least two constituents.
A "*population of cells*" refers to any group of cells wherein there is an underlying commonality or relationship between the members in the population of cells, including a group of cells taken from an organism or from a culture of cells or from a biopsy, for example.
A "*sample*" from a subject may include a single cell or multiple cells or fragments of cells or an aliquot of body fluid, taken from the subject, by means including venipuncture, excretion, ejaculation, massage, biopsy, needle aspirate, lavage sample, scraping, surgical incision or intervention or other means known in the art.
A "*set*" or "*population*" of samples or subjects refers to a defined or selected group of samples or subjects wherein there is an underlying commonality or relationship between the members included in the set or population of samples or subjects.
A "*Signature Profile*" is an experimentally verified subset of a *Gene Expression Profile* selected to discriminate a biological condition, agent or physiological mechanism of action.
A "*Signature Panel*" is a subset of a *Gene Expression Panel (Precision Profile*^{™}), the constituents of which are selected to permit discrimination of a *biological condition, agent* or physiological mechanism of action.
A "*subject*" is a cell, tissue, or organism, human or non-human, whether *in vivo, ex vivo* or *in vitro,* under observation. As used herein, reference to evaluating the biological condition of a subject based on a sample from the subject, includes using blood or other tissue sample from a human subject to evaluate the human subject's condition; it also includes, for example, using a blood sample itself as the subject to evaluate, for example, the effect of therapy or an agent upon the sample. Also included within the definition is the use of blood or other tissue sample from a human or other animal to evaluate a conditionh of the human or animnal in an organ distinct from blood or in a specific physiological domain or tissue distinct from blood.
A "*stimulus*" includes (i) a monitored physical interaction with a subject, for example ultraviolet A or B, or light therapy for seasonal affective disorder, or treatment of psoriasis with psoralen or treatment of cancer with embedded radioactive seeds, other radiation exposure, and (ii) any monitored physical, mental, emotional, or spiritual activity or inactivity of a subject.
"*Therapy*" includes all interventions whether biological, chemical, physical, metaphysical, or combination of the foregoing, intended to sustain or alter the monitored biological condition of a subject.

United States patents 6,960,439 and 6,964,850, each entitled "Identification, Monitoring and Treatment of Disease and Characterization of Biological Condition Using Gene Expression Profiles," and assigned to Source Precision Medicine, Inc., which are incorporated herein by reference in their entirety, disclose the use of Gene Expression Panels for the evaluation of (i) a biological condition (including with respect to health and disease) and (ii) the effect of one or more agents on a biological condition (including with respect to health, toxicity, therapeutic treatment and drug interaction). These patents disclose the unprecedented insight that normal levels of gene expression associated with inflammation, occur in healthy populations of humans, and departures from these normal levels of expression in individual subjects are indicative of departure from health. These patents show that changes to or from such normal levels are indicative of changes to or from health, and so that Gene Expression Panels can be used for monitoring and assessment of treatment of a biological condition, arising, for example from disease. (The observations giving rise to these insights derive from gene expression measurements made under conditions that are substantially repeatable and having, for example, an average coefficient of variation of intra-assay variability or inter-assay variability of less than 20%, more preferably less than 10%, more preferably less than 5%, more preferably less than 4%, more preferably less than 3%, more preferably less than 2%, and even more preferably less than 1%).

It has been surprisingly discovered that expression of genes in rodents, when measured under conditions that are substantially repeatable, exhibits characteristics analogous to gene expression in humans when measured under conditions that are substantially repeatable. In particular, normal levels of gene expression associated with inflammation, occur in healthy populations both of humans and of rodents, and departures from these normal levels of expression in individual subjects are indicative of departure from health. Thus changes to or from such normal levels in rodents as well as humans are indicative of changes to or from health, and allow monitoring and treatment of biological condition, arising, for example from disease.

We have furthermore found these insights in gene expression can be harnessed to make rodents much more effective models for humans in connection with evaluation of the treatment of disease. In particular, a Gene Expression Panel in a rodent context can be used for evaluating the effect of an agent in treating a human biological condition when the rodent Gene Expression Panel is selected with the proper methodology: The selection methodology for the rodent Gene Expression Panel requires identification of genes in rodents which respond similarly to corresponding genes in humans with respect to expression in the context of a given biological condition. Examples of such are provided below.

Furthermore, United States patents 6,960,439 and 6,964,850 disclose the use of indices to characterize a state of health or disease. Indices can be used in the rodent context to characterize the state of health or disease in the rodent for purposes of assessing the effect of the agent, first, in the rodent and, second, as a predictor of the effect of the agent in humans. In particular, indices can be used in the rodent context for predicting therapeutic efficacy of natural or synthetic compositions or stimuli in humans that may be formulated individually or in combinations or mixtures for a range of targeted biological conditions; prediction of toxicological effects and dose effectiveness of a composition or mixture of compositions for an individual or for a population or set of individuals or for a population of cells; determination of how two or more different agents administered in a single treatment might interact so as to predict any of synergistic, additive, negative, neutral or toxic activity in humans; and conducting preliminary dosage studies for these patients prior to conducting phase 1 or 2 trials. In this respect, indices in the mouse context can be used just as described below in the human context.

The term "agent" as used herein is defined above. An agent may be, for example without limitation, a drug or neutraceutical proposed for the treatment of a disease. However, the rodent context as described herein may be used not simply to model and evaluate short-term and long-term efficacy of a drug or other agent, but also to model and evaluate potential toxicity, side effects, and contraindications, of a drug or other agent. Moreover, the rodent model may be useful in evaluating agent effects on subpopulations, such as those based on age, gender, pregnancy, or immune-system compromised status.

In accordance with embodiments herein, a rodent model for a given biological condition may be developed using a "Reverse Engineered Animal Model" (REAM) strategy. In accordance with the REAM strategy, the procedures are as follows:
1. Identify a Gene Expression Panel for humans providing expression levels that are indicative of the biological condition of interest;
2. Assess in a rodent population the genes of the Gene Expression Panel identified in process (1) to determine which constiuents are indicative of the biological condition of interest in both humans and rodents. These constituents constitute a Signature Panel in rodents that can be used to assess the effect of an agent on the biological condition of interest. The assessment of an agent for the treatment of a biological condition includes identifying agents suitable for the treatment of the biological condition of interest. By suitable for treatment is meant determining whether the agent will be efficacious, not efficacious, or toxic for a particular test subject or individual. By toxic it is meant that the manifestations of one or more adverse effects of a drug when administered therapeutically. For example, a drug is toxic when it disrupts one or more normal physiological pathways.

Accordingly, the methods disclosed herein allow for a putative therapeutic or prophylactic to be tested from a rodent sample in order to determine if the agent is a suitable for treating or preventing a biological condition of interest in a human subject. The agents can be compounds known to treat the biological condition of interest or novel agents that have not been previously shown to treat the biological condition of interest.

The effect of an agent on a biological condition of interest is evaluated by determinining the level of expression (*e.g*., a quantitative measure) of one or more relevant genes in the rodent Signature panel. The level of expression is determined by any means known in the art, such as for example quantitative PCR. The measurement is obtained under conditions that are substantially repeatable, as described below.. Optionally, the qualitative measure of the constituent is compared to a baseline level (e.g. baseline profile set). A baseline level is a level of expression of the constituent in one or more subjects known not to be suffering from the biological condition of interest (e.g., normal, healthy subject(s)).

To identify a therapeutic that is appropriate for a human subject, a test sample from a rodent subject is exposed to a candidate therapeutic agent, and the expression of one or more of genes indicative of the biological condition of interest in both humans and rodents (referred to as the Signature Panel in rodents, described above) is determined. The rodent sample is incubated in the presence of a candidate agent and the pattern of gene expression in the rodent test sample is measured and compared to a reference sample, e.g., a baseline profile for the biological condition condition of interest, or an index value. The test agent can be any compound or composition. A similarity in the expression pattern of genes from the rodent test sample compared to a reference sample indicates that the treatment is predicted to be efficacious in a human subject. Whereas a change in the expression pattern of genes in the test sample compared to the reference sample indicates a less favorable clinical outcome or prognosis in a human subject.

By "efficacious", is meant that the treatment leads to a decrease of a sign or symtptom of the biological condition of interest in the subject or a change in the pattern of expression of one or more genes indicative of the biological condition such that the gene expression pattern has an increase in similarity to that of a normal baseline pattern of gene expression. Assessment of the biological condition of interest is made using standard clinical protocols. Efficacy is determined in association with any known method for diagnosing or treating the biological condition of interest.

The human and rodent Gene Expression Panels (Precision Profile^{™}) and Signature panels are selected in a manner so that quantitative measurement of RNA or protein constituents in the Panel constitutes a measurement of a biological condition of a subject. In one kind of arrangement, a calibrated profile data set is employed. Each member of the calibrated profile data set is a function of (i) a measure of a distinct constituent of a Gene Expression Panel (Precision Profile^{™}) and (ii) a baseline quantity.

It has been discovered that valuable and unexpected results may be achieved when the quantitative measurement of constituents is performed under repeatable conditions (within a degree of repeatability of measurement of better than twenty percent, and preferably ten percent or better, more preferably five percent or better, and more preferably three percent or better). For the purposes of this description and the following claims, a degree of repeatability of measurement of better than twenty percent as providing measurement conditions that are "substantially repeatable". In particular, it is desirable that each time a measurement is obtained corresponding to the level of expression of a constituent in a particular sample, substantially the same measurement should result for substantially the same level of expression. In this manner, expression levels for a constituent in a Gene Expression Panel (Precision Profile^{™}) may be meaningfully compared from sample to sample. Even if the expression level measurements for a particular constituent are inaccurate (for example, say, 30% too low), the criterion ofrepeatability means that all measurements for this constituent, if skewed, will nevertheless be skewed systematically, and therefore measurements of expression level of the constituent may be compared meaningfully. In this fashion valuable information may be obtained and compared concerning expression of the constituent under varied circumstances.

In addition to the criterion of repeatability, it is desirable that a second criterion also be satisfied, namely that quantitative measurement of constituents is performed under conditions wherein efficiencies of amplification for all constituents are substantially similar as defined herein. When both of these criteria are satisfied, then measurement of the expression level of one constituent may be meaningfully compared with measurement of the expression level of another constituent in a given sample and from sample to sample.

Additional embodiments relate to the use of an index or algorithm resulting from quantitative measurement of constituents, and optionally in addition, derived from either expert analysis or computational biology (a) in the analysis of complex data sets; (b) to control or normalize the influence of uninformative or otherwise minor variances in gene expression values between samples or subjects; (c) to simplify the characterization of a complex data set for comparison to other complex data sets, databases or indices or algorithms derived from complex data sets; (d) to monitor a biological condition of a subject; (e) for measurement of therapeutic efficacy of natural or synthetic compositions or stimuli that may be formulated individually or in combinations or mixtures for a range of targeted biological conditions; (f) for predictions of toxicological effects and dose effectiveness of a composition or mixture of compositions for an individual or for a population or set of individuals or for a population of cells; (g) for determination of how two or more different agents administered in a single treatment might interact so as to detect any of synergistic, additive, negative, neutral of toxic activity (h) for performing pre-clinical and clinical trials by providing new criteria for pre-selecting subjects according to informative profile data sets for revealing disease status and conducting preliminary dosage studies for these patients prior to conducting phase 1 or 2 trials.

Gene expression profiling and the use of index characterization for a particular condition or agent or both may be used to reduce the cost of phase 3 clinical trials and may be used beyond phase 3 trials; labeling for approved drugs; selection of suitable medication in a class of medications for a particular patient that is directed to their unique physiology; diagnosing or determining a prognosis of a medical condition or an infection which may precede onset of symptoms or alternatively diagnosing adverse side effects associated with administration of a therapeutic agent; managing the health care of a patient; and quality control for different batches of an agent or a mixture of agents.

### The subject

The methods disclosed here may be applied to cells of humans, mammals or other organisms without the need for undue experimentation by one of ordinary skill in the art because all cells transcribe RNA and it is known in the art how to extract RNA from all types of cells. A subject can include those who have not been previously diagnosed as having a biological condition, or those who have not been induced to have a biological condition, such as a disease. Alternatively, a subject can also include those who have already been diagnosed as having a biological condition, or those who have been induced to have a biological condition, such as a disease. Optionally, the subject has previously been treated with a therapeutic agent. A subject can also include those who are suffering from, or at risk of developing a biological condition.

### Selecting constituents of a Gene Expression Panel

The general approach to selecting constituents of a Gene Expression Panel (Precision Profile^{™}) has been described in PCT application publication number WO 01/25473, incorporated herein in its entirety. A wide range of Gene Expression Panels (Precision Profiles^{™}) have been designed and experimentally validated, each panel providing a quantitative measure of biological condition that is derived from a sample of blood or other tissue. For each panel, experiments have verified that a Gene Expression Profile using the panel's constituents is informative of a biological condition. It has also been demonstrated that in being informative of biological condition, the Gene Expression Profile is used, among other things, to measure the effectiveness of therapy, as well as to provide a target for therapeutic intervention.

Tables 1-9 listed below, include relevant genes which may be selected for a given rodent Signature panel of genes, useful for the assessment of an agent on a human biological condition. One of ordinary skill in the art would recognize that orthologues and/or homologs of any of the genes listed in Tables 1-9 below may also be selected for a given rodent Signature Panel, useful for the assessment of an agent on a human biological condition.
Table 1. Inflammation Gene Expression Panel
Table 2. Rhumatoid Arthritis or Inflammatory Conditions Related to Rheumatoid Arthritis Gene Expression Panel
Table 3. Mouse Gene Expression Panel (24-Gene) for Inflammation
Table 4. Mouse 8-Gene Signature Panel for Inflammation (LPS Infusion)
Table 5. Mouse 20-Gene Signature Panel for Inflammation
Table 6. Mouse 8-Gene Signature Panel for Inflammation (LPS + Dexamethasone).
Table 7. Mouse 9-Gene Signature Panel for Inflammation (LPS-Stimulated Whole Blood Response).
Table 8. Mouse 8-Gene Signature Panel for Inflammation (LPS-Stimulated Whole Blood Response).
Table 9. Mouse 40-Gene Expression Panel for Rheumatoid Arthritis

In addition to the panels shown in Tables 1 through 9 above, other panels may be constructed and experimentally verified by one of ordinary skill in the art in accordance with the principles articulated in the present application.

In general, panels may be constructed and experimentally validated by one of ordinary skill in the art in accordance with the principles articulated in the present application.

### Design of assays

Typically, a sample is run through a panel in replicates of three for each target gene (assay); that is, a sample is divided into aliquots and for each aliquot the concentrations of each constituent in a Gene Expression Panel (Precision Profile^{™}) is measured. From over a total of 900 constituent assays, with each assay conducted in triplicate, an average coefficient of variation was found (standard deviation/average)* 100, of less than 2 percent among the normalized ACt measurements for each assay (where normalized quantitation of the target mRNA is determined by the difference in threshold cycles between the internal control (*e.g*., an endogenous marker such as 18S rRNA, or an exogenous marker) and the gene of interest. This is a measure called "intra-assay variability". Assays have also been conducted on different occasions using the same sample material. This is a measure of "inter-assay variability". Preferably, the average coefficient of variation ofintra- assay variability or inter-assay variability is less than 20%, more preferably less than 10%, more preferably less than 5%, more preferably less than 4%, more preferably less than 3%, more preferably less than 2%, and even more preferably less than 1%.

It has been determined that it is valuable to use the quadruplicate or triplicate test results to identify and eliminate data points that are statistical "outliers"; such data points are those that differ by a percentage greater, for example, than 3% of the average of all three or four values. Moreover, if more than one data point in a set of three or four is excluded by this procedure, then all data for the relevant constituent is discarded.

### Measurement of Gene Expression for a Constituent in the Panel

For measuring the amount of a particular RNA in a sample, methods known to one of ordinary skill in the art were used to extract and quantify transcribed RNA from a sample with respect to a constituent of a Gene Expression Panel (Precision Profile^{™}). (See detailed protocols below. Also see PCT application publication number WO 98/24935 herein incorporated by reference for RNA analysis protocols). Briefly, RNA is extracted from a sample such as any tissue, body fluid, cell, or culture medium in which a population of cells of a subject might be growing. For example, cells may be lysed and RNA eluted in a suitable solution in which to conduct a DNAse reaction. Subsequent to RNA extraction, first strand synthesis may be performed using a reverse transcriptase. Gene amplification, more specifically quantitative PCR assays, can then be conducted and the gene of interest calibrated against an internal marker such as 18S rRNA (Hirayama et al., Blood 92, 1998: 46-52). Any other endogenous marker can be used, such as 28S-25S rRNA and 5S rRNA. Samples are measured in multiple replicates, for example, 3 replicates. In an embodiment of the invention, quantitative PCR is performed using amplification, reporting agents and instruments such as those supplied commercially by Applied Biosystems (Foster City, CA). Given a defined efficiency of amplification of target transcripts, the point (*e.g*., cycle number) that signal from amplified target template is detectable may be directly related to the amount of specific message transcript in the measured sample. Similarly, other quantifiable signals such as fluorescence, enzyme activity, disintegrations per minute, absorbance, etc., when correlated to a known concentration of target templates (*e.g.*, a reference standard curve) or normalized to a standard with limited variability can be used to quantify the number of target templates in an unknown sample.

Although not limited to amplification methods, quantitative gene expression techniques may utilize amplification of the target transcript. Alternatively or in combination with amplification of the target transcript, quantitation of the reporter signal for an internal marker generated by the exponential increase of amplified product may also be used. Amplification of the target template may be accomplished by isothermic gene amplification strategies or by gene amplification by thermal cycling such as PCR.

It is desirable to obtain a definable and reproducible correlation between the amplified target or reporter signal, i.e., internal marker, and the concentration of starting templates. It has been discovered that this objective can be achieved by careful attention to, for example, consistent primer-template ratios and a strict adherence to a narrow permissible level of experimental amplification efficiencies (for example 90.0 to 100% +/- 5% relative efficiency, typically 99.8 to 100% relative efficiency). For example, in determining gene expression levels with regard to a single Gene Expression Profile, it is necessary that all constituents of the panels, including endogenous controls, maintain similar amplification efficiencies, as defined herein, to permit accurate and precise relative measurements for each constituent. Amplification efficiencies are regarded as being "substantially similar", for the purposes of this description and the following claims, if they differ by no more than approximately 10%, preferably by less than approximately 5%, more preferably by less than approximately 3%, and more preferably by less than approximately 1%. Measurement conditions are regarded as being "substantially repeatable, for the purposes of this description and the following claims, if they differ by no more than approximately +/- 10% coefficient of variation (CV), preferably by less than approximately +/- 5% CV, more preferably +/- 2% CV. These constraints should be observed over the entire range of concentration levels to be measured associated with the relevant biological condition. While it is thus necessary for various embodiments herein to satisfy criteria that measurements are achieved under measurement conditions that are substantially repeatable and wherein specificity and efficiencies of amplification for all constituents are substantially similar, nevertheless, it is within the scope of the present invention as claimed herein to achieve such measurement conditions by adjusting assay results that do not satisfy these criteria directly, in such a manner as to compensate for errors, so that the criteria are satisfied after suitable adjustment of assay results.

In practice, tests are run to assure that these conditions are satisfied. For example, the design of all primer-probe sets are done in house, experimentation is performed to determine which set gives the best performance. Even though primer-probe design can be enhanced using computer techniques known in the art, and notwithstanding common practice, it has been found that experimental validation is still useful. Moreover, in the course of experimental validation, the selected primer-probe combination is associated with a set of features:

The reverse primer should be complementary to the coding DNA strand. In one embodiment, the primer should be located across an intron-exon junction, with not more than four bases of the three-prime end of the reverse primer complementary to the proximal exon. (If more than four bases are complementary, then it would tend to competitively amplify genomic DNA.)

In an embodiment of the invention, the primer probe set should amplify cDNA of less than 110 bases in length and should not amplify, or generate fluorescent signal from, genomic DNA or transcripts or cDNA from related but biologically irrelevant loci.

A suitable target of the selected primer probe is first strand cDNA, which in one embodiment may be prepared from whole blood as follows:

### (a) Use of cell systems or whole blood for ex vivo assessment of a biological condition affected by an agent.

In one embodiment of the invention, any tissue, body fluid, or cell(s) may be used for *ex vivo* assessment of a biological condition affected by an agent. Nucleic acids, RNA and/or DNA are purified from cells, tissues or fluids of the test population of cells or indicator cell lines. RNA is preferentially obtained from the nucleic acid mix using a variety of standard procedures (or RNA Isolation Strategies, pp. 55-104, in RNA Methodologies, A laboratory guide for isolation and characterization, 2nd edition, 1998, Robert E. Farrell, Jr., Ed., Academic Press), in the present using a filter-based RNA isolation system from Ambion (RNAqueous ^{™}, Phenol-free Total RNA Isolation Kit, Catalog #1912, version 9908; Austin, Texas).

In another embodiment of the invention, human blood is obtained by venipuncture and prepared for assay by separating samples for baseline, no exogenous stimulus, and one or more pro-disease stimulus with sufficient volume for at least three time points. The aliquots of heparinized, whole blood are mixed with additional test therapeutic compounds and held at 37°C in an atmosphere of 5% CO₂ for 30 minutes. Stimulus is added at varying concentrations, mixed and held loosely capped at 37°C for the prescribed timecourse. At defined time-points, cells are lysed and RNA extracted by various standard means.

In accordance with one procedure, the whole blood assay for Gene Expression Profiles determination is carried out as follows: Human whole blood is drawn into 10 mL Vacutainer tubes with Sodium Heparin. Blood samples are mixed by gently inverting tubes 4-5 times. The blood is used within 10-15 minutes of draw. In the experiments, blood is diluted 2-fold, i.e. per sample per time point, 0.6 mL whole blood + 0.6 mL stimulus. The assay medium is prepared and the stimulus added as appropriate.

A quantity (0.6 mL) of whole blood is then added into each 12 x 75 mm polypropylene tube. 0.6 mL of 2X LPS (from *E. coli* serotype 0127:B8, Sigma#L3880 or serotype 055, Sigma #L4005, 10ng/mL subject to change in different lots) into LPS tubes is added. Next, 0.6 mL assay medium is added to the "control" tubes. The caps are closed tightly. The tubes are inverted 2-3 times to mix samples. Caps are loosened to first stop and the tubes incubated at 37°C, 5% CO₂ for 6 hours. At 6 hours, samples are gently mixed to resuspend blood cells, and 0.15 mL is removed from each tube (using a micropipettor with barrier tip), and transfered to 0.15 mL of lysis buffer and mixed. Lysed samples are extracted using an ABI 6100 Nucleic Acid Prepstation following the manufacturer's recommended protocol.

The samples are then centrifuged for 5 min at 500 x g, ambient temperature (IEC centrifuge or equivalent, in microfuge tube adapters in swinging bucket), and as much serum from each tube is removed as possible and discarded. Cell pellets are placed on ice; and RNA extracted as soon as possible using an Ambion RNAqueous kit.

### (b) Amplification strategies.

Specific RNAs are amplified using message specific primers or random primers. The specific primers are synthesized from data obtained from public databases (*e.g*., Unigene, National Center for Biotechnology Information, National Library of Medicine, Bethesda, MD), including information from genomic and cDNA libraries obtained from humans and other animals. Primers are chosen to preferentially amplify from specific RNAs obtained from the test or indicator samples (see, for example, RT PCR, Chapter 15 in RNA Methodologies, A laboratory guide for isolation and characterization, 2nd edition, 1998, Robert E. Farrell, Jr., Ed., Academic Press; or Chapter 22 pp.143-151, RNA isolation and characterization protocols, Methods in molecular biology, Volume 86, 1998, R. Rapley and D. L. Manning Eds., Human Press, or 14 in Statistical refinement of primer design parameters, Chapter 5, pp.55-72, PCR applications: protocols for functional genomics, M.A.Innis, D.H. Gelfand and J.J. Sninsky, Eds., 1999, Academic Press). Amplifications are carried out in either isothermic conditions or using a thermal cycler (for example, a ABI 9600 or 9700 or 7900 obtained from Applied Biosystems, Foster City, CA; see Nucleic acid detection methods, pp. 1-24, in Molecular methods for virus detection, D.L.Wiedbrauk and D.H., Farkas, Eds., 1995, Academic Press). Amplified nucleic acids are detected using fluorescent-tagged detection oligonucleotide probes (see, for example, TaqmanTM PCR Reagent Kit, Protocol, part number 402823, Revision A, 1996, Applied Biosystems, Foster City CA) that are identified and synthesized from publicly known databases as described for the amplification primers. In the present case, amplified cDNA is detected and quantified using the ABI Prism 7900 Sequence Detection System obtained from Applied Biosystems (Foster City, CA). Amounts of specific RNAs contained in the test sample or obtained from the indicator cell lines can be related to the relative quantity of fluorescence observed (see for example, Advances in quantitative PCR technology: 5' nuclease assays, Y.S. Lie and C.J. Petropolus, Current Opinion in Biotechnology, 1998, 9:43-48, or Rapid thermal cycling and PCR kinetics, pp. 211-229, chapter 14 in PCR applications: protocols for functional genomics, M.A. Innis, D.H. Gelfand and J.J. Sninsky, Eds., 1999, Academic Press).

As a particular implementation of the approach described here in detail is a procedure for synthesis of first strand cDNA for use in PCR. This procedure can be used for both whole blood RNA and RNA extracted from cultured cells (*e.g*., trabecular meshwork, retinal Ganglion cells, optic nerve head cells and choroid epithelial cells).

### Materials

1. Applied Biosystems TAQMAN Reverse Transcription Reagents Kit (P/N 808-0234). Kit Components: 10X TaqMan RT Buffer, 25 mM Magnesium chloride, deoxyNTPs mixture, Random Hexamers, RNase Inhibitor, MultiScribe Reverse Transcriptase (50 U/mL) (2) RNase / DNase free water (DEPC Treated Water from Ambion (P/N 9915G), or equivalent)

### Methods

1. Place RNase Inhibitor and MultiScribe Reverse Transcriptase on ice immediately. All other reagents can be thawed at room temperature and then placed on ice.
2. Remove RNA samples from -80°C freezer and thaw at room temperature and then place immediately on ice.
3. Prepare the following cocktail of Reverse Transcriptase Reagents for each 100 mL RT reaction (for multiple samples, prepare extra cocktail to allow for pipetting error):

| 1 reaction (mL) | 11X, e.g. 10 samples (µL) | |
|---|---|---|
| 10X RT Buffer | 10.0 | 110.0 |
| 25 mM MgCl₂ | 22.0 | 242.0 |
| dNTPs | 20.0 | 220.0 |
| Random Hexamers | 5.0 | 55.0 |
| RNAse Inhibitor | 2.0 | 22.0 |
| Reverse Transcriptase | 2.5 | 27.5 |
| Water | 18.5 | 203.5 |
| Total: | 80.0 | 880.0 (80 µL per sample) |

4. Bring each RNA sample to a total volume of 20 µL in a 1.5 mL microcentrifuge tube (for example, for THP-1 RNA, remove 10 µL RNA and dilute to 20 µL with RNase / DNase free water, for whole blood RNA use 20 µL total RNA) and add 80 µL RT reaction mix from step 5,2,3. Mix by pipetting up and down.
5. Incubate sample at room temperature for 10 minutes.
6. Incubate sample at 37°C for 1 hour.
7. Incubate sample at 90°C for 10 minutes.
8. Quick spin samples in microcentrifuge.
9. Place sample on ice if doing PCR immediately, otherwise store sample at-20°C for future use.
10. PCR QC should be run on all RT samples using 18S and β-actin.

The use of the primer probe with the first strand cDNA as described above to permit measurement of constituents of a Gene Expression Panel (Precision Profile^{™}) is as follows:

### Materials

1. 20X Primer/Probe Mix for each gene of interest.
2. 20X Primer/Probe Mix for 18S endogenous control.
3. 2X Taqman Universal PCR Master Mix.
4. cDNA transcribed from RNA extracted from cells.
5. Applied Biosystems 96-Well Optical Reaction Plates.
6. Applied Biosystems Optical Caps, or optical-clear film.
7. Applied Biosystem Prism 7700 or 7900 Sequence Detector.

### Methods

1. Make stocks of each Primer/Probe mix containing the Primer/Probe for the gene of interest, Primer/Probe for 18S endogenous control, and 2X PCR Master Mix as follows. Make sufficient excess to allow for pipetting error e.g., approximately 10% excess. The following example illustrates a typical set up for one gene with quadruplicate samples testing two conditions (2 plates).

| | 1X (1 well) (µL) |
|---|---|
| 2X Master Mix | 7.5 |
| 20X 18S Primer/Probe Mix | 0.75 |
| 20X Gene of interest Primer/Probe Mix | 0.75 |
| Total | 9.0 |

2. Make stocks of cDNA targets by diluting 95µL of cDNA into 2000µL of water. The amount of cDNA is adjusted to give Ct values between 10 and 18, typically between 12 and 16.
3. Pipette 9 µL of Primer/Probe mix into the appropriate wells of an Applied Biosystems 384-Well Optical Reaction Plate.
4. Pipette 10µL of cDNA stock solution into each well of the Applied Biosystems 384-Well Optical Reaction Plate.
5. Seal the plate with Applied Biosystems Optical Caps, or optical-clear film.
6. Analyze the plate on the ABI Prism 7900 Sequence Detector.

In another embodiment of the invention, the use of the primer probe with the first strand cDNA as described above to permit measurement of constituents of a Gene Expression Panel (Precision Profile^{™}) is performed using a QPCR assay on Cepheid SmartCycler®. and GeneXpert® Instruments as follows:

I. To run a QPCR assay in duplicate on the Cepheid SmartCycler® instrument containing three target genes and one reference gene, the following procedure should be followed.

### A. With 20X Primer/Probe Stocks.

### Materials

1. SmartMix™-HM lyophilized Master Mix.
2. Molecular grade water.
3. 20X Primer/Probe Mix for the 18S endogenous control gene. The endogenous control gene will be dual labeled with VIC-MGB or equivalent.
4. 20X Primer/Probe Mix for each for target gene one, dual labeled with FAM-BHQ1 or equivalent.
5. 20X Primer/Probe Mix for each for target gene two, dual labeled with Texas Red-BHQ2 or equivalent.
6. 20X Primer/Probe Mix for each for target gene three, dual labeled with Alexa 647-BHQ3 or equivalent.
7. Tris buffer, pH 9.0
8. cDNA transcribed from RNA extracted from sample.
9. SmartCycler®.25 µL tube.
10. Cepheid SmartCycler® instrument.

### Methods

1. For each cDNA sample to be investigated, add the following to a sterile 650 µL tube.

| SmartMix™-HM lyophilized Master Mix | 1 bead |
|---|---|
| 20X 18S Primer/Probe Mix | 2.5 µL |
| 20X Target Gene 1 Primer/Probe Mix | 2.5 µL |
| 20X Target Gene 2 Primer/Probe Mix | 2.5 µL |
| 20X Target Gene 3 Primer/Probe Mix | 2.5 µL |
| Tris Buffer, pH 9.0 | 2.5 µL |
| Sterile Water | 34.5 µL |
| Total | 47 µL |

Vortex the mixture for 1 second three times to completely mix the reagents. Briefly centrifuge the tube after vortexing.
2. Dilute the cDNA sample so that a 3 µL addition to the reagent mixture above will give an 18S reference gene CT value between 12 and 16.
3. Add 3 µL of the prepared cDNA sample to the reagent mixture bringing the total volume to 50 µL. Vortex the mixture for 1 second three times to completely mix the reagents. Briefly centrifuge the tube after vortexing.
4. Add 25 µL of the mixture to each of two SmartCycler® tubes, cap the tube and spin for 5 seconds in a microcentrifuge having an adapter for SmartCycler® tubes.
5. Remove the two SmartCycler® tubes from the microcentrifuge and inspect for air bubbles. If bubbles are present, re-spin, otherwise, load the tubes into the SmartCycler® instrument.
6. Run the appropriate QPCR protocol on the SmartCycler®, export the data and analyze the results.

### B. With Lyophilized SmartBeads™.

### Materials

1. SmartMix™-HM lyophilized Master Mix.
2. Molecular grade water.
3. SmartBeads™ containing the 18S endogenous control gene dual labeled with VIC-MGB or equivalent, and the three target genes, one dual labeled with FAM-BHQ1 or equivalent, one dual labeled with Texas Red-BHQ2 or equivalent and one dual labeled with Alexa 647-BHQ3 or equivalent.
4. Tris buffer, pH 9.0
5. cDNA transcribed from RNA extracted from sample.
6. SmartCycler® 25 µL tube.
7. Cepheid SmartCycler® instrument.

### Methods

1. For each cDNA sample to be investigated, add the following to a sterile 650 µL tube.

| | |
|---|---|
| SmartMix™-HM lyophilized Master Mix | 1 bead |
| SmartBead™ containing four primer/probe sets | 1 bead |
| Tris Buffer, pH 9.0 | 2.5 µL |
| Sterile Water | 44.5 µL |
| Total | 47 µL |

Vortex the mixture for 1 second three times to completely mix the reagents. Briefly centrifuge the tube after vortexing.
2. Dilute the cDNA sample so that a 3 µL addition to the reagent mixture above will give an 18S reference gene CT value between 12 and 16.
3. Add 3 µL of the prepared cDNA sample to the reagent mixture bringing the total volume to 50 µL. Vortex the mixture for 1 second three times to completely mix the reagents. Briefly centrifuge the tube after vortexing.
4. Add 25 µL of the mixture to each of two SmartCycler® tubes, cap the tube and spin for 5 seconds in a microcentrifuge having an adapter for SmartCycle® tubes.
5. Remove the two SmartCycler® tubes from the microcentrifuge and inspect for air bubbles. If bubbles are present, re-spin, otherwise, load the tubes into the SmartCycler® instrument.
6. Run the appropriate QPCR protocol on the SmartCycler®, export the data and analyze the results.

II. To run a QPCR assay on the Cepheid GeneXpert® instrument containing three target genes and one reference gene, the following procedure should be followed. Note that to do duplicates, two self contained cartridges need to be loaded and run on the GeneXpert® instrument.

### Materials

1. Cepheid GeneXpert® self contained cartridge preloaded with a lyophilized SmartMix™-HM master mix bead and a lyophilized SmartBead™ containing four primer/probe sets.
2. Molecular grade water, containing Tris buffer, pH 9.0.
3. Extraction and purification reagents.
4. Clinical sample (whole blood, RNA, etc.)
5. Cepheid GeneXpert®. instrument.

### Methods

1. Remove appropriate GeneXpert®. self contained cartridge from packaging.
2. Fill appropriate chamber of self contained cartridge with molecular grade water with Tris buffer, pH 9.0.
3. Fill appropriate chambers of self contained cartridge with extraction and purification reagents.
4. Load aliquot of clinical sample into appropriate chamber of self contained cartridge.
5. Seal cartridge and load into GeneXpert® instrument.
6. Run the appropriate extraction and amplification protocol on the GeneXpert® and analyze the resultant data.

Methods herein may also be applied using proteins where sensitive quantitative techniques, such as an Enzyme Linked ImmunoSorbent Assay (ELISA) or mass spectroscopy, are available and well-known in the art for measuring the amount of a protein constituent. (see WO 98/24935 herein incorporated by reference).

### Baseline profile data sets

The analyses of samples from single individuals and from large groups of individuals provide a library of profile data sets relating to a particular panel or series of panels. These profile data sets may be stored as records in a library for use as baseline profile data sets. As the term "baseline" suggests, the stored baseline profile data sets serve as comparators for providing a calibrated profile data set that is informative about a biological condition or agent. Baseline profile data sets may be stored in libraries and classified in a number of cross-referential ways. One form of classification may rely on the characteristics of the panels from which the data sets are derived. Another form of classification may be by particular biological condition, e.g., inflammation. The concept of biological condition encompasses any state in which a cell or population of cells may be found at any one time. This state may reflect geography of samples, sex of subjects or any other discriminator. Some of the discriminators may overlap. The libraries may also be accessed for records associated with a single subject or particular clinical trial. The classification of baseline profile data sets may further be annotated with medical information about a particular subject, a medical condition, and/or a particular agent.

The choice of a baseline profile data set for creating a calibrated profile data set is related to the biological condition to be evaluated, monitored, or predicted, as well as, the intended use of the calibrated panel, e.g., as to monitor drug development, quality control or other uses. It may be desirable to access baseline profile data sets from the same subject for whom a first profile data set is obtained or from different subject at varying times, exposures to stimuli, drugs or complex compounds; or may be derived from like or dissimilar populations or sets of subjects. The baseline profile data set may be normal, healthy baseline.

The profile data set may arise from the same subject for which the first data set is obtained, where the sample is taken at a separate or similar time, a different or similar site or in a different or similar biological condition. For example, a sample may be taken before stimulation or after stimulation with an exogenous compound or substance, such as before or after therapeutic treatment. The profile data set obtained from the unstimulated sample may serve as a baseline profile data set for the sample taken after stimulation. The baseline data set may also be derived from a library containing profile data sets of a population or set of subjects having some defining characteristic or biological condition. The baseline profile data set may also correspond to some *ex vivo* or *in vitro* properties associated with an *in vitro* cell culture. The resultant calibrated profile data sets may then be stored as a record in a database or library along with or separate from the baseline profile data base and optionally the first profile data set although the first profile data set would normally become incorporated into a baseline profile data set under suitable classification criteria. The remarkable consistency of Gene Expression Profiles associated with a given biological condition makes it valuable to store profile data, which can be used, among other things for normative reference purposes. The normative reference can serve to indicate the degree to which a subject conforms to a given biological condition (healthy or diseased) and, alternatively or in addition, to provide a target for clinical intervention.

Selected baseline profile data sets may be also be used as a standard by which to judge manufacturing lots in terms of efficacy, toxicity, etc. Where the effect of a therapeutic agent is being measured, the baseline data set may correspond to Gene Expression Profiles taken before administration of the agent. Where quality control for a newly manufactured product is being determined, the baseline data set may correspond with a gold standard for that product. However, any suitable normalization techniques may be employed. For example, an average baseline profile data set is obtained from authentic material of a naturally grown herbal nutraceutical and compared over time and over different lots in order to demonstrate consistency, or lack of consistency, in lots of compounds prepared for release.

### Calibrated data

Given the repeatability achieved in measurement of gene expression, described above in connection with "Gene Expression Panels" (Precision Profiles^{™}) and "gene amplification", it was concluded that where differences occur in measurement under such conditions, the differences are attributable to differences in biological condition. Thus, it has been found that calibrated profile data sets are highly reproducible in samples taken from the same individual under the same conditions. Similarly, it has been found that calibrated profile data sets are reproducible in samples that are repeatedly tested. Also found have been repeated instances wherein calibrated profile data sets obtained when samples from a subject are exposed *ex vivo* to a compound are comparable to calibrated profile data from a sample that has been exposed to a sample in vivo. Importantly, it has been determined that an indicator cell line treated with an agent can in many cases provide calibrated profile data sets comparable to those obtained from *in vivo* or *ex vivo* populations of cells. Moreover, it has been determined that administering a sample from a subject onto indicator cells can provide informative calibrated profile data sets with respect to the biological condition of the subject including the health, disease states, therapeutic interventions, aging or exposure to environmental stimuli or toxins of the subject.

### Calculation of calibrated profile data sets and computational aids

The calibrated profile data set may be expressed in a spreadsheet or represented graphically for example, in a bar chart or tabular form but may also be expressed in a three dimensional representation. The function relating the baseline and profile data may be a ratio expressed as a logarithm. The constituent may be itemized on the x-axis and the logarithmic scale may be on the y-axis. Members of a calibrated data set may be expressed as a positive value representing a relative enhancement of gene expression or as a negative value representing a relative reduction in gene expression with respect to the baseline.

Each member of the calibrated profile data set should be reproducible within a range with respect to similar samples taken from the subject under similar conditions. For example, the calibrated profile data sets may be reproducible within one order of magnitude with respect to similar samples taken from the subject under similar conditions. More particularly, the members may be reproducible within 20%, and typically within 10%. In accordance with embodiments of the invention, a pattern of increasing, decreasing and no change in relative gene expression from each of a plurality of gene loci examined in the Gene Expression Panel (Precision Profile^{™}) may be used to prepare a calibrated profile set that is informative with regards to a biological condition, biological efficacy of an agent treatment conditions or for comparison to populations or sets of subjects or samples, or for comparison to populations of cells. Patterns of this nature may be used to identify likely candidates for a drug trial, used alone or in combination with other clinical indicators to be diagnostic or prognostic with respect to a biological condition or may be used to guide the development of a pharmaceutical or nutraceutieal through manufacture, testing and marketing.

The numerical data obtained from quantitative gene expression and numerical data from calibrated gene expression relative to a baseline profile data set may be stored in databases or digital storage mediums and may be retrieved for purposes including managing patient health care or for conducting clinical trials or for characterizing a drug. The data may be transferred in physical or wireless networks via the World Wide Web, email, or internet access site for example or by hard copy so as to be collected and pooled from distant geographic sites.

The method also includes producing a calibrated profile data set for the panel, wherein each member of the calibrated profile data set is a function of a corresponding member of the first profile data set and a corresponding member of a baseline profile data set for the panel, and wherein the baseline profile data set is related to the biological condition, e.g., disease, to be evaluated, with the calibrated profile data set being a comparison between the first profile data set and the baseline profile data set, thereby providing evaluation of the biological condition.

In yet other embodiments, the function is a mathematical function and is other than a simple difference, including a second function of the ratio of the corresponding member of first profile data set to the corresponding member of the baseline profile data set, or a logarithmic function. In such embodiments, the first sample is obtained and the first profile data set quantified at a first location, and the calibrated profile data set is produced using a network to access a database stored on a digital storage medium in a second location, wherein the database may be updated to reflect the first profile data set quantified from the sample. Additionally, using a network may include accessing a global computer network.

In an embodiment of the present invention, a descriptive record is stored in a single database or multiple databases where the stored data includes the raw gene expression data (first profile data set) prior to transformation by use of a baseline profile data set, as well as a record of the baseline profile data set used to generate the calibrated profile data set including for example, annotations regarding whether the baseline profile data set is derived from a particular Signature Panel and any other annotation that facilitates interpretation and use of the data.

Because the data is in a universal format, data handling may readily be done with a computer. The data is organized so as to provide an output optionally corresponding to a graphical representation of a calibrated data set.

For example, a distinct sample derived from a subject being at least one of RNA or protein may be denoted as PI. The first profile data set derived from sample PI is denoted Mj, where Mj is a quantitative measure of a distinct RNA or protein constituent of PI. The record Ri is a ratio of M and P and may be annotated with additional data on the subject relating to, for example, age, diet, ethnicity, gender, geographic location, medical disorder, mental disorder, medication, physical activity, body mass and environmental exposure. Moreover, data handling may further include accessing data from a second condition database which may contain additional medical data not presently held with the calibrated profile data sets. In this context, data access may be via a computer network.

The above described data storage on a computer may provide the information in a form that can be accessed by a user. Accordingly, the user may load the information onto a second access site including downloading the information. However, access may be restricted to users having a password or other security device so as to protect the medical records contained within. A feature of this embodiment of the invention is the ability of a user to add new or annotated records to the data set so the records become part of the biological information.

The graphical representation of calibrated profile data sets pertaining to a product such as a drug provides an opportunity for standardizing a product by means of the calibrated profile, more particularly a signature profile. The profile may be used as a feature with which to demonstrate relative efficacy, differences in mechanisms of actions, etc. compared to other drugs approved for similar or different uses.

The various embodiments of the invention may be also implemented as a computer program product for use with a computer system. The product may include program code for deriving a first profile data set and for producing calibrated profiles. Such implementation may include a series of computer instructions fixed either on a tangible medium, such as a computer readable medium (for example, a diskette, CD-ROM, ROM, or fixed disk), or transmittable to a computer system via a modem or other interface device, such as a communications adapter coupled to a network. The network coupling may be for example, over optical or wired communications lines or via wireless techniques (for example, microwave, infrared or other transmission techniques) or some combination of these. The series of computer instructions preferably embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (for example, shrink wrapped software), preloaded with a computer system (for example, on system ROM or fixed disk), or distributed from a server or electronic bulletin board over a network (for example, the Internet or World Wide Web). In addition, a computer system is further provided including derivative modules for deriving a first data set and a calibration profile data set.

The calibration profile data sets in graphical or tabular form, the associated databases, and the calculated index or derived algorithm, together with information extracted from the panels, the databases, the data sets or the indices or algorithms are commodities that can be sold together or separately for a variety of purposes as described in WO 01/25473.

### Index constriction

In combination, (i) the remarkable consistency of Gene Expression Profiles with respect to a biological condition across a population or set of subject or samples, or across a population of cells and (ii) the use of procedures that provide substantially reproducible measurement of constituents in a Gene Expression Panel (Precision Profile^{™}) giving rise to a Gene Expression Profile, under measurement conditions wherein specificity and efficiencies of amplification for all constituents of the panel are substantially similar, make possible the use of an index that characterizes a Gene Expression Profile, and which therefore provides a measurement of a biological condition.

An index may be constructed using an index function that maps values in a Gene Expression Profile into a single value that is pertinent to the biological condition at hand. The values in a Gene Expression Profile are the amounts of each constituent of the Gene Expression Panel (Precision Profile^{™}) that corresponds to the Gene Expression Profile. These constituent amounts form a profile data set, and the index function generates a single value-the index- from the members of the profile data set.

The index function may conveniently be constructed as a linear sum of terms, each term being what is referred to herein as a "contribution function" of a member of the profile data set. For example, the contribution function may be a constant times a power of a member of the profile data set. So the index function would have the form $I = Σ ⁢ {CiMi}^{P \left(i\right)} ,$
where I is the index, Mi is the value of the member i of the profile data set, Ci is a constant, and P(i) is a power to which Mi is raised, the sum being formed for all integral values of i up to the number of members in the data set. We thus have a linear polynomial expression. The role of the coefficient Ci for a particular gene expression specifies whether a higher ΔCt value for this gene either increases (a positive Ci) or decreases (a lower value) the likelihood of a biological condition, the ΔCt values of all other genes in the expression being held constant.

The values Ci and P(i) may be determined in a number of ways, so that the index I is informative of the pertinent biological condition. One way is to apply statistical techniques, such as latent class modeling, to the profile data sets to correlate clinical data or experimentally derived data, or other data pertinent to the biological condition. In this connection, for example, may be employed the software from Statistical Innovations, Belmont, Massachusetts, called Latent Gold^{®}.
Alternatively, other simpler modeling techniques may be employed in a manner known in the art. The index function for inflammation may be constructed, for example, in a manner that a greater degree of inflammation correlates with a large value of the index function. In a simple embodiment, therefore, each P(i) may be +1 or -1, depending on whether the constituent increases or decreases with increasing inflammation.

Just as a baseline profile data set, discussed above, can be used to provide an appropriate normative reference, and can even be used to create a Calibrated profile data set, as discussed above, based on the normative reference, an index that characterizes a Gene Expression Profile can also be provided with a normative value of the index function used to create the index. This normative value can be determined with respect to a relevant population or set of subjects or samples or to a relevant population of cells, so that the index may be interpreted in relation to the normative value. The relevant population or set of subjects or samples, or relevant population of cells may have in common a property that is at least one of age range, gender, ethnicity, geographic location, nutritional history, medical condition, clinical indicator, medication, physical activity, body mass, and environmental exposure.

As an example, the index can be constructed, in relation to a normative Gene Expression Profile for a population of healthy subjects, in such a way that a reading of approximately 1 characterizes normative Gene Expression Profiles of healthy subjects. Let us further assume that the biological condition that is the subject of the index is inflammation; a reading of 1 in this example thus corresponds to a Gene Expression Profile that matches the norm for healthy subjects. A substantially higher reading then may identify a subject experiencing an inflammatory condition. The use of 1 as identifying a normative value, however, is only one possible choice; another logical choice is to use 0 as identifying the normative value. With this choice, deviations in the index from zero can be indicated in standard deviation units (so that values lying between -1 and +1 encompass 90% of a normally distributed reference population. Since it was determined that Gene Expression Profile values (and accordingly constructed indices based on them) tend to be normally distributed, the 0-centered index constructed in this manner is highly informative. It therefore facilitates use of the index in diagnosis of disease and setting objectives for treatment.

As another embodiment of the invention, an index function I of the form $I = {C}_{0} + Σ {C}_{i} ⁢ {{M}_{1 ⁢ i}}^{P ⁢ 1 \left(i\right)} ⁢ {{M}_{2 ⁢ i}}^{P ⁢ 2 \left(i\right)} ,$
can be employed, where M₁ and M₂ are values of the member i of the profile data set, Cᵢ is a constant determined without reference to the profile data set, and P1 and P2 are powers to which M₁ and M₂ are raised. The role of P1(i) and P2(i) is to specificy the specific functional form of the quadratic expression, whether in fact the equation is linear, quadratic, contains cross-product terms, or is constant. For example, when P1 = P2 = 0, the index function is simply the sum of constants; when P1 = 1 and P2 = 0, the index function is a linear expression; when P1 = P2 =1, the index function is a quadratic expression.

The constant C₀ serves to calibrate this expression to the biological population of interest that is characterized by having a biological condition, such as for illustrative purposes and without limitation, inflammation or an inflammatory related disease. In this embodiment, when the index value equals 0, the odds are 50:50 of the subject having inflammation vs a normal subject. More generally, the predicted odds of the subject having inflammation is [exp(Iᵢ)], and therefore the predicted probability of having inflammation is [exp(Iᵢ)]/[1+exp((Iᵢ)]. Thus, when the index exceeds 0, the predicted probability that a subject has inflammation is higher than .5, and when it falls below 0, the predicted probability is less than .5.

The value of C₀ may be adjusted to reflect the prior probability of being in this population based on known exogenous risk factors for the subject. In an embodiment where Co is adjusted as a function of the subject's risk factors, where the subject has prior probability pᵢ of having inflammation based on such risk factors, the adjustment is made by increasing (decreasing) the unadjusted C₀ value by adding to C₀ the natural logarithm of the ratio of the prior odds of having inflammation taking into account the risk factors to the overall prior odds of having inflammation *without* taking into account the risk factors.

### Kits

The invention also includes a biological condition detection reagent, i.e., nucleic acids that specifically identify one or more biological conditions (e.g., any gene listed in Tables 1-9), by having homologous nucleic acid sequences, such as oligonucleotide sequences, complementary to a portion of the nucleic acids encoding a disease related gene or antibodies to proteins encoded by the nucleic acids encoding disease related genes, packaged together in the form of a kit. The oligonucleotides can be fragments of the disease related. For example the oligonucleotides can be 200, 150, 100, 50, 25, 10 or less nucleotides in length. The kit may contain in separate containers a nucleic acid or antibody (either already bound to a solid matrix or packaged separately with reagents for binding them to the matrix), control formulations (positive and/or negative), and/or a detectable label. Instructions (i.e., written, tape, VCR, CD-ROM, etc.) for carrying out the assay may be included in the kit. The assay may for example be in the form of PCR, a Northern hybridization or a sandwich ELISA, as known in the art.

For example, inflammatory disease genes detection reagents can be immobilized on a solid matrix such as a porous strip to form at least one disease related gene detection site. The measurement or detection region of the porous strip may include a plurality of sites containing a nucleic acid. A test strip may also contain sites for negative and/or positive controls. Alternatively, control sites can be located on a separate strip from the test strip. Optionally, the different detection sites may contain different amounts of immobilized nucleic acids, i.e., a higher amount in the first detection site and lesser amounts in subsequent sites. Upon the addition of test sample, the number of sites displaying a detectable signal provides a quantitative indication of the amount of inflammatory disease genes present in the sample. The detection sites may be configured in any suitably detectable shape and are typically in the shape of a bar or dot spanning the width of a test strip.

Alternatively, inflammatory disease detection genes can be labeled (e.g., with one or more fluorescent dyes) and immobilized on lyophilized beads to form at least one inflammatory gene detection site. The beads may also contain sites for negative and/or positive controls. Upon addition of the test sample, the number of sites displaying a detectable signal provides a quantitative indication of the amount of inflammatory disease genes present in the sample.

Alternatively, the kit contains a nucleic acid substrate array comprising one or more nucleic acid sequences. The nucleic acids on the array specifically identify one or more nucleic acid sequences represented by disease genes (see Tables 1-9). In various embodiments, the expression of 1, 2, 3, 4, 5, 6, 7, 8, 9, 10, 15, 20, 25, 40 or 50 or more of the sequences represented by inflammatory disease genes (see Tables 1-9) can be identified by virtue of binding to the array. The substrate array can be on, i.e., a solid substrate, *i.e*., a "chip" as described in U.S. Patent No. 5,744,305. Alternatively, the substrate array can be a solution array, i.e., Luminex, Cyvera, Vitra and Quantum Dots' Mosaic.

The skilled artisan can routinely make antibodies, nucleic acid probes, i.e., oligonucleotides, aptamers, siRNAs, antisense oligonucleotides, against any of inflammatory disease related genes listed in Tables 1-9.

### Other Embodiments

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EXAMPLES

### Example 1: Mouse Gene Expression Analysis in Whole Blood

### Assay 1

Whole blood samples from five male and five female BALB/c mice were collected on a weekly basis over the course of three weeks to evaluate the longitudinal gene expression response in murine whole blood. Gene expression analysis was performed by quantitative PCR (QPCR) using a custom 24-gene Mouse Expression Panel (Precision Profile™) for Inflammation (Table 3).

Normalized ΔCt values for all ten mice (BALB/c) over a 3 week time period are provided in Table 10.

Inter-subject variability within individual groups was determined by the %CV (coefficient of variation) from all ten animals on a weekly basis (Table 10). Percent CV's were observed to be less than 4%, with the exception of one gene, PLAU, at week 2. These data demonstrate a remarkable consistency in levels of gene expression within the 10 animals in each group on a weekly basis for all three weeks.

Intersubject variability for all three groups was also determined by the %CV from all ten animals over the total three week time period. Percent CV's were again observed to be less than 4%, with the exception of one gene, MMP9 (Table 10). These data demonstrate a remarkable consistency in levels of gene expression within the 10 animals over a three week period, not unexpectedly since the intersubject variability observed on a weekly basis was small.

A comparison of mean ΔCt values for each gene at weeks 1, 2 and 3 were quite similar, differing on average by less than 1 Ct, which is also consistent with the small % CV's observed across all groups.

### Assay 2

Male Swiss Webster mice were challenged with LPS bacterial endotoxin after a one hour pretreatment with either vehicle or dexamethasone. Whole blood samples were collected at 1.5, 4 and 24 hours post-treatment with LPS, to evaluate the gene expression response. In addition, baseline whole blood samples were collected from untreated mice: Gene expression analysis was performed by quantitative PCR (QPCR) to measure gene expression levels of constituents selected from from a custom 24-gene Mouse Expression Panel (Precision Profile™) for Inflammation (Table 3), thereby establishing a preliminary LPS Endotoxin (+/- Dexamethasone) mouse response profiles from whole blood.

Normalized ΔCt values for all mice in each of the seven groups are provided in Table 11. Intersubject variability within individual groups was determined by the %CV (coefficient ofvariation) from all animals in each group (Table 11). The %CV values demonstrate a slightly more variable response to the LPS and Dexamethasone + LPS challenge across each group, respectively.

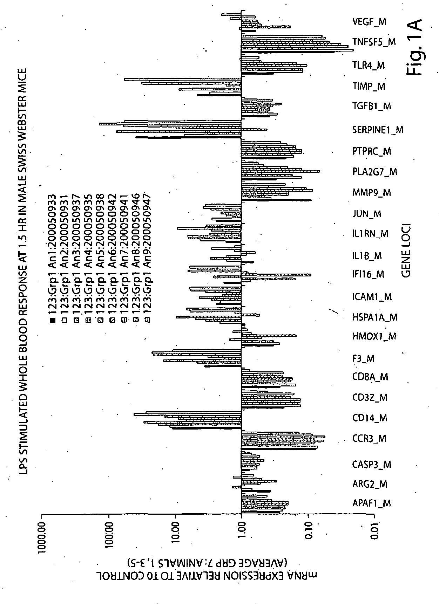

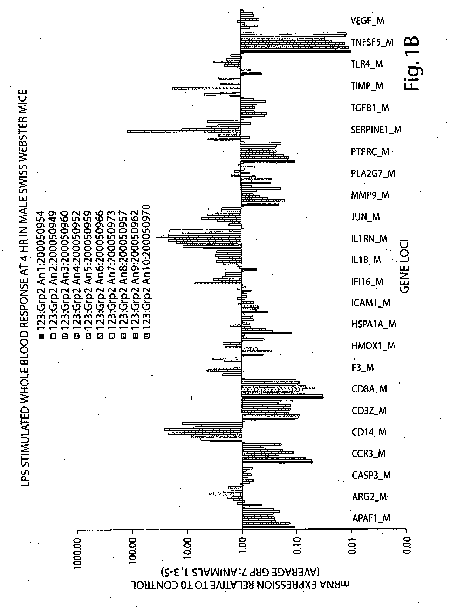

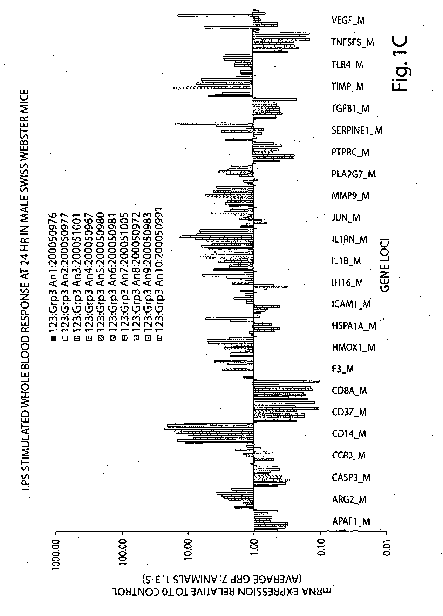

The gene expression response of LPS challenged animals at all time points compared to that of the untreated, To Control animals is provided in Table 12 and Figures 1A-1C. Figures 1A through 1C show LPS-stimulated whole blood response at 1.5 hr (Group 1), 4 hr (group 2) and 24 hr (Group 3), respectively, in three groups of male Swiss Webster mice for a 24-gene expression panel for Inflammation (Table 3). The relative gene expression response for all animals within each of the three groups was very uniform at all time points, though the magnitude of response was variable.

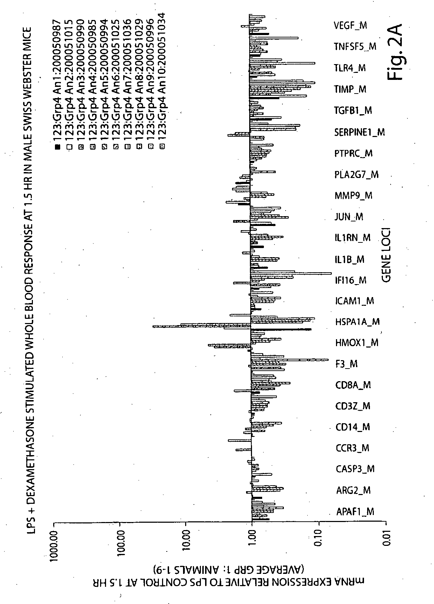

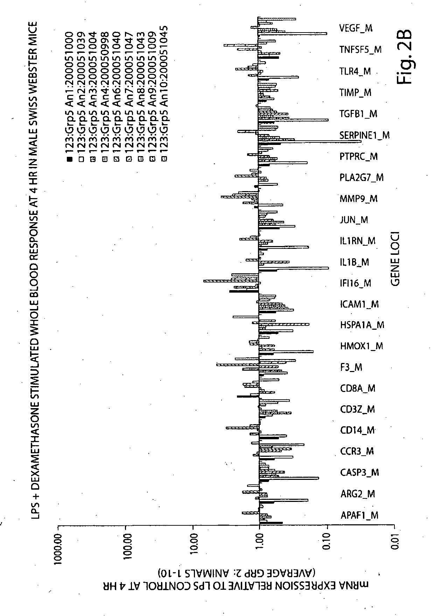

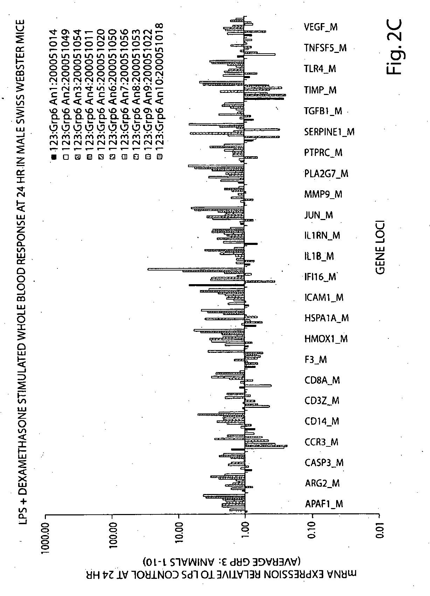

The gene expression response of Dexamethasone + LPS challenged animals compared to that of the LPS treated, time-matched animals is provided in Table 13 and Figures 2A-2C. Figures 2A-2C show LPS + Dexamethasone-stimulated whole blood response at 1.5 hr (Group 4), 4 hr (Group 5) and 24 hr (Group 6), respectively, in three groups of male Swiss Webster mice for the 24-Gene Expression Panel for Inflammation (Table 3).

The relative gene expression response for all animals within two of the groups was quite uniform (1.5 and 24 hr time points), though the magnitude of response was variable. The relative response was observed to be slightly more variable at the 4 hour time point.

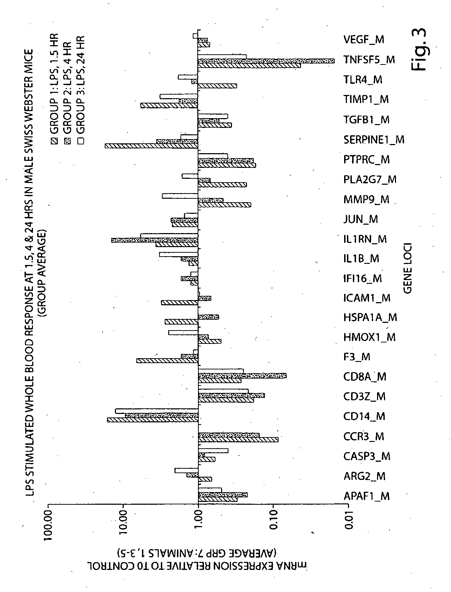

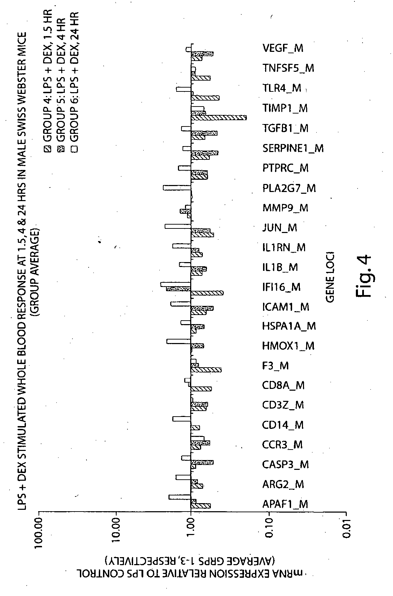

The group averaged relative gene expression response for LPS and LPS + Dexamethasone challenged animals is provided in Tables 14 and 15 and Figures 3 & 4, respectively. The group-averaged gene expression response for the LPS challenge, shown in Fig. 3, demonstrates a discernable time course of response for many genes, post-treatment with LPS. The group-averaged gene expression response for the Dexamethasone + LPS challenge, shown in Fig. 4, demonstrates a somewhat limited response from Dexamethasone pre-treatment prior to LPS challenge for all three time points.

Based on these studies, preliminary mouse response profiles to LPS stimulation, and LPS + Dexamethasone stimulation in whole blood were derived, as shown in Tables 4, 5, 6, 7, and 8 below.

### Example 2: Gene Expression in Human and Mouse Whole Blood stimulated with LPS in vivo

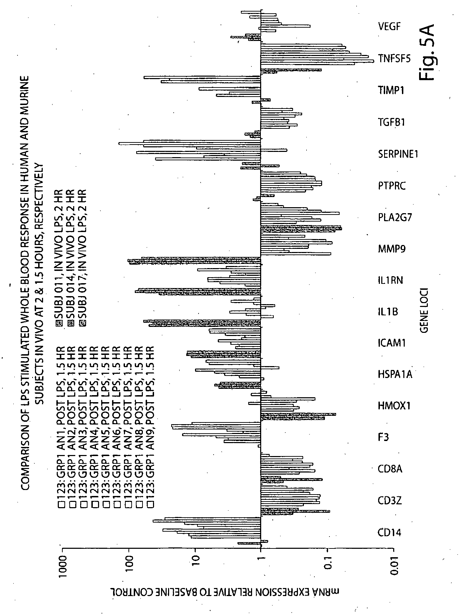

The gene expression response in whole blood from human (N=3) and murine (N=9-10) subjects exposed to a single dose of bacterial endotoxin (lipopolysaccharide, LPS) is presented in Table 16 and Figures 5A-5C. Whole blood samples were collected at three time points post LPS dosing for all subjects. A comparison of the human and murine response relative to that of the untreated baseline control is collectively shown in Figures 5A-5C.

The relative gene expression response of human and murine whole blood at 2 and 1.5 hours post LPS is shown in Fig 5A for 17 genes. The pattern of response for 9 of the 17 genes, specifically, CD3Z, CD8A, HMOX1, HSPA1A, ICAM1, IL1RN, PLA2G7 SERPINE1 and TNFSF5, is very similar between human and murine subjects, though the magnitude of response for some of these genes is variable. Two genes, MMP9 and TGBF1 show a divergent response at these time-points. The remaining genes differ slightly in the magnitude of response or are somewhat variable in the pattern of response.

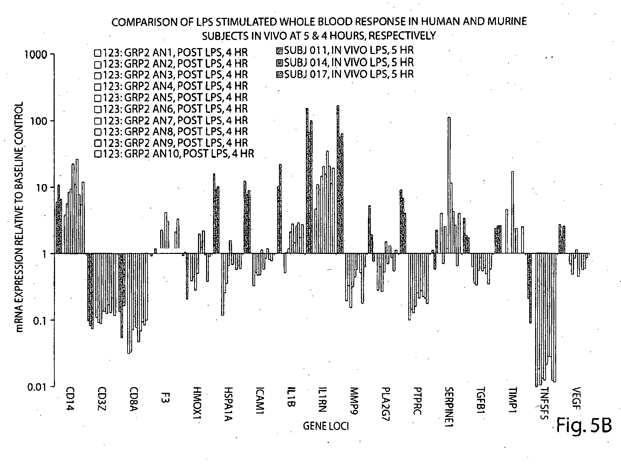

Interestingly, some of the genes that exhibited a similar pattern and magnitude of response at the earlier 2 hour time-point show a divergent response at the later 5 and 4 hour time-point, such as HSPA1A, ICAM1 and PLA2G7 (Fig. 5B). In addition and in contrast to the earlier time-point, CD14 and TIMP1 now exhibit a similar pattern and magnitude of response at this later time-point for both human and murine subjects.

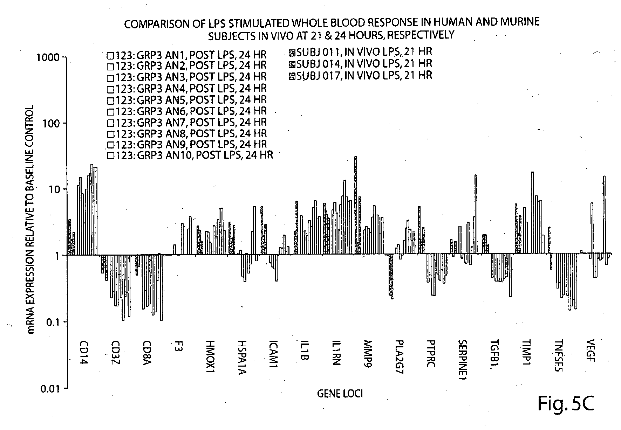

The magnitude of gene expression response at the 21 and 24 hour timepoints (Fig. 5C), has diminished for the majority of genes in both human and murine subjects, returning toward baseline levels of expression in many instances. Of interest is the change in pattern of expression observed for HMOX1 (both human and murine) and MMP9 (murine) compared to the two earlier time points. In summary, the gene expression response of whole blood to LPS treatment *in vivo* for human and murine subjects exhibit a similar pattern and magnitude of response for many proinflammatory genes over a 24 hour time course.

### Example 3: Gene Expression in Human Whole Blood Stimulated with LPS in vivo and in vitro

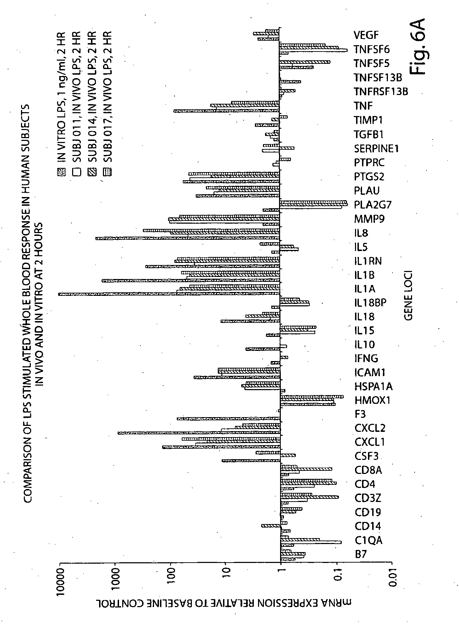

The gene expression response in whole blood from human subjects (N=3) exposed to a single dose of bacterial endotoxin (lipopolysaccharide, LPS) *in vivo* and human whole blood treated with LPS *in vitro* (N=1), is presented in Table 17 and Figures 6A-6C. Whole blood samples were collected at three time points post LPS dosing for all subjects. A comparison of the *in vivo* and *in vitro* response relative to that of the untreated baseline control is collectively shown in Figures 6A-6C.

At the 2 hour time-point, 21 of 38 genes show a strikingly similar pattern of expression for both *in vivo* and *in vitro* samples (Fig 6A) for 31 the 31 genes examined. For most genes, the magnitude of expression of the *in vitro* sample is greater than that observed *in vivo*. A few differences in expression can also be noted, specifically, the genes CSF3, F3 and IL10 are induced *in vitro* and remain unchanged *in vivo.*

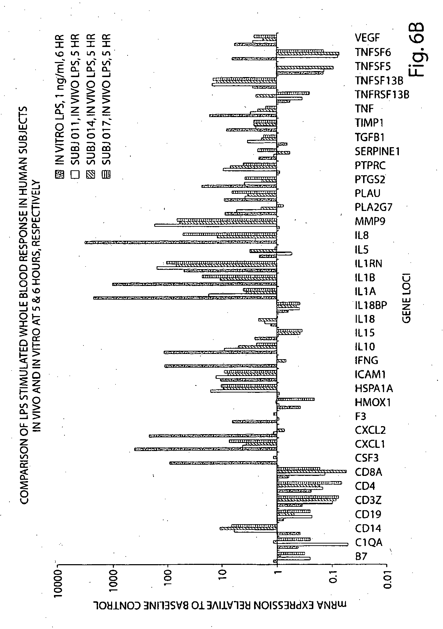

The magnitude of response for many genes, such as CXCL1, CXCL2, HMOX1, IL1A and PLA2G7 has diminished significantly at the 5 hour time point for the *in vivo* samples in contrast to the *in vitro* sample (Fig 6B).

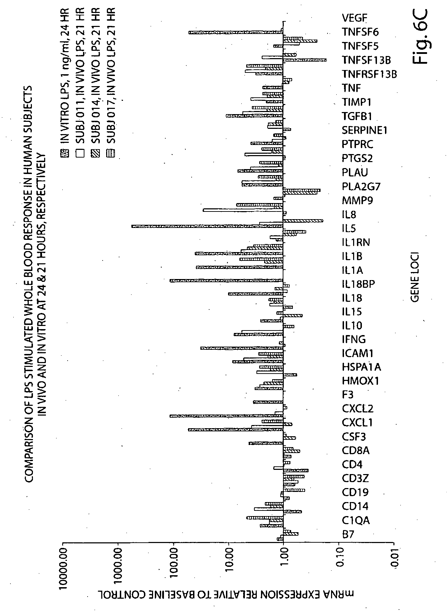

Finally, by 21-24 hours post dose, the levels of expression have returned to near baseline for the *in vivo* samples and have continued to decrease for the *in vitro* sample, though higher levels of expression may still be observed for many genes, especially for VEGF (Fig. 6C). In summary, the pattern of gene expression response in whole blood from LPS stimulation is strikingly similar at early time points for *in vivo* and *in vitro* samples. The magnitude of response at later time points is significantly decreased more rapidly for *in vivo* samples compared with *in vitro* samples.

### Example 4: Gene Expression in LPS Treated Whole Blood from murine (in vivo) and Human (in vitro) subjects

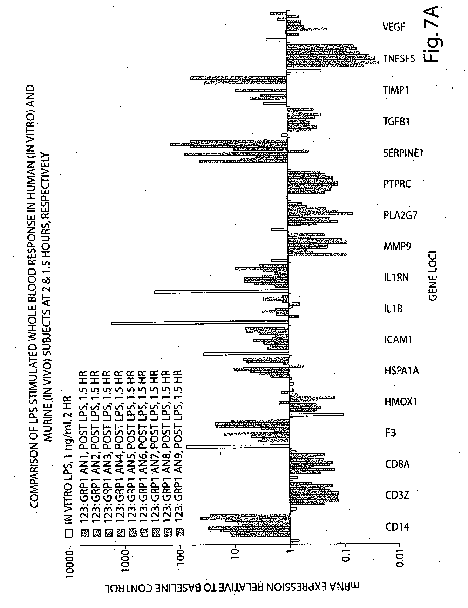

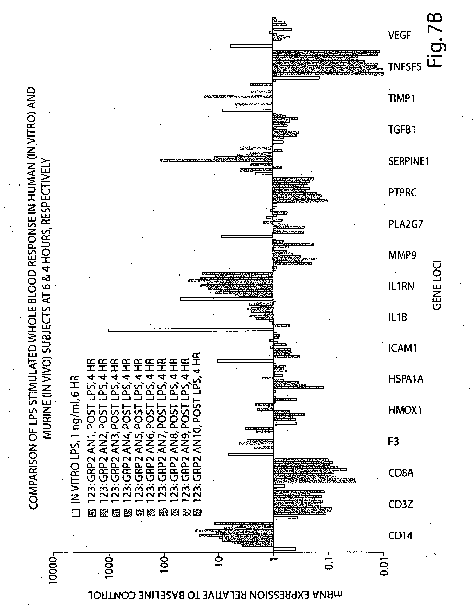

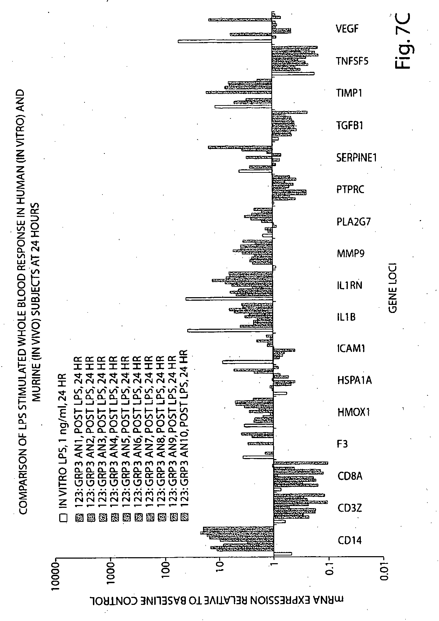

The gene expression response in whole blood from murine (N=9-10) and human (N=1) subjects exposed to a single dose of bacterial endotoxin (lipopolysaccharide, LPS) *in vivo* and *in vitro,* respectively, is presented in Table 18 and Figures 7A-7C. Whole blood samples were collected at three time points post LPS dosing for all subjects. A comparison of the murine and human response relative to that of the untreated baseline control is collectively shown in Figures 7A-7C, for the same 17 genes examined in Figures 5A - 5C. The pattern of gene expression response observed for the murine (*in vivo*) and human (*in vitro*) subjects is similar for 8 of the 17 genes at the early 1.5 and 2 hour time point, including CD3Z, CD8A, HOMX1, F3, ICAM1, IL1RN, TIMP1 and TKNFSF5. The magnitude of response is variable, depending upon the sample type and gene itself. With the exception of HSPA1A and ICAM1, the pattern of response had not changed significantly at the later 4 and 6 hour time points.

Finally, the magnitude of expression is approaching baseline levels by the 24 hour time point, with the exception of VEGF. In summary, the gene expression response for LPS treated whole blood from murine (*in vivo*) and human (*in vitro*) revealed a subset of genes exhibiting a similar pattern of response. This subset of genes varied slightly from those defined in Figures 5A-5C, comparing the *in vivo* LPS challenged human subjects.

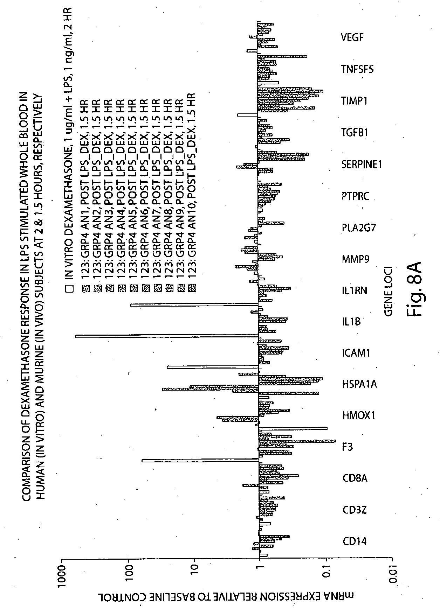

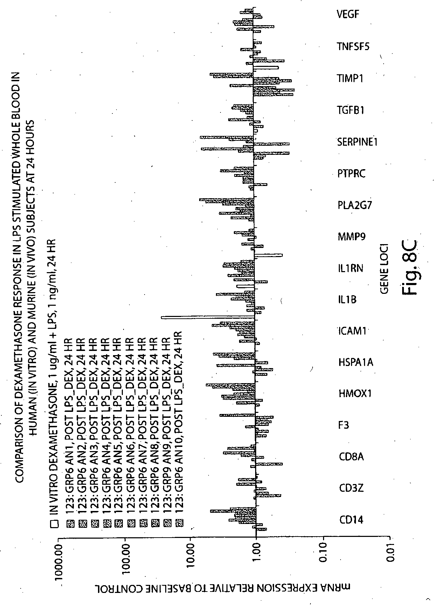

The gene expression response in whole blood from murine (N=9-10) and human (N=1) subjects exposed to a single dose of bacterial endotoxin (lipopolysaccharide, LPS) following prior treatment with Dexamethasone, *in vivo* and *in vitro* respectively, is presented in Table 19 and Figures 8A-8C. Whole blood samples were collected at three time points post LPS dosing for all subjects. A comparison of the murine and human response relative to that of the untreated baseline control is collectively shown in Figures 8A-8C, for the same 17 genes as examined in Figs. 5A - 5C and 7A- 7C.

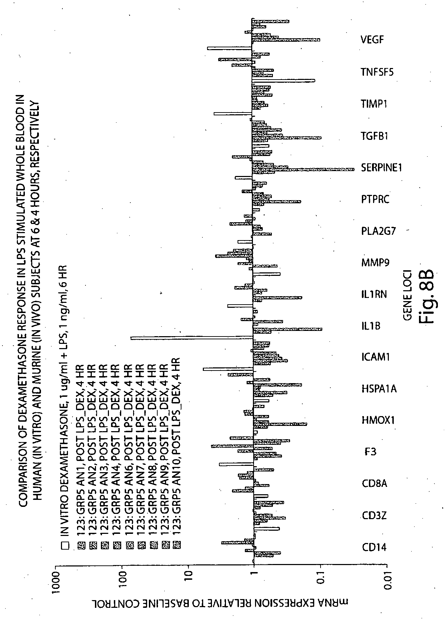

The gene expression response for the murine subjects at the 1.5 and 4 hour time-points is very similar for the majority of genes, and shows a significantly diminished induction (Figs. 8A-8B) when compared to LPS treatment alone (Figs. 7A-7B). This is also true for the human *in vitro* whole blood sample, which shows a significant reduction in the expression of most genes, especially at the 6 hour time-point. A similar pattern of expression between the two sample types can be observed in Figure 8B for many of the genes - i.e: induction is in a downward direction.

### Example 5: High Precision Gene Expression Analysis of Two Murine Models of Arthritis

Whole blood gene expression changes were evaluated in vehicle control and drug-treated murine subjects across two murine models of arthritis according to the following arthritis models:
KRN Transgenic Mouse (K/BxN): K/BxN serum from transgenic mouse used to induce arthritis in female BALB/c mice (n=54).
Collagen Induced Arthritis (CIA): Bovine type II collagen used to induce arthritis in male DBA/1 mice (n=54).

Cohorts of 6 animals in the CIA and KRN arms of the study were treated with either vehicle control or dexamethasone at multiple time points post induction of arthritis to assess disease progression and response to dexamethasone treatment. In addition, naïve, untreated animal groups at baseline and terminal day were included to control for potential age-related changes in gene expression over the extended study periods. Animal groups, time-points, and treatment schedules are summarized in Tables 20A and 20B, respectively.

Whole blood samples from animals were collected by retro-orbital bleed at selected time-points in accordance with the arthritis study schemas described in Tables 10A and 10B, and transferred into sample collection tubes containing a 1.5X lysis solution and RPMI. Samples were processed into total RNA and cDNA. Gene expression analysis was performed by QPCR using a custom murine 40-gene Precision Profile^{™} for Rheumatoid Arthritis (Table 9), providing a molecular characterization of disease in CIA and KRN murine models of arthritis and response to dexamethasone treatment. It was anticipated from these murine models would provide a better understanding of the relevant molecular response of arthritis induction and their potential correlation to the human disease condition.

### Normal Murine Subject Assessment: CIA and KRN Study Arms

Normal murine subject reference values (represented as normalized Ct or ΔCt values) were established for male DBA/1 mice (n=6 for the CIA study arm) and female BALB/c mice (n=6 for the KRN study arm). In both arms of the study, gene expression response of normal (naïve, non-immunized and untreated) murine subjects were evaluated in groups of 6 each at day 0 and day 60 for the CIA study arm, and at day 0 and day 21 for the KRN study arm (see Tables 20A and 20B).

### Intra-Day and Inter-Day variability CIA Arm, Normal Murine Subjects

Variability, within groups of normal (naïve, non-immunized and untreated) male DB/1 mice at days 0 (intra-day, n=6) or day 60 (intra-day, n=6) was observed to be tight (<5% coeffiecient of variation (CV)) for most target genes, as shown in Table 21A and Table 22. Several target genes, including F3 and VEGF, were observed to have higher variability (>5% CV) however, no target genes were observed to have %CVs greater than seven (note that F3 is a low to non-expressing target gene, consequently higher variability can be expected across individual mice).

Variability across these same groups of normal male DBA/1 mice at days 0 and day 60 (inter-day, n=12), was observed to be tight (<5% CV), with some exceptions, including ARG2, CSPG2, F3, IL1RAP, and VEGF, as indicated in Table 21A and Table 22, reflecting the higher cohort variability or the moderate difference in the ΔCt value between cohorts. It is noteworthy that a comparison of the average ΔCt values for the day 0 and day 60 mouse groups revealed ΔCt differences greater than 0.5 Ct for most target genes.

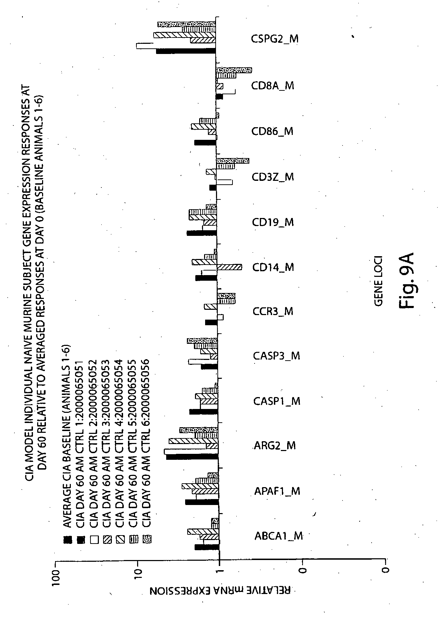

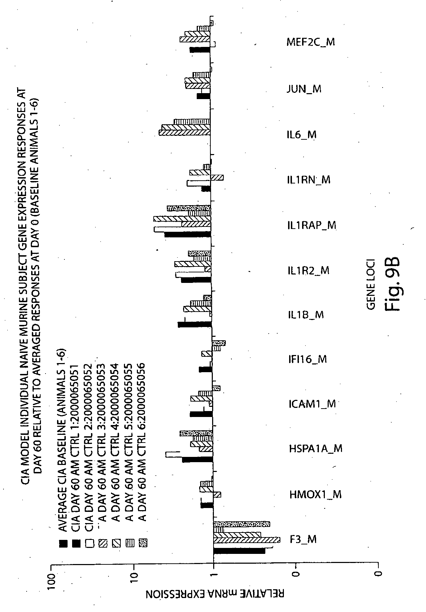

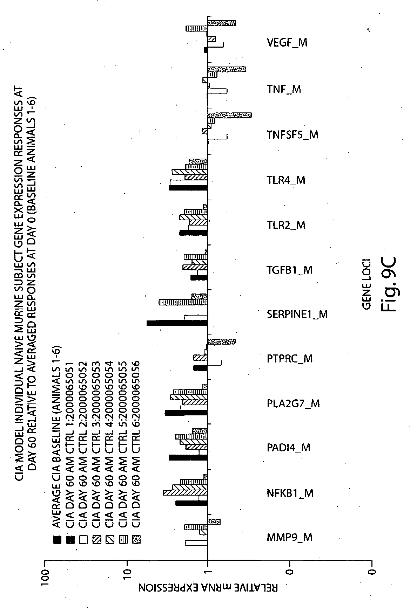

Alternatively, when comparing individual gene expression responses of the normal male DBA/1 mice at day 60 relative to the averaged normal male DBA/1 mice at day 0, consistently increased expression (primarily <6-fold) was observed across all DBA/1 mice in multiple target genes, as shown in Figures 9A-9C (and Table 24). Decreased expression was also observed in some select target genes, however this was not typically found to be consistent across all DBA/1 mice. (Note that F3 expression is somewhat variable (low of off) within the mouse groups at day 0 and day 60, therefore at the relative expression level, the observed decreased expression of F3 across all DBA/1 mice (as shown in Figure 9B) should be interpreted with caution). Without intending to be bound by any theory, these results may support potential age-related changes in gene expression over the 60-day study period.

### Intra-Day and Inter-Day variability: KRN Arm, Normal Murine Subjects

Variability within groups of normal (naïve, non-immunized, and untreated) female BALB/c mice at days 0 (intra-day, n=6) or day 21 (intra-day, n=6), was observed to be tight (<5% CV) for all target genes, as shown in Table 21B and Table 23.

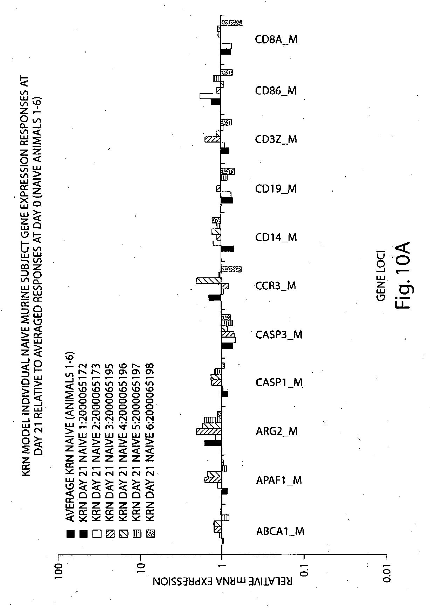

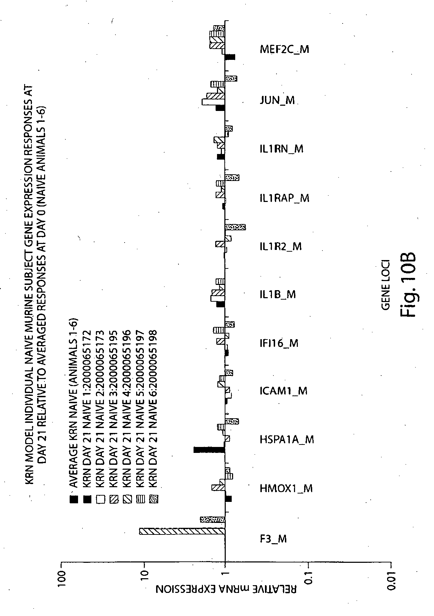

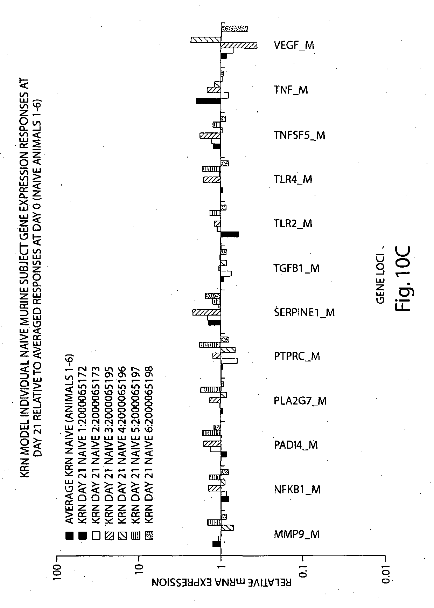

Variability across these same groups of normal female BALB/c mice at days 0 and 21 (inter-day, n=12) was observed to be equally tight, as shown in Table 21B and Table 23. A comparison of the average ΔCt values for the day 0 and day 21 mouse groups revealed differences well under 0.5 Ct for all target genes with the exception of ARG2 and SEPRINE (average ΔCt differences of 0.61 and 0.55 respectively, as shown in Table. 21B and Table 23) (note that F3 is a low to non-expressing target gene, consequently higher variability can be expected across individual mice). Furthermore, a comparison of individual gene expression responses of the normal femal BALB/c mice at day 21 relative to the averaged normal female BALB/c mice at day 0 revealed little differences in gene expression (<2-fold) across all target genes as shown in Figures 10A-10C (and Table 25). These results support a consistency of the mouse groups as the molecular level over this shorter 21-day study period.

### CIA Arthritis Model: Disease Progression and Response to Dexamethasone Treatment

CIA model male DBA/1 mice were either untreated, vehicle-treated, or dexamethasone-treated according to the study scheme shown in Table 20A. Gene expression responses for this arthritis-induced murine model were evaluated for untreated mice at day 24 and vehicle-treated mice at days 33, 42, and 60 to characterize disease progression. Similarly, gene expression responses were evaluated for dexamethasone-treated mice at days 33, 42, and 60 to assess response to dexamethasone treatment.

### Untreated and Vehicle-Treated DBAl1 Mice: Assessment ofDisease Progression:

Type II collagen-induction of arthritis in male DBA/1 mice (both untreated at day 24 and vehicle-treated at days 33, 42, and 60) resulted in substantial and consistent changes in-gene expression relative to the averaged normal baseline group (naïve, non-immunized, and untreated at day 0), as shown in Figures 11A-11C (and Table 26).

Multiple target genes, including APAF1, ARG2, CASP3, CD14, CSPG2, IL1B, IL1R2, IL1RAP, MMP9, PAD14, PLA2G7, TGFB1, TLR2, and TLR4 exhibited sustained induction of expression that was consistent across all DBA/1 mice and study time-points. A small subset of genes (IL1RAP, MMP9, PAD14, and PLA2G7) exhibited slightly decreased levels of expression at the later study time-points.

In contrast, several target genes such as CD3Z, CD8A, F3, IFI16, TNFSF5 and TNF exhibited a pattern of suppression over the study course. However, this was not necessarily consistent across all DBA/1 mice within a study time-point. Overall, these studies show the molecular profile for CIA is characterized by consistent and substantial gene expression responses that were maintained over the course of the study. These results are consistent with previous studies of CIA in female DBA/1 mice (with and without LPS boost).

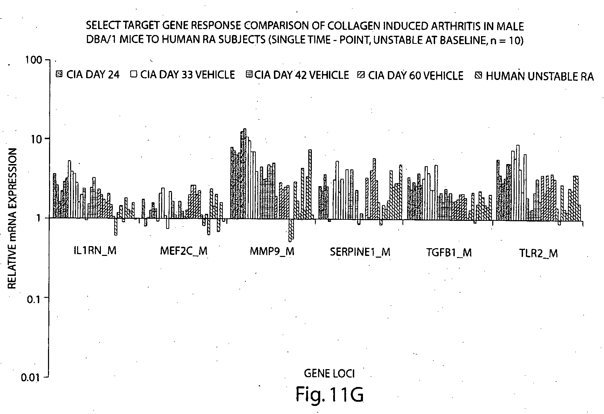

This CIA time-course of response was compared to 10 human unstable RA subjects that failed DMARD therapy and were about to be transitioned to anti-TNF therapy (study not shown). A direct comparison of the induced arthritis in murine subjects over the 60 day period to a single time-point measurement in human RA subjects for select target genes is provided in Figures 11F and 11G (species specific (human and murine) primer-probes were designed and used in these studies). The translation across species and time-points is striking in this limited comparison. This begins to provide some preliminary insights into the correlation of arthritis induction in murine subjects to the human disease condition at the molecular level.

### Drug-Treated DBA/1 Mice: Assessment ofResponse to Dexamethasone

Dexamethasone treatment in male DBA/1 mice was initiated after symptoms of arthritis had been well established. Response to dexamethasone treatment in DBA/1 mice with established arthritis was assessed by comparing these drug-treated male DBA/1 mice to their vehicle-treated counterparts.

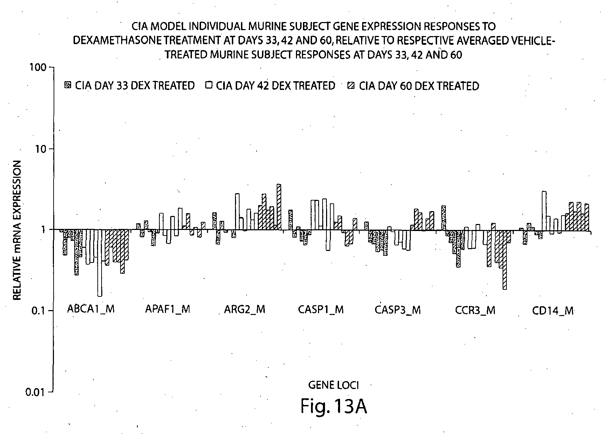

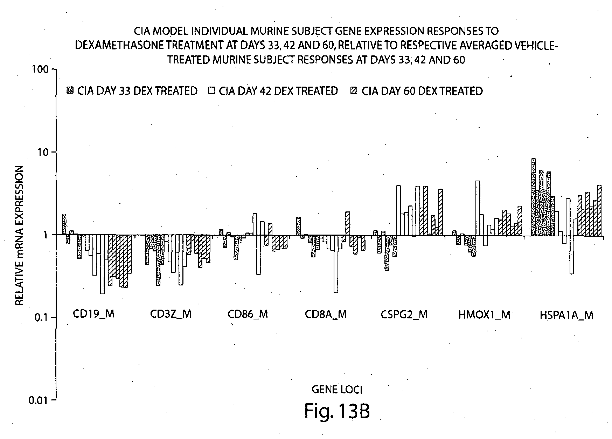

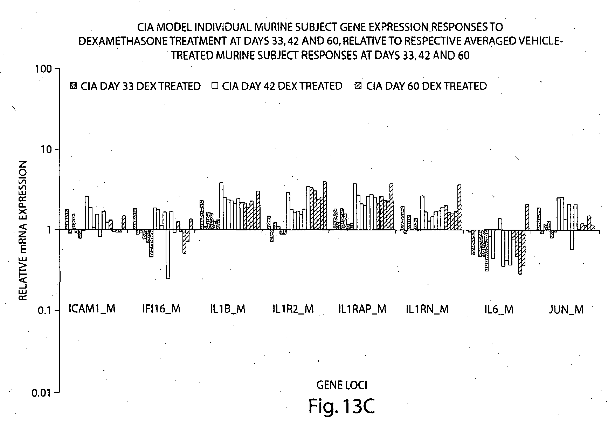

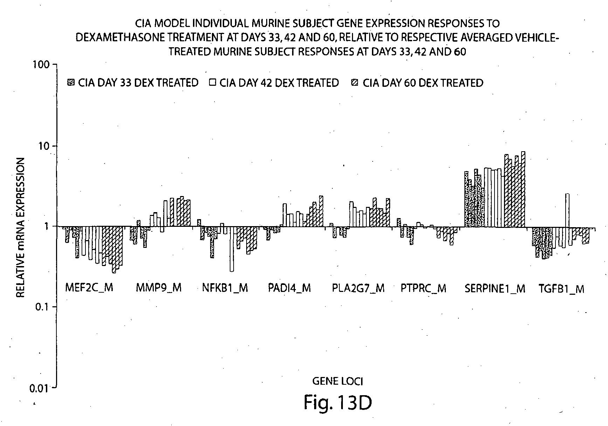

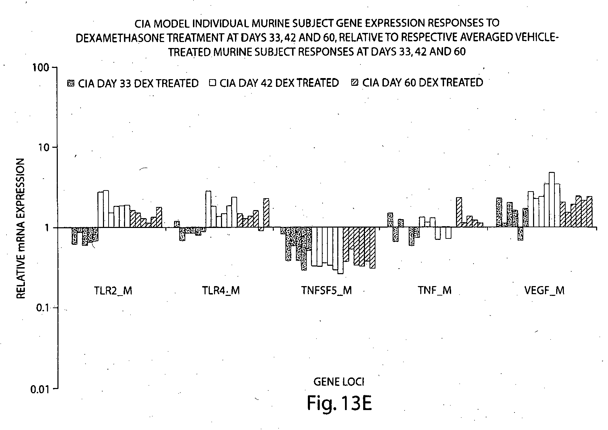

Individual DBA/1 mouse gene expression responses to dexamethasone treatment at days 33, 42, and 60 relative to their respective vehicle-treated controls at days 33, 42, and 60 are provided in Figures 13A-13E (and Table 28). Uniformity or variability of gene expression response to dexamethasone treatment across murine subjects was target gene and time-point dependent.

Dexamethasone treatment was observed to block select target gene expression, including ABCA1, CD3Z, MEF2C, NFKB1, TGFB1 and TNFSF5, across time-points and murine subjects (with CD19 blocking at the later time-points). In contrast, dexamethasone treatment was observed to increase expression consistently across all time-points and murine subjects in other target genes, such as IL1B, IL1RAP, and SERPINE1

Additional target genes exhibited uniformity of response in blocking effect or increased expression that was more time-point specific. For example, HSAP1A exhibited uniformly increased expression at days 33 and 60, with more variable responses at day 33. Dexamethasone treatment consistently blocked TLR2 expression at day 33, yet consistent increased expression was observed by days 42 and 60. Despite some individual murine subject variability, a trend similar to TLR2 can be observed for other target genes such as CSPG2, HMOX1, MMP9, PAD14 and PLA2G7.

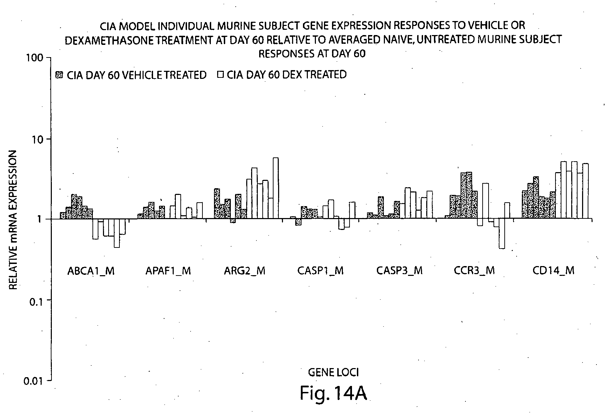

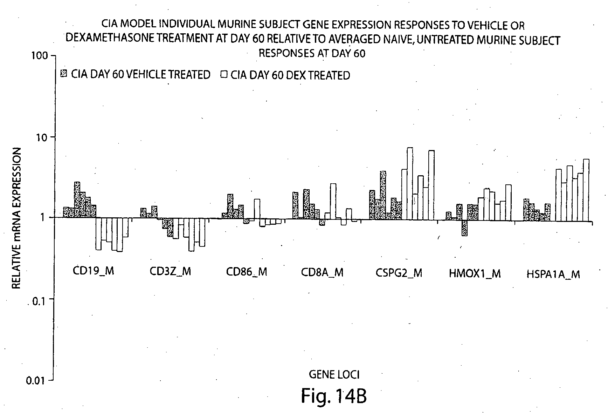

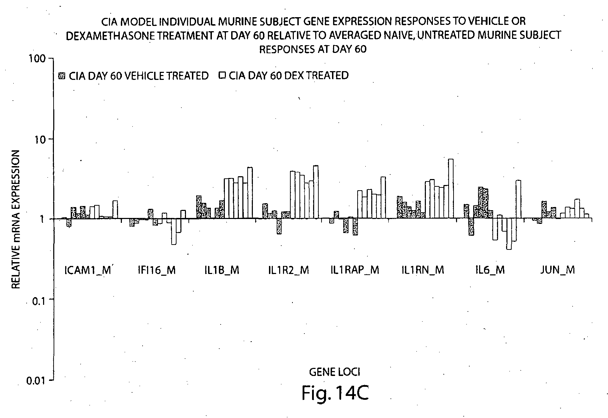

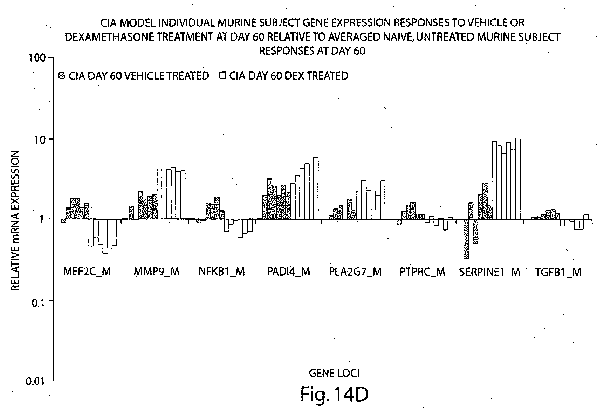

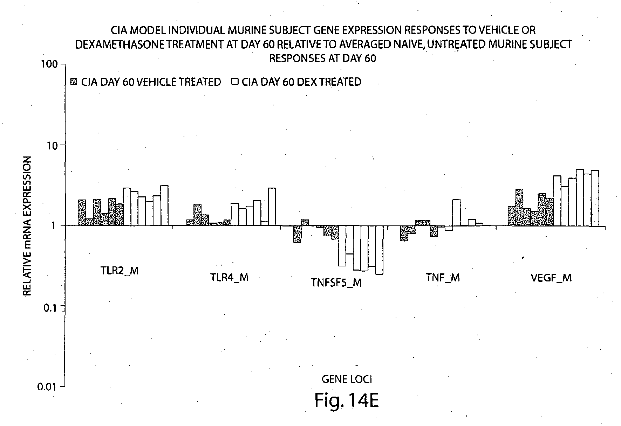

Given the availability of untreated, vehicle-treated, and dexamethasone-treated DBA/1 mouse groups at day 60, a comparison of the treated relative to untreated DBA/1 mouse groups at this terminal day was made. Individual DBA/1 mouse gene expression responses to vehicle or dexamethasone treatment relative to the average, time-matched normal group (naïve, non-immunized and untreated at day 60) is provided in Figures 14A-14E (and Table 29). This additional relative expression in view further supports previous observeations made from comparisons of dexamethasone-treated DBA/1 mice relative to their vehicle-treated counterparts (Figures 5a-5e) at this specific time-point (day 60).

### KRN Arthritis Model: Disease Progression and Response to Dexamethasone Treatment

KRN model female BALB/c mice were either untreated, vehicle-treated, or dexamethasone treated according to the study scheme shown in Table 20B. Gene expression responses for the arthritis-induced murine model were evaluated for untreated mice at day 3 and vehicle-treated mice at days 7, 14 and 21 to characterize disease progression. Similarly, gene expression responses were evaluated for dexamethasone-treated mice at days 7, 14, and 21 to assess response to dexamethasone treatment.

### Untreated and Vehicle Treated BALB/c Mice: Assessment ofDisease Progression

Serum transfer induction of arthritis in female BALB/c mice (both untreated at day 3 and vehicle-treated at days 7, 14, and 21) resulted in modest, albeit consistent changes in gene expression relative to the normal (naive, non-immunized and untreated at day 0) baseline group as shown in Figures 12A-12E (and Table 27). Overall, gene expression responses were observed to be very time-dependent. In some cases, target genes such as CASP3, CSPG2, HMOX1, MMP9, PADI4 and TLR2 showed a pattern of consistent induction of gene expression at days 3, 7, and 14, followed by suppression of gene expression by day 21. In other cases, many target genes such as APAF1, CASP1, CD3Z, CD86, CD8A, ICAM1, IFI16, IL1B, NFKB1, PTPRC, TLR4, TNFSF5, and TNF exhibited patterns of early suppression (day 3), followed by consistent patterns of induction by days 7 and 14 with a return towards suppression by day 21.

The molecular profile for the serum transfer induction of arthritis is characterized here by more moderate gene expression responses with distinct inflections in response (decreased to increased expression, and vice versa) over the shorter study course. Again, these results are consistent with previous studies (data not shown) of serum transfer induction of arthritis in a different strain of mice (female DBA/1).

As previously done in the CIA model, this serum transfer model of arthritis induction was compared to the human RA condition using the study of 10 human unstable RA subjects that failed DMARD therapy and were about to be transitioned to anti-TNF therapy. A direct comparison of the induced arthritis in murine subjects over the 21 day period to a single time-point measurement in human RA subjects for the same select target genes is provided in Figures 12F and 12G. In this case, the translation of response across species is equally striking, however time-point dependent over the KRN model time-course.

### Drug-Treated BABL/c Mice: Assessment of Response to Dexamethasone

Similar to the CIA model, dexamethasone treatment in female BALB/c mice was initiated after symptoms of arthritis had been well established. Response to Dexamethasone treatment in BALB/c mice with established arthritis was assessed by comparing these drug-treated female BALB/c mice to their vehicle-treated counterparts.

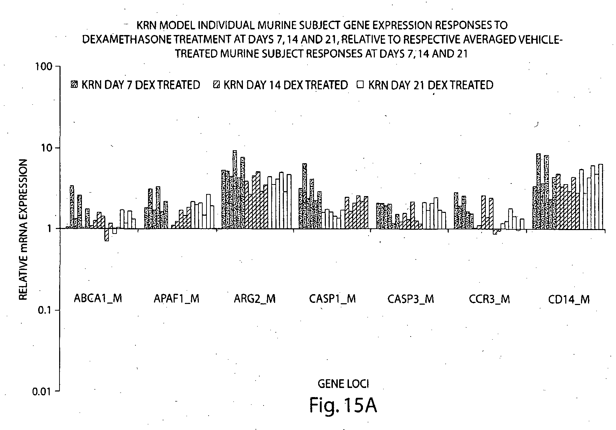

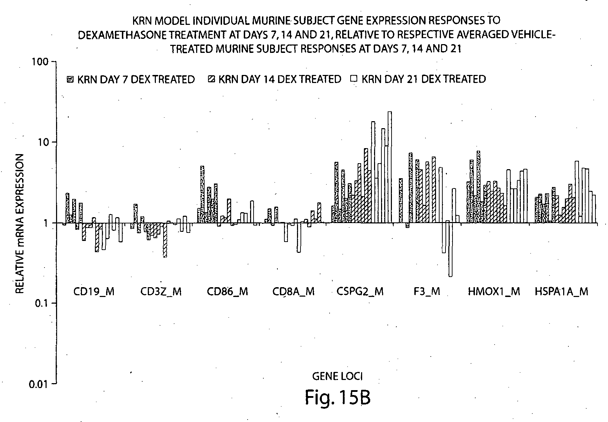

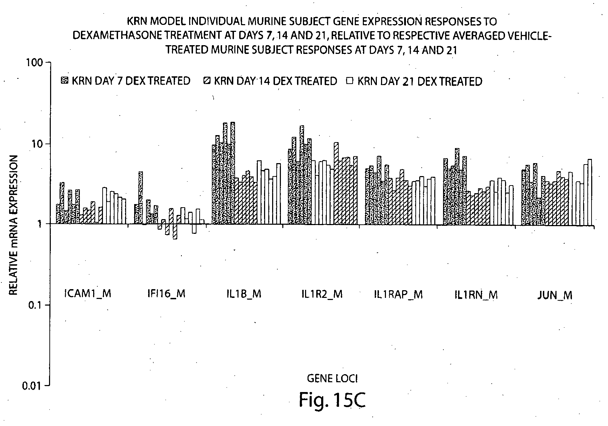

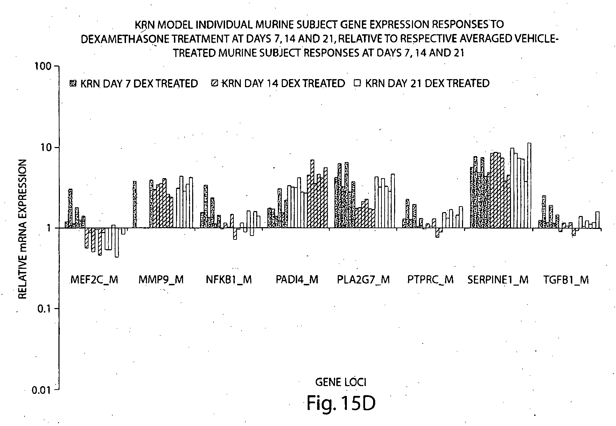

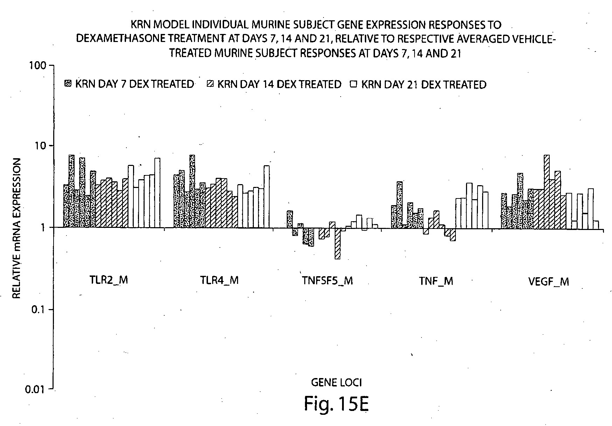

Individual BALB/c mouse gene expression responses to dexamethasone treatment at days 7, 14, and 21 relative to their respective vehicle-treated controls at days 7, 14, and 21 are provided in Figures 15A-15E (and Table 30). Uniformity of gene expression responses to dexamethasone treatment across murine subjects was dominant across target gene time-points.

Dexamethasone treatment was observed to consistently increase expression across all murine subjects and time-points for multiple target genes, including APAF1, ARG2, CASP1, CASP3, CD14, CSPG2, HMOX1, HSPA1A, ICAM, IL1B, IL1R2, IL1RAP, IL1RN, JUN, PADI4, PLA2G7, SERPINE1, TLR2, TLR4, and VEGF. Multiple additional target genes demonstrated this same pattern of increased expression, however a somewhat decreased magnitude of induction or more individual variability was observed (for example, see ABCA1, CCR3, CD86, MMP9, and TNF). While some blocking effect was observed (CD19, CD3Z, MEF2C, and TNFSF5), this was time-point dependent and was subject to individual BALB/c mouse variability.

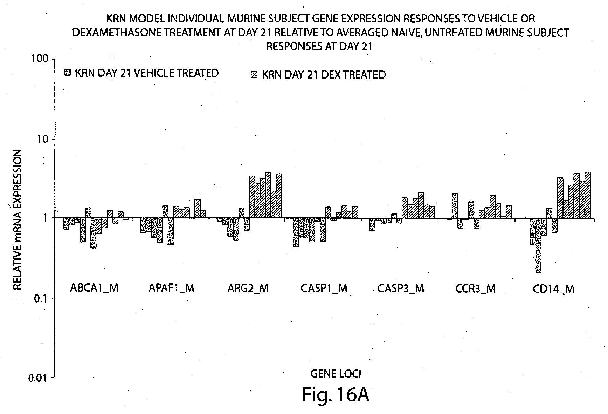

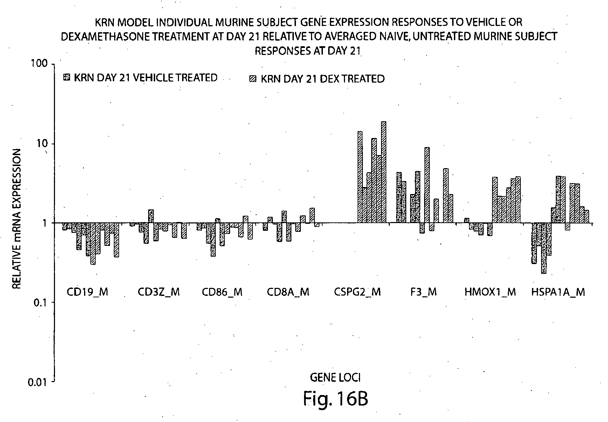

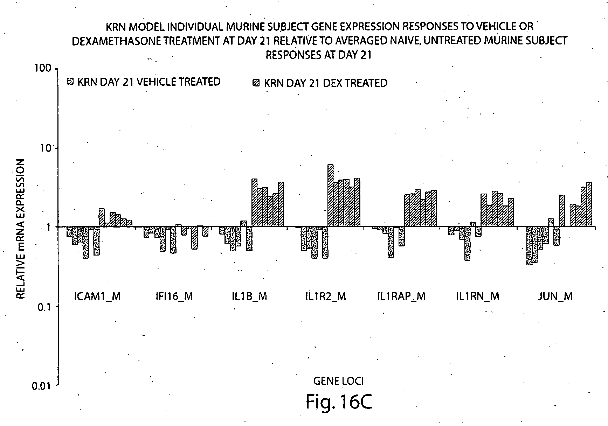

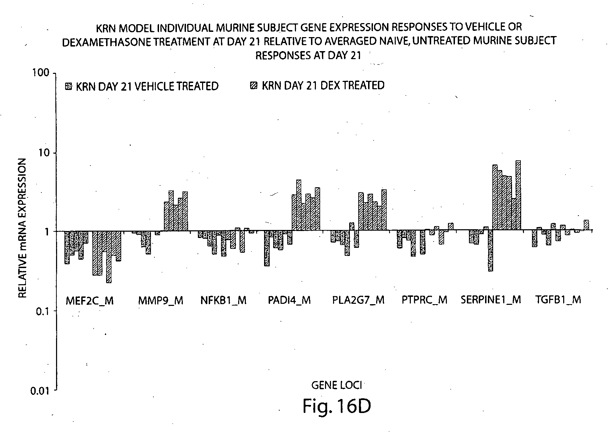

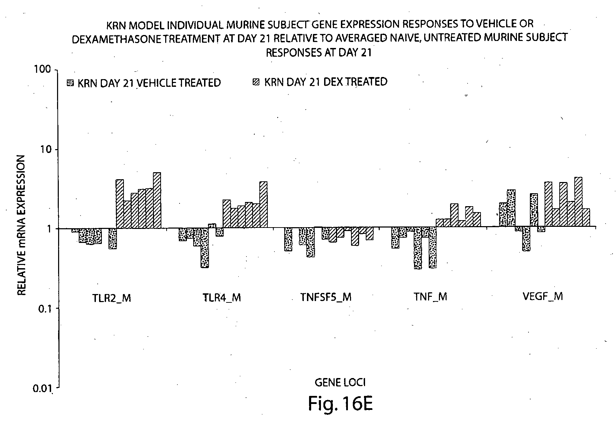

The availability of untreated, vehicle-treated and dexamethasone-treated BALB/c mouse groups at day 21 provided an opportunity to compare the treated relative to untreated BALB/c mouse groups at this terminal day. Individual BALB/c mouse gene expression responses to vehicle or dexamethasone treatment relative to the average, time-matched normal group (naïve, non-immunized and untreated at day 21) is provided in Figures 16A-16E (and Table 31). This additional relative expression view further supports the consistently induced expression observed previously in the counterparts (Figures 15A-15E) at this specific time-point (day 21).

These data support our conclusion that Gene Expression Profiles with sufficient precision and calibration as described herein for humans and other mammals such as rodents (1) can determine subpopulations of individuals with a known biological condition; (2) may be used to monitor the response of patients to therapy; (3) may be used to assess the efficacy and safety of therapy; and (4) may be used to guide the medical management of a patient by adjusting therapy to bring one or more relevant Gene Expression Profiles closer to a target set of values, which may be normative values or other desired or achievable values. We have shown that Gene Expression Profiles may provide meaningful information even when derived from ex vivo treatment of blood or other tissue. We have also shown that Gene Expression Profiles derived from peripheral whole blood are informative of a wide range of conditions neither directly nor typically associated with blood.

Furthermore, in embodiments of the present invention, Gene Expression Profiles can also be used for characterization and early identification (including pre-symptomatic states) of inflammatory conditions associated with biological conditions, including disease. This characterization includes discriminating between healthy and non-healthy individuals, bacterial and viral infections, autoimmune and pathogenic biological conditions, specific subtypes of pathogenic agents and/or conditions, stages of the natural history of the biological condition (e.g., early or late), and assessing prognosis. Use of the algorithmic and statistical approaches discussed above to achieve such identification and to discriminate in such fashion is within the scope of various embodiments herein.

The technology of this application also includes methods for identifying Signature Panels for rodents that can be used to model how humans will respond to various therapeutic agents, nutraceuticals, circumstances, external factors such as environment, location, secondary infections and/or conditions. This in turn is used to identify and monitor therapeutic treatments, including prophylactic and maintenance treatments, for human biological conditions of interest.

The references listed below are hereby incorporated herein by reference.

### References

Magidson, J. GOLDMineR User's Guide (1998). Belmont, MA: Statistical Innovations Inc.
Vermunt J.K. and J. Magidson. Latent GOLD 4.0 User's Guide. (2005) Belmont, MA: Statistical Innovations Inc.
Vermunt J.K. and J. Magidson. Technical Guide for Latent GOLD 4.0: Basic and Advanced (2005) Belmont, MA: Statistical Innovations Inc.
Vermunt J.K. and J. Magidson. Latent Class Cluster Analysis in (2002) J. A. Hagenaars and A. L. McCutcheon (eds.), Applied Latent Class Analysis, 89-106. Cambridge: Cambridge University Press.
Magidson, J. "Maximum Likelihood Assessment of Clinical Trials Based on an Ordered Categorical Response." (1996) Drug Information Journal, Maple Glen, PA: Drug Information Association, Vol. 30, No. 1, pp 143-170.

**Table 1. Inflammation Gene Expression Panel**

| **Symbol** | **Name** | **Classification** | **Description** |
|---|---|---|---|
| IL1A | Interleukin I, alpha | cytokines-chemokines-growth factors | Proinflammatory; constitutively and inducibly expressed in variety of cells. Generally cytosolic and released only during severe inflammatory disease |
| IL1B | Interleukin 1, beta | cytokines-chemokines-growth factors | Proinflammatory;constitutivelyand inducibly expressed by many cell types, secreted |
| TNFA | Tumor necrosis factor, alpha | cytokines-chemokines-growth factors | Proinflammatory, TH1, mediates host response to bacterial stimulus, regulates cell growth & differentiation |
| IL6 | Interleukin 6 (interferon, beta 2) | cytokines-chemokines-growth factors | Pro- and antiinflammatory activity, TH2 cytokine, regulates hemotopoietic system and activation of innate response |
| IL8 | Interleukin 8 | cytokines-chemokines-growth factors | Proinflammatory, major secondary inflammatory mediator, cell adhesion, signal transduction, cell-cell signaling, angiogenesis, synthesized by a wide variety of cell types |
| IFNG | Interferon gamma | cytokines-chemokines-growth factors | Pro- and antiinflammatory activity, TH1 cytokine, nonspecific inflammatory mediator, produced by activated T-cells |
| IL2 | Interleukin 2 | cytokines-chemokines-growth factors | T-cell growth factor, expressed by activated T-cells, regulates lymphocyte activation and differentiation; inhibits apoptosis, TH1 cytokine |
| IL12B | Interleukin 12 p40 | cytokines-chemokines-growth factors | Proinflammatory; mediator of innate immunity, TH1 cytokine, requires co-stimulation with 1L-18 to induce IFN-g |
| IL15 | Interleukin 15 | cytokines-chemokines-growth factors | Proinflammatory; mediates T-cell activation, inhibits apoptosis, synergizes with IL-2 to induce IFN-g and TNF-a |
| IL18 | Interleukin 18 | cytokines-chemokines-growth factors | Proinflammatory, TH1, innate and aquired immunity, promotes apoptosis, requires co-stimulation with IL-1 or IL-2 to induce TH1 cytokines in T- and NK-cells |
| ILA | Interleukin 4 | cytokines-chemokines-growth factors | Antiinflammatory; TH2; suppresses proinflammatory cytokines, increases expression of IL-1RN, regulates lymphocyte activation |
| IL5 | Interleukin 5 | cytokines-chemokines-growth factors | Eosinophil stimulatory factor; stimulates late B cell differentiation to secretion of Ig |
| IL10 | Interleukin 10 | cytokines-chemokines-growth factors | Antiinflammatory; TH2; suppresses production of proinflammatory cytokines |
| IL13 | Interleukin 13 | cytokines-chemokines-growth factors | Inhibits inflammatory cytokine production |
| IL1RN | Interleukin 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| IL18BP | IL-18 Binding Protein | cytokines-chemokines-growth factors | Implicated in inhibition of early TH1 cytokine responses |
| TGFB1 | Transformin g growth factor, beta 1 | cytokines-chemokines-growth factors | Pro- and antiinflammatory activity, anti-apoptotic; cell-cell signaling, can either inhibit or stimulate cell growth |
| IFNA2 | Interferon, alpha 2 | cytokines-chemokines-growth factors | interferon produced by macrophages with antiviral effects |
| GRO1 | GRO1 oncogene (melanoma gowth stimulating activity, alpha) | cytokines-chemokines-growth factors | AKA SCYB1; chemotactic for neutrophils |
| GRO2 | GRO2 oncogene | cytokines-chemokines-growth factors | AKA MIP2, SCYB2; Macrophage inflammatory protein produced by moncytes and neutrophils |
| TNFSF5 | Tumor necrosis factor (ligand) superfamily, member 5 | cytokines-chemokines-gowth factors | ligand for CD40; expressed on the surface of T cells. It regulates B cell function by engaging CD40 on the B cell surface |
| TNFSF6 | Tumor necrosis factor (ligand) superfamily, member 6 | cytokines-chemokines-growth factors | AKA FasL; Ligand for FAS antigen; transduces apoptotic signals into cells. |
| CSF3 | Colony stimulating factor 3 (granulocyte ) | cytokines-chemokines-growth factors | AKA GCSF;cytokine that stimulates granulocyte development |
| B7 | B7 protein | cell signaling and activation | Regulatory protein that may be associated with lupus |
| CSF2 | Granulocyte-monocyte colony stimulating factor | cytokines-chemokines-growth factors | AKA GM-CSF; Hematopoietic growth factor; stimulates growth and differentiation of hematopoietic precursor cells from various lineages, including granulocytes, macrophages, eosinophils, and erythrocytes |
| TNFSF13B | Tumor necrosis factor (ligand) superfamily, member 13b | cytokines-chemokines-growth factors | B cell activating factor, TNF family |
| TACI | Transmembr ane activator and CAML interactor | cytokines-chemokines-growth factors | T cell activating factor and calcium cyclophilin modulator |
| VEGF | vascular endothelial growth factor | cytokines-chemokines-growth factors | Produced by monocytes |
| ICAM1 | Intercellular adhesion molecule 1 | Cell Adhesion / Matrix Protein | Endothelial cell surface molecule; regulates cell adhesion and trafficking, upregulated during cytokine stimulation |
| PTGS2 | Prostaglandi n-endoperoxid e synthase 2 | Enzyme / Redox | AKA COX2; Proinflammatory, member of arachidonic acid to prostanoid conversion pathway; induced by proinflammatory cytokines |
| NOS2A | Nitric oxide synthase 2A | Enzyme / Redox | AKA iNOS; produces NO which is bacteriocidal/tumoricidal |
| PLA2G7 | Phospholipa se A2, group VII (platelet activating factor acetylhydrol ase, plasma) | Enzyme / Redox | Platelet activating factor |
| HMOX1 | Heme oxygenase (decycling) 1 | Enzyme Redox | Endotoxin inducible |
| F3 | F3 | Enzyme / Redox | AKA thromboplastin, Coagulation Factor 3; cell surface glycoprotein responsible for coagulation catalysis |
| CD3Z | CD3 antigen, zeta polypeptide | Cell Marker | T-cell surface glycoprotein |
| PTPRC | protein tyrosine phosphatase, receptor type, C | Cell Marker | AKA CD45; mediates T-cell activation |
| CD14 | CD14 antigen | Cell Marker | LPS receptor used as marker for monocytes |
| CD4 | CD4 antigen (p55) | Cell Marker | Helper T-cell marker |
| CDBA | CD8 antigen, alpha polypeptide | Cell Marker | Suppressor T cell marker |
| CD19 | CD19 antigen | Cell Marker | AKA Leu 12; B cell growth factor |
| HSPA1A | Heat shock protein 70 | Cell Signaling and activation | heat shock protein 70 kDa |
| MMP3 | Matrix metalloprotei nase 3 | Proteinase / Proteinase Inhibitor | AKA stromelysin; degrades fibronectin, laminin and gelatin |
| MMP9 | Matrix metalloprotei nase 9 | Proteinase / Proteinase Inhibitor | AKA gelatinase B; degrades extracellular matrix molecules, secreted by IL-8-stimulated neutrophils |
| PLAU | Plasminogen activator, urokinase | Proteinase / Proteinase Inhibitor | AKA uPA; cleaves plasminogen to plasmin (a protease responsible for nonspecific extracellular matrix degradation) |
| SERPINE1 | Serine (or cysteine) protease inhibitor, clade B (ovalbumin), member 1 | Proteinase/ Proteinase Inhibitor | Plasminogen activator inhibitor-1 / PAI-1 |
| TIMP1 | tissue inhibitor of metalloprotei nase 1 | Proteinase / Proteinase Inhibitor | Irreversibly binds and inhibits metalloproteinases, such as collagenase |
| C1QA | Complement component 1, q subcompone nt, alpha polypeptide | Proteinase / Proteinase Inhibitor | Serum complement system; forms C1 complex with the proenzymes c1r and c1s |
| HLA-DRB1 | Major histocompati bility complex, class II, DR beta 1 | Histocompatib ility | Binds antigen for presentation to CD4+ cells |

**Table 2. Rheumatoid Arthritis or Inflammatory Conditions Related to Rheumatoid Arthritis Gene Expression Panel**

| **Symbol** | **Name** | **Classification** | **Description** |
|---|---|---|---|
| APAF1 | Apoptotic Protease Activating Factor 1 | Protease activating peptide | Cytochrome c binds to APAF1, triggering activation of CASP3, leading to apoptosis. May also facilitate procaspase 9 auto activation. |
| BCL2 | B-cell CLL / lymphoma 2 | Apoptosis Inhibitor - cell cycle control -oncogenesis | Blocks apoptosis by interfering with the activation ofcaspases |
| BPI | Bactericidal/perm eability-increasing protein | Membrane-bound protease | LPS binding protein; cytotoxic for many gram negative organisms; found in myeloid cells |
| C1QA | Complement component 1, q subcomponent, alpha polypeptide | Proteinase/ proteinase inhibitor | Serum complement system; forms C1 complex with the proenzymes c1r and C1s |
| CASP1 | Caspase 1 | Proteinase | Activates IL1B; stimulates apoptosis |
| CASP3 | Caspase 3 | Proteinase / Proteinase Inhibitor | Involved in activation cascade of caspases responsible for apoptosis -cleaves CASP6, CASP7, CASP9 |
| CASP9 | Caspase 9 | Proteinase | Binds with APAF1 to become activated; cleaves and activates CASP3 |
| CCL1 | Chemokine (C-C Motif) ligand 1 | Cytokines-chemokines-growth factors | Secreted by activated T cells; chemotactic for monocytes but not neutrophils; binds to CCR8 |
| CCL2 | Chemokine (C-C Motif) ligand 2 | Cytokines-chemokines-growth factors | CCR2 chemokine; Recruits monocytes to areas of injury and infection; Upregulated in liver inflammation; Stimulates IL-4 production; Implicated in diseases involving monocyte, basophil infiltration of tissue (e.g. psoriasis, rheumatoid arthritis, atherosclerosis) |
| CCL3 | Chemokine (C-C motif) ligand 3 | Cytokines-chemokines-growth factors | AKA: MIP1-alpha; monkine that binds to CCR1, CCR4 and CCR5; major HIV-suppressive factor produced by CD8 cells. |
| CCL4 | Chemokine (C-C Motif) ligand 4 | Cytokines-chemokines-growth factors | Inflammatory and chemotactic monokine; binds to CCR5 and CCR8 |
| CCL5 | Chemokine (C-C Motif) ligand 5 | Cytokines-chemokines-growth factors | Binds to CCR1, CCR3, and CCR5 and is a chemoattractant for blood monocytes, memory T-helper cells and eosinophils; A major HIV-suppressive factor produced by CD8-positive T-cells |
| CCR3 | Chemokine (C-C motif) receptor 3 | Chemokine receptor | C-C type chemokine receptor (Eotaxin receptor) binds to Eotaxin, Eotaxin-3, MCP-3, MCP-4, SCYA5/RANTES and mip-1 delta thereby mediating intracellular calcium flux. Alternative co-receptor with CD4 for HIV-1 infection. Involved in recruitment of eosinophils. Primarily a Th2 cell chemokine receptor. |
| CD14 | CD14 antigen | Cell Marker | LPS receptor used as marker for monocytes |
| CD19 | CD19 antigen | Cell Marker | AKA Leu 12; B cell growth factor |
| CD3Z | CD3 antigen, zeta polypeptide | Cell Marker | T-cell surface glycoprotein |
| CD4 | CD4 antigen (p55) | Cell Marker | Helper T-cell marker |
| CD86 | CD 86 Antigen (cD 28 antigen ligand) | Cell signaling and activation | AKA B7-2; membrane protein found in B lymphocytes and monocytes; co-stimulatory signal necessary for T lymphocyte proliferation through IL2 production. |
| CD8A | CD8 antigen, alpha polypeptide | Cell Marker | Suppressor T cell marker |
| CKS2 | CDC28 protein kinase regulatory subunit 2 | Cell signaling and activation | Essential for function of cyclin-dependent kinases |
| CSF2 | Granulocyte-monocyte colony stimulating factor | Cytokines-chemokines-growth factors | AKA GM-CSF; Hematopoietic growth factor; stimulates growth and differentiation of hematopoietic precursor cells from various lineages, including granulocytes, macrophages, eosinophils, and erythrocytes |
| CSF3 | Colony stimulating factor 3 (granulocyte) | Cytokines-chemokines-gowth factors | AKA GCSF controls production ifferentiation and function of ganulocytes. |
| CXCL1 | Chemokine (C-X-C- motif) ligand 1 | Cytokines-chemokines-growth factors | Melanoma growth stimulating activity, alpha; Chemotactic pro-inflammatory activation-inducible cytokine. |
| CXCL3 | Chemokine (C-X-C- motif) ligand 3 | Cytokines-chemokines-gowth factors | Chemotactic pro-inflammatory activation-inducible cytokine, acting primarily upon hemopoietic cells in immunoregulatory processes, may also play a role in inflammation and exert its effects on endothelial cells in an autocrine fashion. |
| CXCL10 | Chemokine (C-X-C motif) ligand 10 | Cytokines-chemokines-growth factors | AKA: Gamma IP10; interferon inducible cytokine IP10; SCYB10; Ligand for CXCR3; binding causes stimulation of monocytes, NK cells; induces.T cell migration |
| DPP4 | Dipeptidyl-peptidase 4 | Membrane protein; exopeptidase | Removes dipeptides from unmodified, n-terminus prolines; has role in T cell activation |
| ELA2 | Elastase 2, neutrophil | Protease | Modifies the functions of NK cells, monocytes and granulocytes |
| HMOX1 | Heme oxygenase (decycling) 1 | Enzyme / Redox | Endotoxin inducible |
| HSPA1A | Heat shock protein 70 | Cell Signaling and activation | heat shock protein 70 kDa; Molecular chaperone, stabilizes AU rich mRNA |
| HIST1H1C | Histo 1, Hic | Basic nuclear protein | Responsible for the nucleosome structure within the chromosomal fiber in eukaryotes; may attribute to modification of nitrotyrosine-containing proteins and their immunoreactivity to antibodies against nitrotyrosine |
| ICAM1 | Intercellular adhesion molecule 1 | Cell Adhesion / Matrix Protein | Endothelial cell surface molecule; regulates cell adhesion and trafficking, unregulated during cytokine stimulation |
| IFI16 | Gamma interferon inducible protein 16 | Cell signaling and activation | Transcriptional repressor |
| IFNA2 | Interferon, alpha 2 | Cytokines-chemokines-gowth factors | interferon produced by macrophages with antiviral effects |
| IFNG | Interferon, Gamma | Cytokines / Chemokines / Growth Factors | Pro- and anti-inflammatory activity; TH1 cytokine; nonspecific inflammatory mediator; produced by activated T-cells. |
| IL10 | Interleukin 10 | Cytokines-chemokines-growth factors | Anti-inflammatory; TH2; suppresses productionofproinflammatory cytokines |
| IL12B | Interleukin 12 p40 | Cytokines-chemokines-growth factors | Proinflammatory; mediator of innate immunity, TH1 cytokine, requires co-stimulation with IL-18 to induce IFN-g |
| IL13 | Interleukin 13 | Cytokines / Chemokines / Growth Factors | Inhibits inflammatory cytokine production |
| IL18 | Interleukin 18 | Cytokines-chemokines-growth factors | Proinflammatory, TH1, innate and acquired immunity, promotes apoptosis, requires co-stimulation with IL-1 or IL-2 to induce TH1 cytokines in T- and NK-cells |
| IL18RI | Interleukin 19 receptor 1 | Membrane protein | Receptor for interleukin 18; binding the agonist leads to activation of NFKB-B; belongs to IL1 family but does not bind IL1A or IL1B. |
| IL1A - | Interleukin 1, alpha | Cytokines-chemokines-growth factors | Proinflammatory; constitutively and inducibly expressed in variety of cells. Generally cytosolic and released only during severe inflammatory disease |
| IL1B | Interleukin 1, beta | Cytokines-chemokines-gowth factors | Proinflammatory;constitutively and inducibly expressed by many cell types, secreted |
| IL1R1 | Interleukin 1 receptor, type I | Cell signaling and activation | AKA: CD12 or IL1R1RA; Binds all three forms of interleukin-1 (ILIA, IL1B and IL1RA). Binding of agonist leads to NFKB activation |
| IL1RN | Interleukin I Receptor Antagonist | Cytokines / Chemokines / Growth Factors | IL1 receptor antagonist; Anti-inflammatory; inhibits binding of IL-1 to IL-1 receptor by binding to receptor without stimulating II-1-like activity |
| IL2 | Interleukin 2 | Cytokines / Chemokines / Growth Factors | T-cell growth factor, expressed by activated T-cells, regulates lymphocyte activation and differentiation; inhibits apontosis, TH1 cytokine |
| IL4 | Interleukin 4 | Cytokines / Chemokines / Growth Factors | Anti-inflammatory; TH2; suppresses proinflammatory cytokines, increases expression of IL-1RN, regulates lymphocyte activation |
| IL5 | Interleukin 5 | Cytokines / Chemokines / Growth Factors | Eosinophil stimulatory factor; stimulates late B cell differentiation to secretion of Ig |
| IL6 | Interleukin 6 (interFeron, beta 2) | Cytokines-chemokines-growth factors | Pro- and anti-inflammatory activity, TH2 cytokine, regulates hematopoietic system and activation of innate response |
| IL8 | Interleukin 8 | Cytokines-chemokines-growth factors | Proinflammatory, major secondary inflammatory mediator, cell adhesion, signal transduction, cell-cell signaling, angiogenesis, synthesized by a wide variety of cell types |
| IRF7 | Interferon regulatory factor regulatory | Transcription Factor | Regulates transcription of interferon genes through DNA sequence-specific binding. Diverse roles include virus-mediated activation of interferon, and modulation of cell growth, differentiation, apoptosis, and immune system activity. |
| LTA | LTA (lymphotoxin alpha) | Cytokine | Cytokine secreted by lymphocytes and cytotoxic for a range of tumor cells; active in vitro and in vivo |
| LTB | Lymphotoxin beta (TNFSF3) | Cytokine | Inducer of inflammatory response and normal lymphoid tissue development |
| JUN | v-jun avian sarcoma virus 17 oncogene homolop | Transcription factor-DNA binding | Proto-oncoprotein; component of transcription factor AP-1 that interacts directly with target DNA sequences to regulate pence expression |
| MIF | Macrophage migration inhibitory factor | Cell signaling and gowth factor | AKA; GIF; lymphokine, regulators macrophage functions through suppression of anti-inflammatory effects of glucocorticoids |
| MMP9 | Matrix metalloproteinase 9 | Proteinase / Proteinase Inhibitor | AKA gelatinase B; degrades extracellular matrix molecules, secreted by IL-8-stimulated neutrophils |
| N33 | Putative prostate cancer tumor suppressor | Tumor Suppressor | Integral membrane protein. Associated with homozygous deletion in metastatic prostate cancer. |
| NFKB1 | Nuclear factor of kappa light polypeptide gene enhancer in B-cells 1 (p105) | Transcription Factor | p105 is the precursor of the p50 subunit of the nuclear factor NFKB, which binds to the kappa-b consensus sequence located in the enhancer region of genes involved in immune response and acute phase reactions; the precursor does not bind DNA itself |
| NFKBIB | Nuclear factor of kappa light polypeptide gene **.** enhancer in B-cells inhibitor, beta | Transcription Regulator | Inhibits/regulates NFKB complex activity by trapping NFKB in the cytoplasm. Phosphorylated serine residues mark the NFKBIB protein for destruction thereby allowing activation of the NFKB complex. |
| PF4 | Platelet Factor 4 (SCYB4) | Chemokine | PF4 is released during platelet aggregation and is chemotactic for neutrophils and monocytes. PF4's major physiologic role appears to be neutralization of heparin-like molecules on the endothelial surface of blood vessels, thereby inhibiting local antithrombin III activity and promoting coagulation. |
| PI3 | Proteinase inhibitor 3 skin derived | Proteinase inhibitor-protein binding-extracellular matrix | aka SKALP; Proteinase inhibitor found in epidermis of several inflammatory skin diseases; it's expression can be used as a marker of skin irritancy |
| PLA2G7 | Phospholipase A2, group VII (platelet activating factor acetylhydrolase, plasma) | Enzyme/Redox | Platelet activating factor |
| PTGS2 | Prostaglandin-endoperoxide synthase 2 | Enzyme | Cytokine secreted by lymphocytes and cytotoxic for a range of tumor cells; active in vitro and in vivo |
| PTX3 | Pentaxin-related gene, rapidly induced by IL-1 beta | Acute Phase Protein | Inducer of inflammatory response and normal lymphoid tissue development |
| RAD52 | RAD52 (S. cerevisiae) homolog | DNA binding proteinsor | Involved in DNA double-stranded break repair and meiotic / mitotic recombination |
| SERPINE1 | Serine (or cysteine) protease inhibitor, clade B (ovalbumin), member 1 | Proteinase / Proteinase Inhibitor | Plasminogen activator inhibitor-1 / PAI-1 |
| SLC7A1 | Solute carrier family 7, member 1 | Membrane protein; permease | High affinity, low capacity permease invovled in the transport of positively charged amino acids |
| STAT3 | Signal transduction and activator of transcription 3 | Transcription factor | AKA APRF: Transcription factor for acute phase response genes; rapidly activated in response to certain cytokines and gowth factors; binds to IL6 response elements |
| TGFB1 | Transforming growth factor, beta 1 | cytokines-chemokines-growth factors | Pro- and antiinflammatory activity, anti-apoptotic; cell-cell signaling, can either inhibit or stimulate cell growth |
| TGFBR2 | Transforming growth factor, beta receptor II | Membrane protein | AKA: TGFR2; membrane protein involved in cell signaling and activation, ser/thr protease; binds to DAXX. |
| TIMP1 | Tissue inhibitor of metalloproteinase 1 | Proteinase / Proteinase Inhibitor | Irreversibly binds and inhibits metalloproteinases, such as collagenase |
| TLR2 | Toll-like receptor 2 | cell signaling and activation | mediator ofpetidoglycan and lipotechoic acid induced signalling |
| TNF | Tumor necrosis factor | Cytokine/tumor necrosis factor receptor ligand | Negative regulation of insulin action. Produced in excess by adipose tissue of obese individuals - increases IRS-1 phosphorylation and decreases insulin receptor kinase activity. |
| TNPRSF7 | Tumor necrosis factor receptor superfamily, member 7 | Membrane protein; receptor | Receptor for CD27L; may play a role in activation of T cells |
| TNFSF13B | Tumor necrosis factor (ligand) superfamily, member 13b | Cytokines-chemokines-growth factors | B cell activating factor, TNF family |
| TNFRSF13B | Tumor necrosis factor receptor superfamily, member 13, subunit beta | Cytokines-chemokines growth factors | B cell activating factor, TNF family |
| TNFSF5 | Tumor necrosis factor (ligand) superfamily, member 5 | Cytokines-chemokines-growth factors | Ligand for CD40; expressed on the surface of T cells. It regulates B cell function by engaging CD40 on the B cell surface. |
| TNFSF6 | Tumor necrosis factor (ligand) superfamily, member 6 | Cytokines-chemokines-growth factors | AKA FasL; Ligand for FAS antigen; transduces apoptotic signals into cells |

**Table 3. Mouse 24-Gene Gene Expression Panel (Precision Profile™) for Inflammation**

| **Symbol** | **Name** | **Classification** | **Description** |
|---|---|---|---|
| **APAF1 M** | Apoptotic Protease Activating Factor 1 | protease activator | Cytochrome c binds to APAF1, triggering activation of CASP3, leading to apoptosis. May also facilitate procaspase 9 autoactivation. |
| **ARG2 M** | Arginase II | Enzyme/redox | Catalyzes the hydrolysis of arginine to ornithine and urea; may play a role in down regulation of nitric oxide synthesis |
| **CASP3 M** | Caspase 3 | proteinase | Involved in activation cascade of caspases responsible for apoptosis -cleaves CASP6, CASP7, CASP9 |
| **CCR3 M** | chemokine (C-C motif) receptor 3 | Chemokine receptor | C-C type chemokine receptor (Eotaxin receptor) binds to Eotaxin, Eotaxin-3, MCP-3, MCP-4, SCYA5/RANTES and mip-1 delta thereby mediating intracellular calcium flux. Alternative co-receptor with CD4 for IIIV-1 infection. Involved in recruitment of eosinophils. Primarily a Th2 cell chemokine receptor. |
| **CD14 M** | CD14 antigen | Cell Marker | LPS receptor used as marker for monocytes |
| **CD3Z M** | CD3 antigen, zeta polypeptide | Cell Marker | T-cell surface glycoprotein |
| **CD8A M** | CD8 antigen, alpha polypeptide | Cell Marker | Suppressor T cell marker |
| **F3 M** | F3 | Enzyme / Redox | AKA thromboplastin, Coagulation Factor 3; cell surface glycoprotein responsible for coagulation catalysis |
| **HMOX1 M** | Heme oxygenase (decycling) 1 | Enzyme / Redox | Endotoxin inducible |
| **HSPA1A M** | Heat shock protein 70 | Cell Signaling and activation | heat shock protein 70 kDa |
| **ICAM1 M** | Intercellular adhesion molecule 1 | Cell Adhesion / Matrix Protein | Endothelial cell surface molecule; regulates cell adhesion and trafficking, upregulated during cytokine stimulation |
| **IFI16 M** | gamma interferon inducible protein 16 | cell signaling and activation | Transcriptional repressor |
| **IL1B-M** | Interleukin 1, beta | cytokines-chemokines-growth factors | Proinflammatory;constitutively and inducibly expressed by many cell types, secreted |
| **IL1RN M** | Interleukin 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| **JUN M** | v-jun avian sarcoma virus 17 oncogene homolog | transcription factor-DNA binding | Proto-oncoprotein; component of transcription factor AP-1 that interacts directly with target DNA sequences to regulate gene expression |
| **MMP9 M** | Matrix metalloprotei nase 9 | Proteinase / Proteinase Inhibitor | AKA gelatinase B; degrades extracellular matrix molecules, secreted by IL-8-stimulated neutrophils |
| **PLA2G7 M** | Phospholipa se A2, group VII (platelet activating factor acetylhydrol ase, plasma) | Enzyme / Redox | Platelet activating factor |
| **PTPRC M** | protein tyrosine phosphatase, receptor type, C | Cell Marker | AKA CD45; mediates T-cell activation |
| **SERPINE1 M** | Serine (or cysteine) protease inhibitor, clade B (ovalbumin), member 1 | Proteinase / Proteinase Inhibitor | Plasminogen activator inhibitor-1 / PAI-1 |
| **TGFB1 M** | Transformin g growth factor, beta 1 | cytokines-chemokines-growth factors | Pro- and antiinflammatory activity, anti-apoptotic; cell-cell signaling, can either inhibit or stimulate cell growth |
| **TIMP1 M** | tissue inhibitor of metalloprotei | Proteinase / Proteinase Inhibitor | Irreversibly binds and inhibits metalloproteinases, such as collagenase |
| | nase 1 | | |
| **TLR4 M** | toll-like receptor 4 | cell signaling and activation | mediator of LPS induced signalling |
| **TNFSF5 M** | Tumor necrosis factor (ligand) superfamily, member 5 | cytokines-chemokines-growth factors | ligand for CD40; expressed on the surface of T cells. It regulates B cell function by engaging CD40 on the B cell surface |
| **VEGF M** | vascular endothelial growth factor | cytokines-chemokines-growth factors | Produced by monocytes |

**Table 4. Mouse 8-Gene Signature Panel for Inflammation (LPS Infusion)**

| **Symbol** | **Name** | **Classification** | **Description** |
|---|---|---|---|
| **CASP3 M** | Caspase 3 | proteinase | Involved in activation cascade of caspases responsible for apoptosis -cleaves CASP6, CASP7, CASP9 |
| **CD14 M** | CD14 antigen | Cell Marker | LPS receptor used as marker for monocytes |
| **HMOX1 M** | Heme oxygenate (decycling) 1 | Enzyme / Redox | Endotoxin inducible |
| **IFI16 M** | gamma interferon inducible protein 16 | cell signaling and activation | Transcriptional repressor |
| **IL1B-M** | Interleukin 1, beta | cytokines-chemokines-growth factors | Proinflammatory;constitutively and inducibly expressed by many cell types, secreted |
| **IL1RN M** | Interleukin 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| **TGFB1 M** | Transformin g growth factor, beta 1 | cytokines-chemokines-growth factors | Pro and-intiinflammatory activity, anti-apoptotic; cell-cell signaling, can either inhibit or stimulate cell growth |
| **TLR4 M** | toll-like receptor 4 | cell signaling and activation | mediator of LPS induced signalling |

**Table 5. Mouse 20-Gene Signature Panel for Inflammation**

| **Symbol** | **Name** | **Classification** | **Description** |
|---|---|---|---|
| **CASP3 M** | Caspase 3 | proteinase | Involved in activation cascade of caspases responsive for apoptosis -cleaves CASP6, CASP7, CASP9 |
| **CD14 M** | CD14 antigen | Cell Marker | LPS receptor used as marker for monocytes |
| **CD3Z M** | CD3 antigen, zeta polypept ide | Cell Marker | T-cell surface glycoprotein |
| **CD8A M** | CD8 antigen, alpha polypept ide | Cell Marker | Suppressor T cell marker |
| **F3 M** | F3 | Enzyme / Redox | AKA thromboplastin, Coagulation Factor 3; cell surface glycoprotein responsible for coagulation catalysis |
| **HMOX1 M** | Heme oxygena se (decyclin g) 1 | Enzyme / Redox | Endotoxin inducible |
| **HSPA1A M** | Heat shock protein 70 | Cell Signaling and activation | heat shock protein 70 kDa |
| **ICAM1 M** | Intercell ular adhesion . molecule 1 | Cell Adhesion / Matrix Protein | Endothelial cell surface molecule; regulates cell adhesion and trafficking, upregulated during cytokine stimulation |
| **IFI16 M** | gamma interfero n inducible protein 16 | cell signaling and activation | Transcriptional repressor |
| **IL1B-M** | Interleuk in 1, beta | cytokines-chemokines-growth factors | Proinflammatory;constitutively and inducibly expressed by many cell types, secreted |
| **IL1RN M** | Interleuk in 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| **MMP9 M** | Matrix metallop roteinase 9 | Proteinase / Proteinase Inhibitor | AKA gelatinase B; degrades extracellular matrix molecules, secreted by IL-8-stimulated neutrophils |
| **PLA2G7 M** | Phosphol ipase A2, group VII (platelet activatin g factor acetylhy drolase, plasma) | Enzyme / Redox | Platelet activating factor |
| **PTPRC M** | protein tyrosine phosphat ase, receptor type, C | Cell Marker | AKA CD45; mediates T-cell activation |
| **SERPINE1 M** | Serine (or cysteine) protease inhibitor, clade B (ovalbu min), member 1 | Proteinase / Proteinase Inhibitor | Plasminogen activator inhibitor-1 / PAI-1 |
| **TGFB1 M** | Transfor ming growth factor, beta 1 | cytokines-chemokines-growth factors | Pro- and antiinflammatory y activity, anti-apoptotic; cell-cell signaling, can either inhibit or stimulate cell growth |
| **TIMP1 M** | tissue inhibitor of metallop roteinase 1 | Proteinase/ Proteinase Inhibitor | Irreversibly binds and inhibits metalloproteinases, such as collagenase |
| **TLR4 M** | toll-like receptor 4 | cell signaling and activation | mediator of LPS induced signalling |
| **TNFSF5 M** | Tumor necrosis factor (ligand) superfam ily, member 5 | cytokines-chemokines-growth factors | ligand for CD40; expressed on the surface of T cells. It regulates B cell function by engaging CD40 on the B cell surface |
| **VEGF M** | vascular endotheli al growth factor | cytokines-chemokines-growth factors | Produced by monocytes |

**Table 6. Mouse 8-Gene Signature Panel for Inflammation (LPS + Dexamethasone)**

| **Symbol** | **Name** | **Classification** | **Description** |
|---|---|---|---|
| **CD14 M** | CD14 antigen | Cell Marker | LPS receptor used as marker for monocytes |
| **HSPA1A M** | Heat shock protein 70 | Cell Signaling and activation | heat shock protein 70 kDa |
| **ICAM1 M** | Intercellular adhesion molecule 1 | Cell Adhesion / Matrix Protein | Endothelial cell surface molecule; regulates cell adhesion and trafficking, upregulated during cytokine stimulation |
| **IFI16 M** | gamma interferon inducible protein 16 | cell signaling and activation | Transcriptional repressor |
| **IL1B-M** | Interleukin 1, beta | cytokines-chemokines-growth factors | Proinflammatory;constitutively and inducibly expressed by many cell types, secreted |
| **IL1RN M** | Interleukin 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| **MMP9 M** | Matrix metalloprotei nase 9 | Proteinase / Proteinase Inhibitor | AKA gelatinase B; degrades extracellular matrix molecules, secreted by IL-8-stimulated neutrophils |
| **PLA2G7 M** | Phospholipa se A2, group VII (platelet activating factor acetylhydrol ase, plasma) | Enzyme / Redox | Platelet activating factor |

**Table 7. Mouse 9-Gene Signature Panel for Inflammation (LPS-Stimulated Whole Blood Response)**

| **Symbol** | **Name** | **Classification** | **Description** |
|---|---|---|---|
| **CD3Z M** | CD3 antigen, zeta polypeptide | Cell Marker | T-cell surface glycoprotein |
| **CD8A M** | CD8 antigen, alpha polypeptide | Cell Marker | Suppressor T cell marker |
| **HMOX1 M** | Heme oxygenase (decycling) 1 | Enzyme / Redox | Endotoxin inducible |
| **HSPA1A M** | Heat shock protein 70 | Cell Signaling and activation | heat shock protein 70 kDa |
| TCAM1 M | Intercellular adhesion molecule 1 | Cell Adhesion / Matrix Protein | Endothelial cell surface molecule; regulates cell adhesion and trafficking, upregulated during cytokine stimulation |
| **IL1RN M** | Interleukin 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| **PLA2G7 M** | Phospholipa se A2, group VII (platelet activating factor acetylhydrol ase, plasma) | Enzyme / Redox | Platelet activating factor |
| **SERPINE1 M** | Serine (or cysteine) protease inhibitor, clade B (ovalbumin), member 1 | Proteinase / Proteinase Inhibitor | Plasminogen activator inhibitor-1 / PAI-1 |
| **TNFSF5 M** | Tumor necrosis factor (ligand) superfamily, member 5 | cytokines-chemokines-growth factors | ligand for CD40; expressed on the surface of T cells. It regulates B cell function by engaging CD40 on the B cell surface |

**Table 8. Mouse 8-Gene Signature Panel for Inflammationm (LPS-Stimulated Whole Blood Response)**

| **Symbol** | **Name** | **Classification** | **Description** |
|---|---|---|---|
| **CD3Z M** | CD3 antigen, zeta polypeptide | Cell Marker | T-cell surface glycoprotein |
| **CD8A M** | CD8 antigen, alpha polypeptide | Cell Marker | Suppressor T cell marker |
| **HMOX1 M** | Heme oxygenase (decycling) 1 | Enzyme / Redox | Endotoxin inducible |
| **F3 M** | F3 | Enzyme / Redox | AKA thromboplastin, Coagulation Factor 3; cell surface glycoprotein responsible for coagulation catalysis |
| **ICAM1 M** | Intercellular adhesion molecule 1 | Cell Adhesion / Matrix Protein | Endothelial cell surface molecule; regulates cell adhesion and trafficking, upregulated during cytokine stimulation |
| **IL1RN M** | Interleukin 1 receptor antagonist | cytokines-chemokines-growth factors | IL1 receptor antagonist; Antiinflammatory; inhibits binding of IL-1 to IL-1 receptor by binding to receptor without stimulating IL-1-like activity |
| **TIMP1 M** | tissue inhibitor of metalloprotei nase I | Proteinase / Proteinase Inhibitor | Irreversibly binds and inhibits metalloproteinases; such as collagenase |
| **TNFSF5 M** | Tumor necrosis factor (ligand) superfamily, member 5 | cytokines-chemokines-growth factors | ligand for CD40; expressed on the surface of T cells. It regulates B cell function by engaging CD40 on the B cell surface |

**Table 9. Murine 40-gene Precision Profile^{™} for Rheumatoid Arthritis**

| **Gene Symbol** | **Gene Name** | **Gene Accession Number** |
|---|---|---|
| Abca1 | ATP-binding cassette, sub-family A (ABC1), member 1 | NM_013454 |
| Apaf1 | apoptotic peptidase activating factor 1 | NM_009684 |
| Arg2 | arginase type II | NM_009705 |
| Casp1 | caspase 1 | NM_009807 |
| Casp3 | caspase 3 | NM_009810 |
| Ccr3 | chemokine (C-C motif) receptor 3 | NM_009914 |
| Cd14 | CD14 antigen | NM_009841 |
| Cd19 | CD19 antigen | NM_009844 |
| Cd3z | CD247 antigen | NM_031162 |
| Cd86 | CD86 antigen | NM_019388 |
| Cd8a | CD8 antigen, alpha chain | XM_132621 |
| Cspq2 | versican | XM_898918 |
| F3 | coagulation factor III | NM_010171 |
| Hmox1 | heme oxygenase (decycling) 1 | NM_010442 |
| Hspa1a | heat shock protein 1A | NM_010479 |
| lcam1 | intercellular adhesion molecule | NM_010493 |
| lfi16 | interferon activated gene 204 | NM_008329 |
| II10 | interleukin 10 | NM_010548 |
| II1a | interleukin 1 alpha | NM_010554 |
| II1b | Interleukin 1, beta | NM_008361 |
| II1r2 | interleukin 1 receptor, type II | NM_010555 |
| IL1rap | Interleukin 1 receptor accessory protein | NM_008364 |
| II1rn | interleukin 1 receptor antagonist | NM_031167 |
| II6 | interleukin 6 | NM_031168 |
| Jun | Jun oncogene | NM_010591 |
| Mef2c | myocyte enhancer factor 2C | NM_025282 |
| Mmp9 | matrix metallopeptidase 9 | NM_013599 |
| Nfkb1 | nuclear factor of kappa light chain gene enhancer in B-cells 1, P105 | NM_008689 |
| Padi4 | peptidyl arginine deiminase, type IV | NM_011061 |
| Pla2g7 | phospholipase A2, group VII (platelet-activating factor acetylhydrolase, plasma) | NM_013737 |
| Ptprc | protein tyrosine phosphatase, receptor type, C | NM_011210 |
| Ptx3 | pentraxin related gene | NM_008987 |
| Serpinel | serine (or cysteine) peptidase inhibitor, clade E, member 1 | NM_008871 |
| Tpfb1 | transforming growth factor, beta 1 | NM_011577 |
| Timp1 | tissue inhibitor of metalloproteinase 1 | NM_011593 |
| TIr2 | toll-like receptor 2 | NM_011905 |
| TIr4 | toll-like receptor 4 | NM_021297 |
| Tnfsf5 | CD40 ligand | NM_011616 |
| Tnf | tumor necrosis factor | NM_013693 |
| Vegf | vascular endothelial growth factor A | NM_009505 |

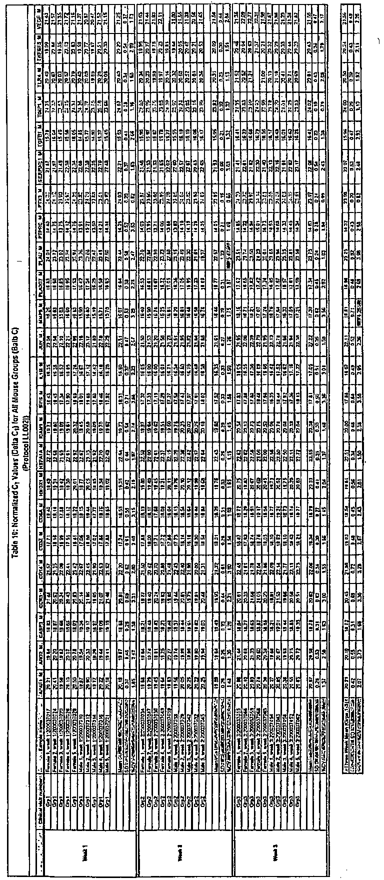

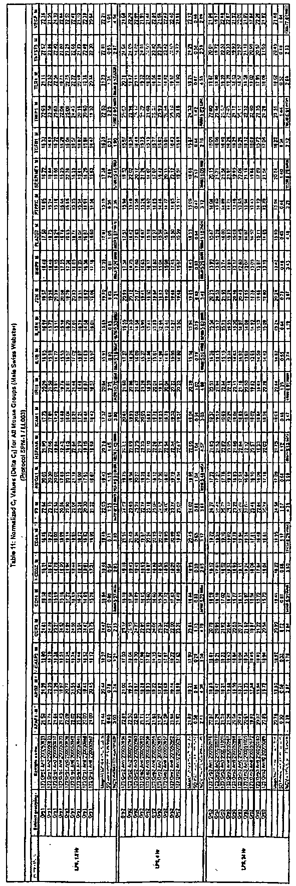

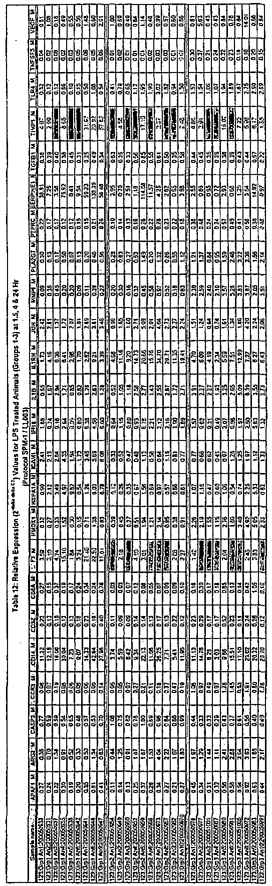

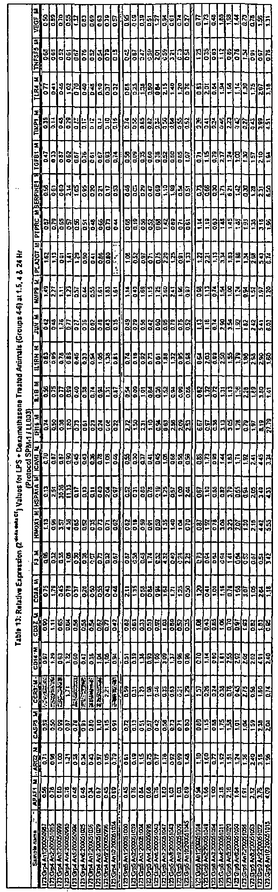

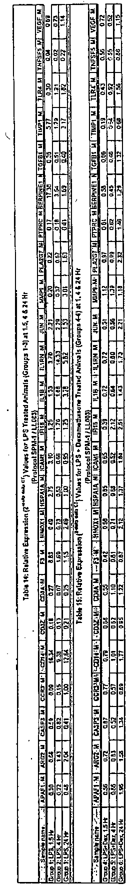

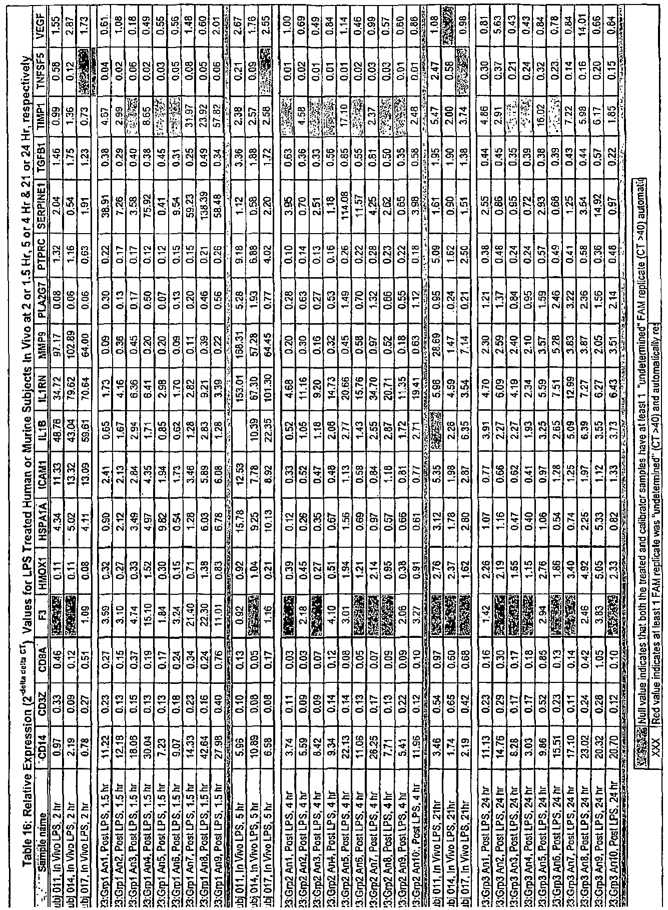

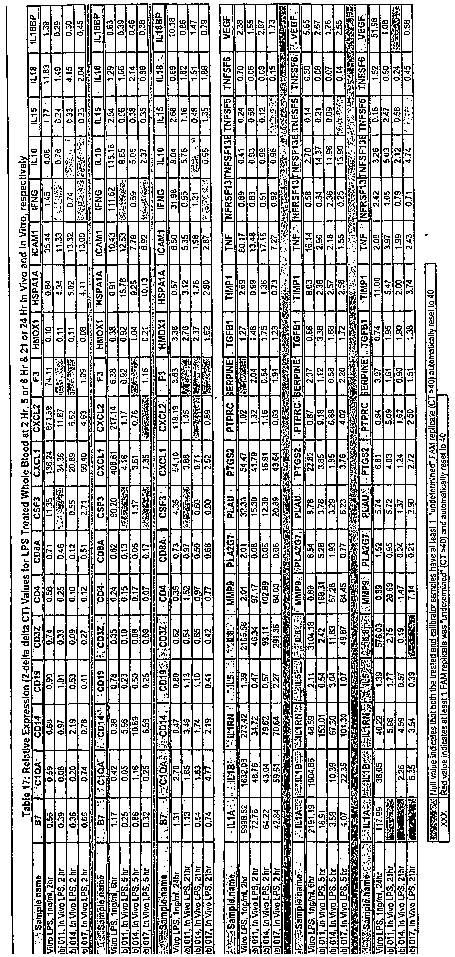

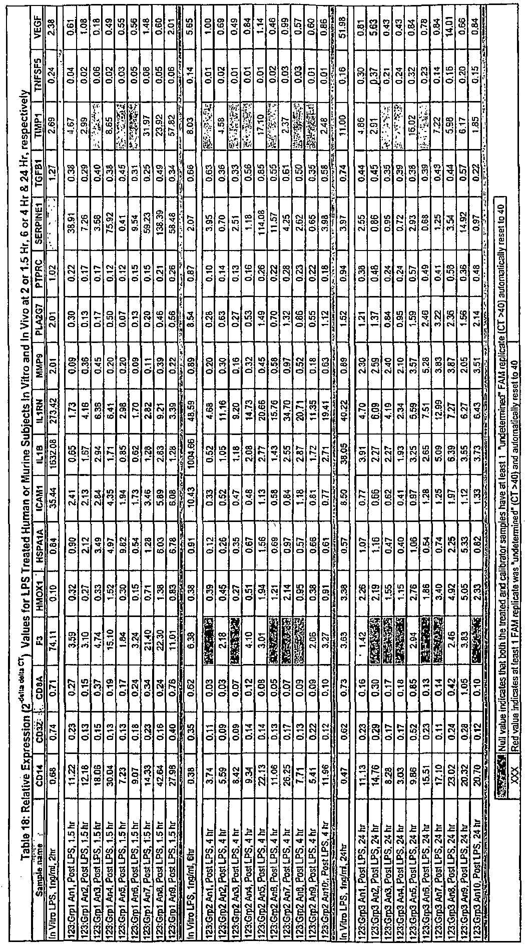

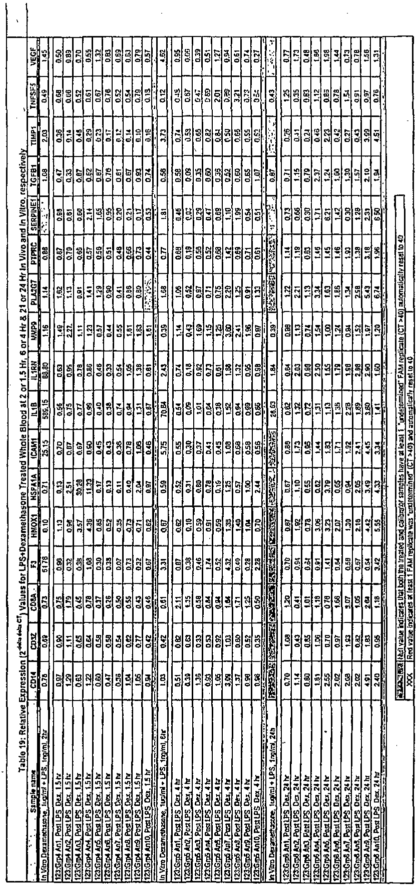

**Table 20A: Study Schema for CIA Murine Model of Arthritis**

| CIA Mouse Model (male DBA/1 mice) | | | | | | |
|---|---|---|---|---|---|---|
| Treament | Baseline naive D0 | CIA D24 | CIA D33 | CIA D42 | CIA D60 | naive D60 |
| CIA vehicle-treated | | | 6 | 6 | 6 | |
| CIA drug-treated | | | 6 | 6 | 6 | |
| Untreated | 6 | 6 | | | | 6 |

**Table 20B: Study Schema for KRN Murine Model of Arthritis**

| KRN Mouse Model (female BALB/c mice) | | | | | | |
|---|---|---|---|---|---|---|
| Treatment | Baseline naive D0 | KRN D3 | KRN D7 | KRN D14 | KRN | naive D21 |
| KRN vehicle-treated | | | 6 | 6 | 6 | |
| KRN drug-treated | | | 6 | 6 | 6 | |
| Untreated | 6 | 6 | | | | 6 |

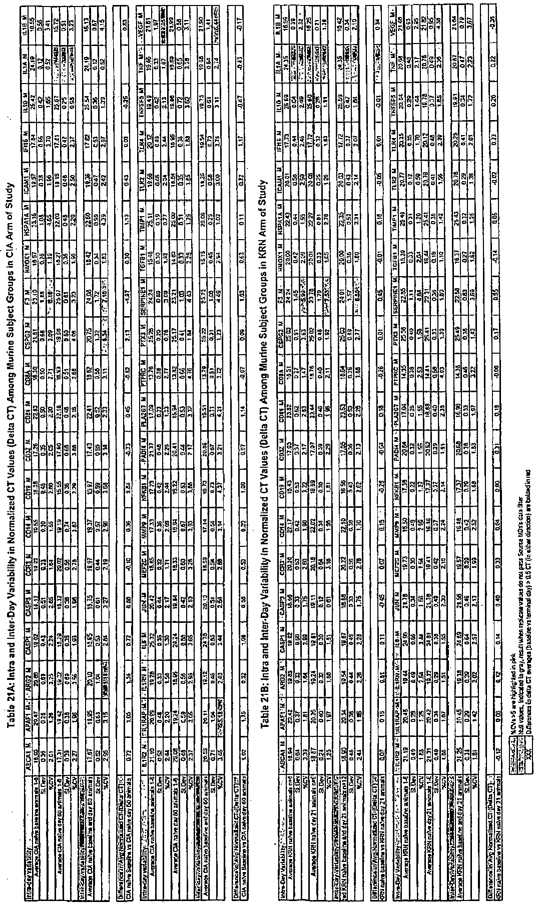

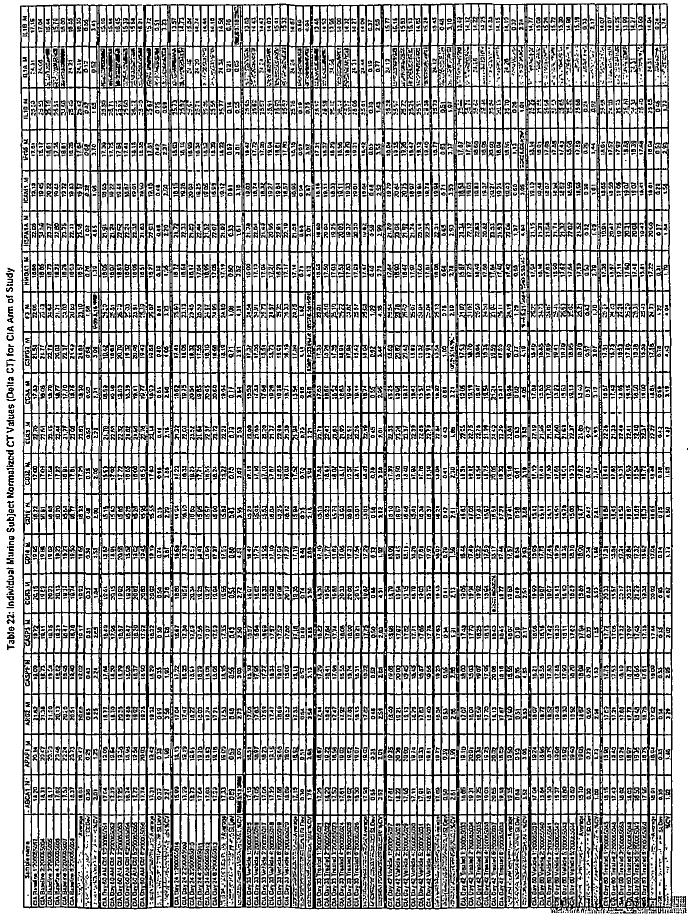

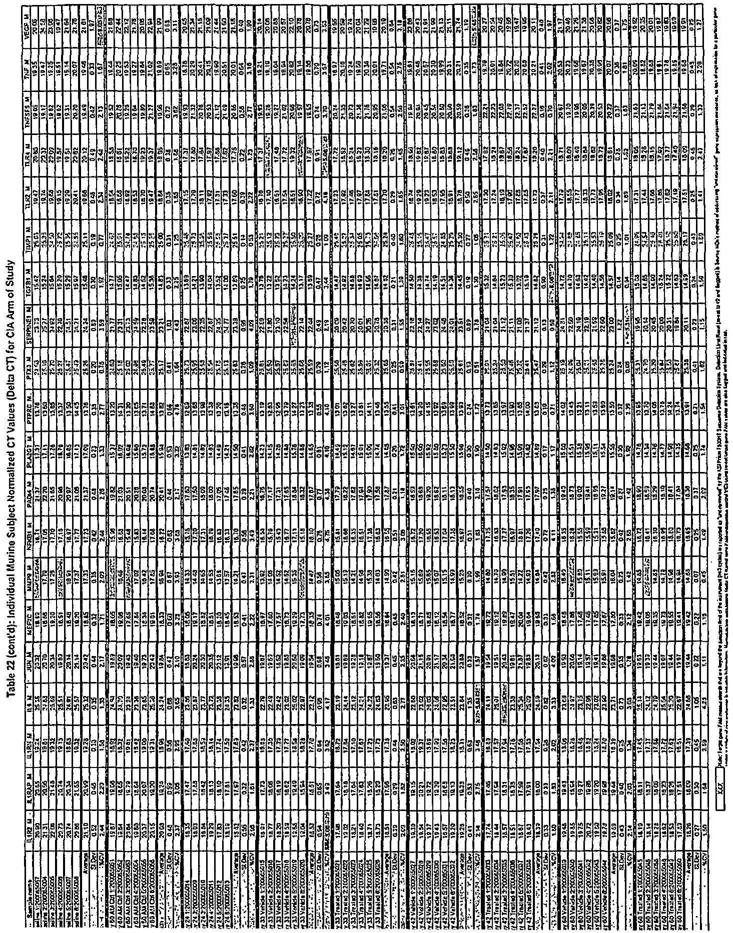

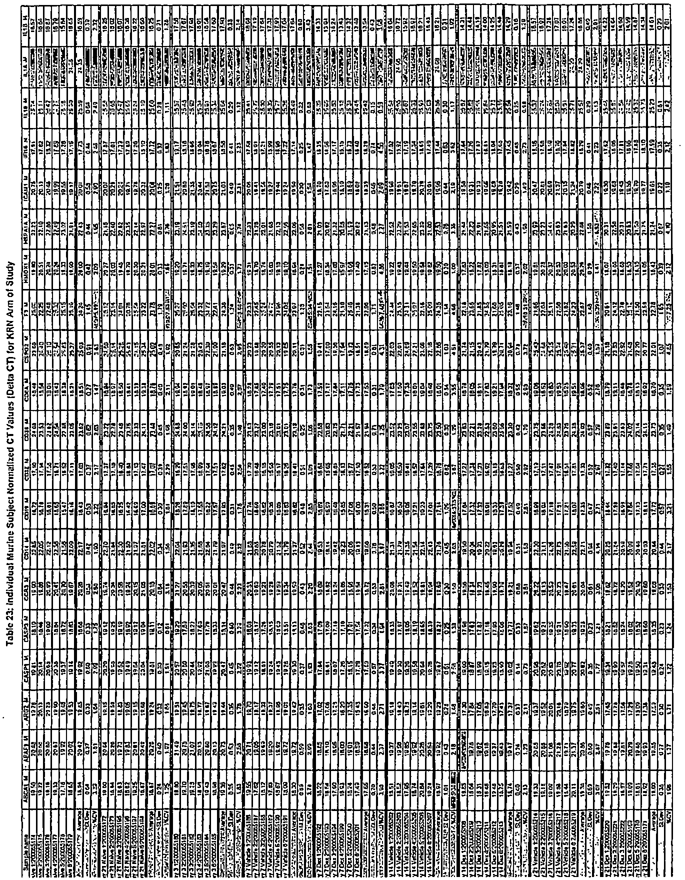

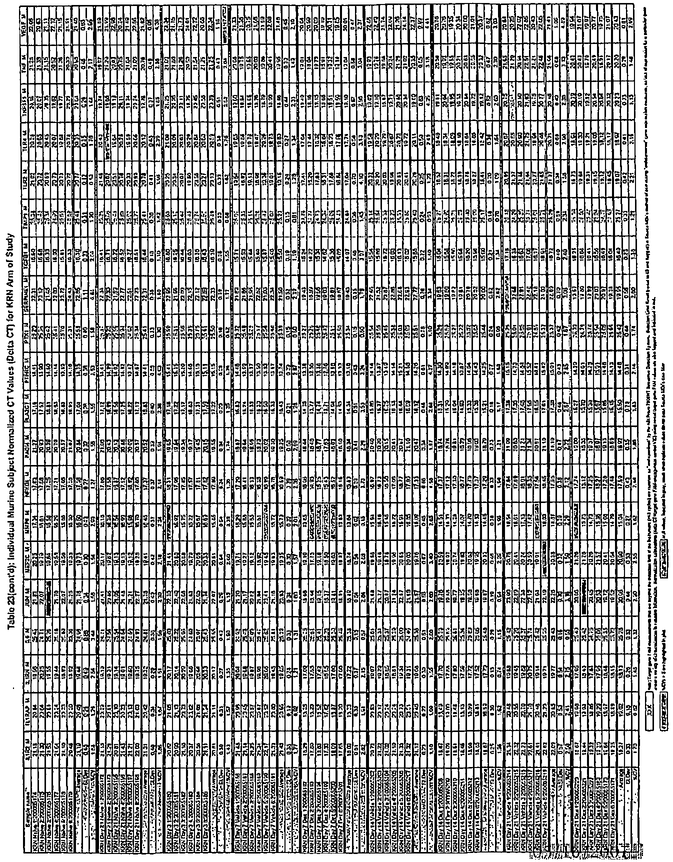

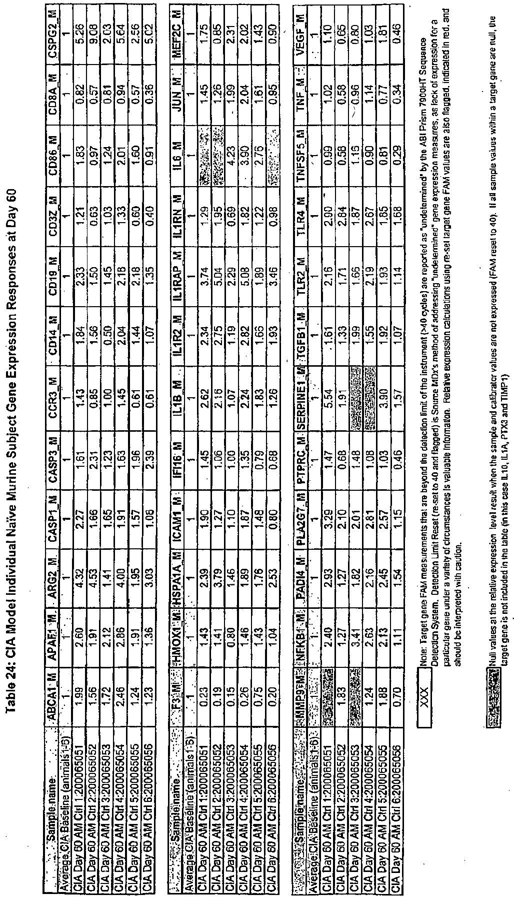

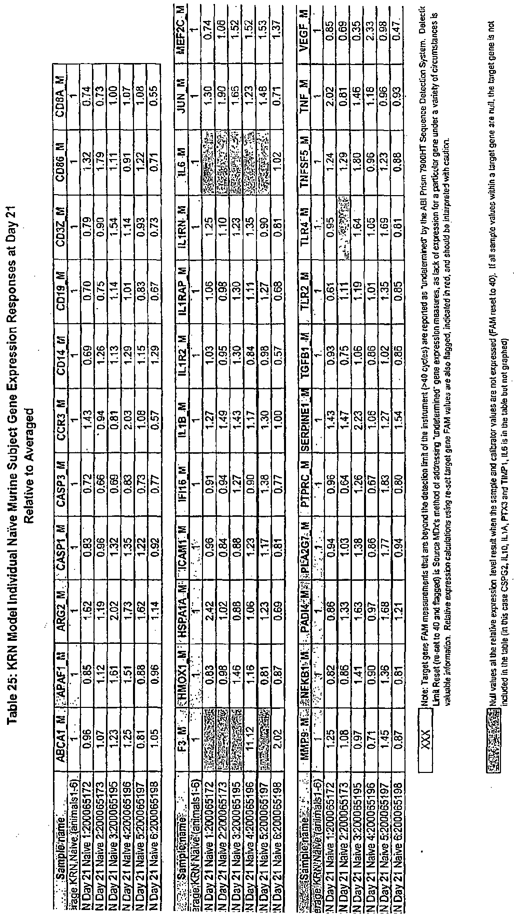

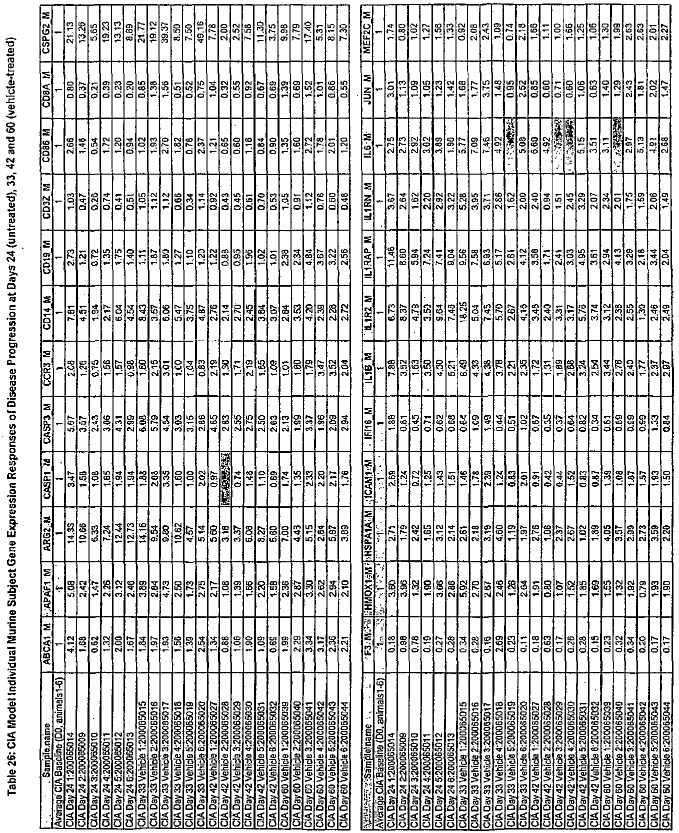

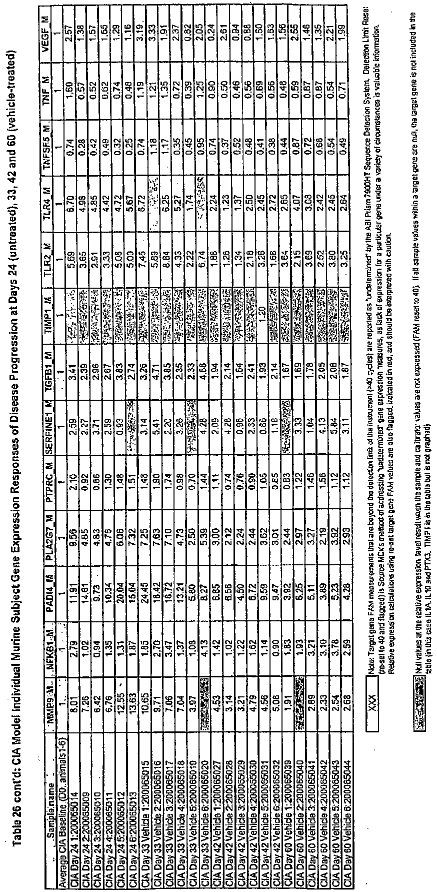

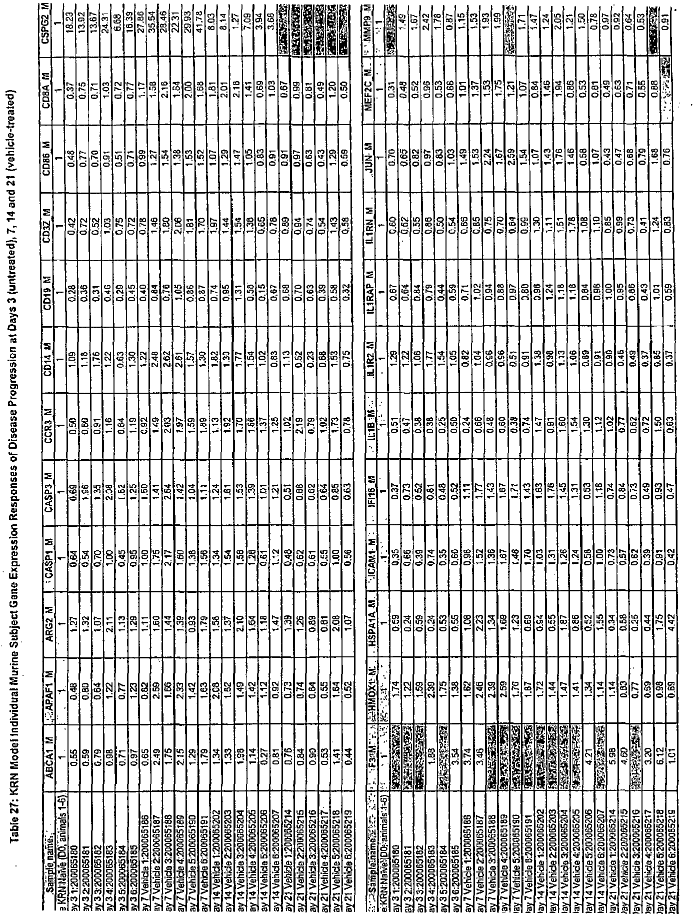

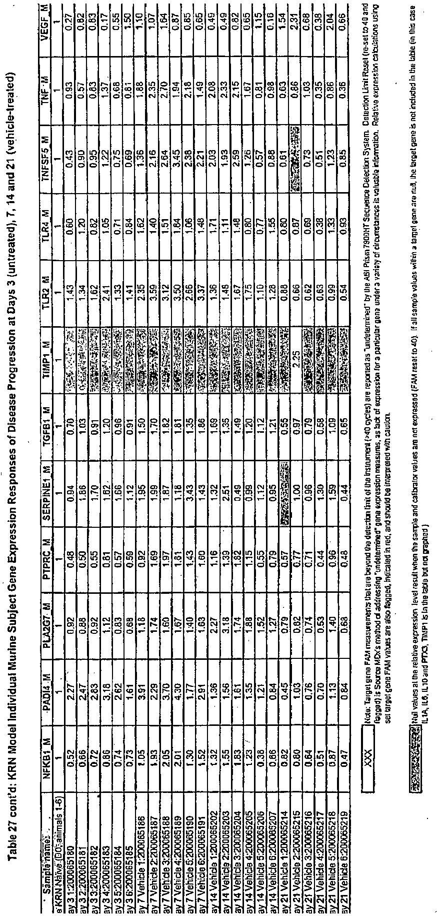

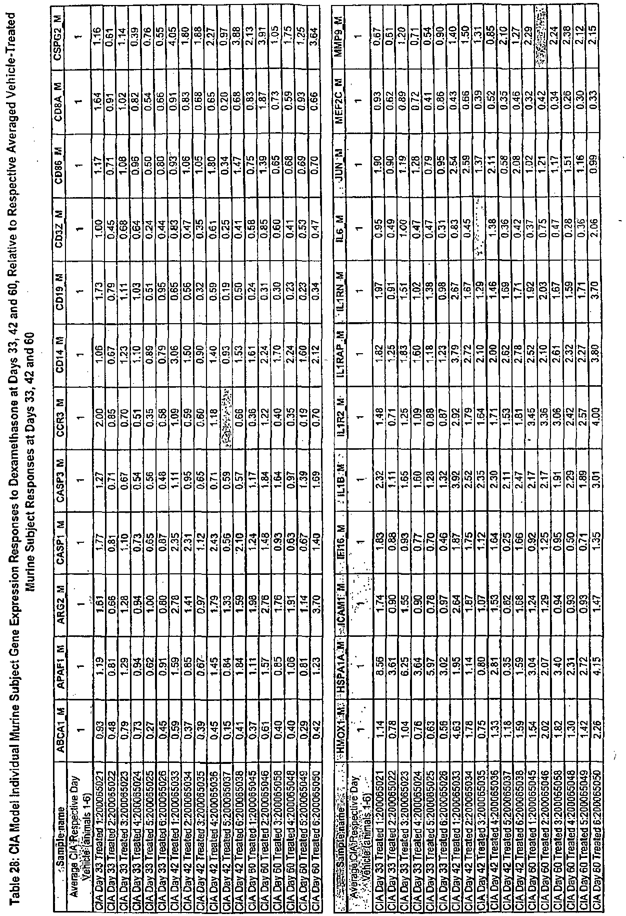

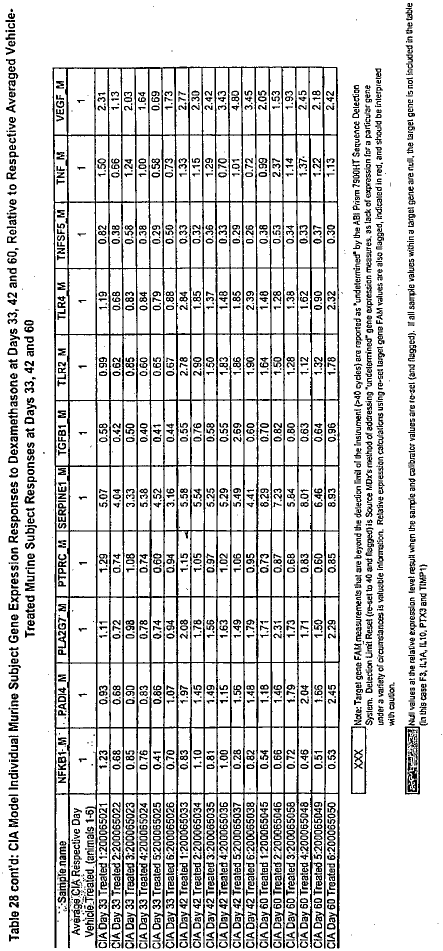

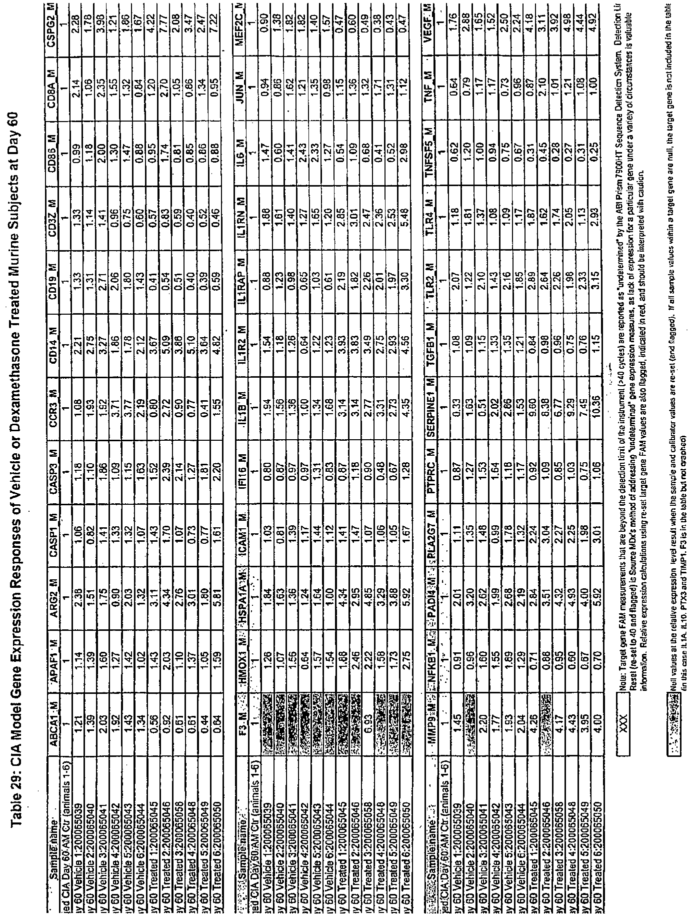

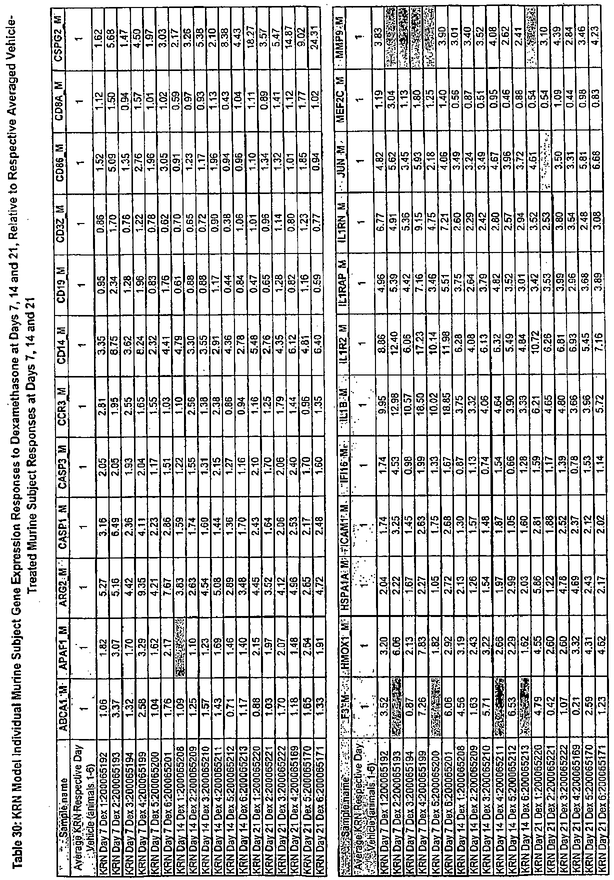

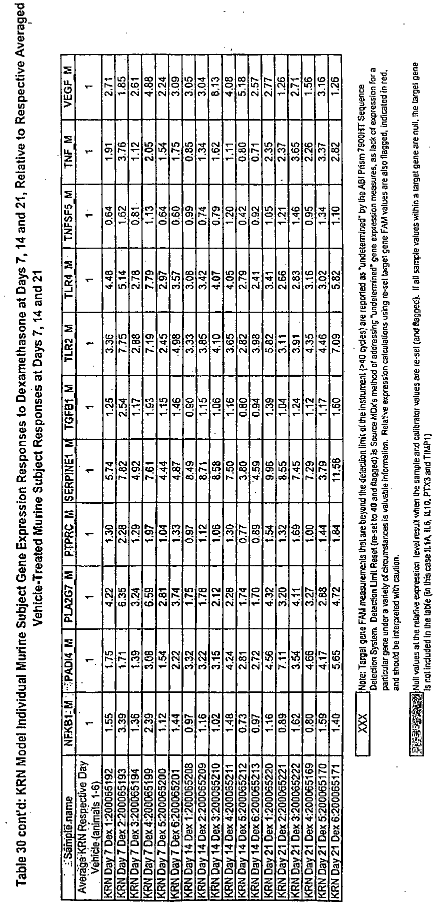

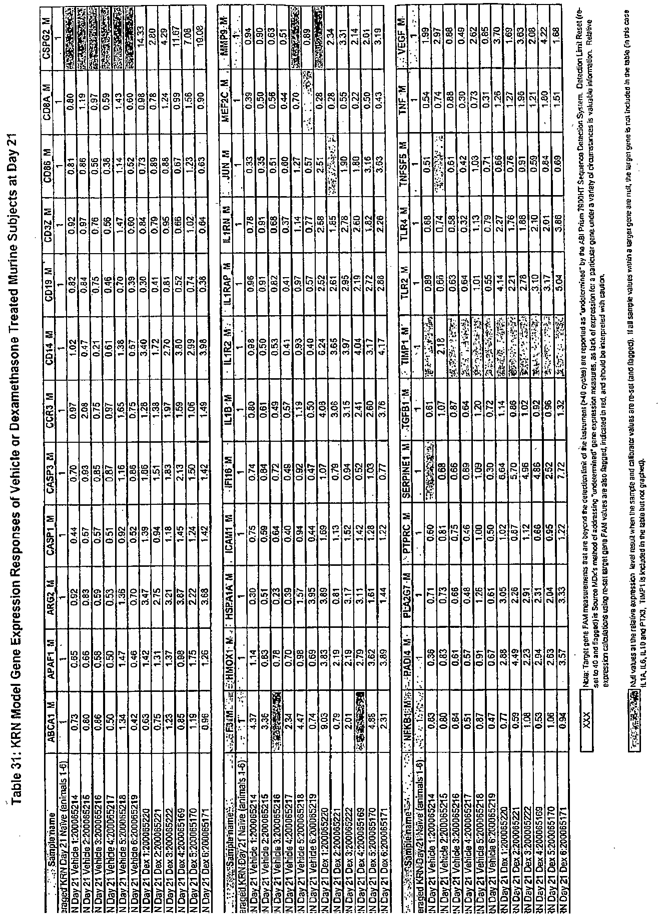

## Claims

1. A method of identifying a rodent Signature gene expression panel for use in assessment of an agent on a human biological condition of interest, the method comprising:
identifying a Gene Expression Panel for humans with respect to which constituent expression levels are indicative of the biological condition of interest;
assessing in a rodent population the constituent genes of the identified Gene Expression Panel to determine which constituents exhibit characteristics analogous to gene expressions in humans, when measured under conditions that are substantially repeatable, and therefore are indicative of the biological condition of interest in both humans and rodents, wherein a set of constituents thus determined to be indicative constitutes the Signature panel and wherein the Signature Panel consisting of a plurality of constituents from any of Tables 3-9.

2. A method for assessing the effect of an agent on a human biological condition of interest, based on a sample from a rodent subject to which the agent has been administered, the sample providing a source of RNAs, the method comprising:
determining a Signature Panel for rodents, the constituents of which correspond to constituents of a human gene expression panel and which exhibit characteristics analogous to gene expressions in humans, when measured under conditions that are substantially repeatable, and the Signature Panel consisting of a plurality of constituents from any of Tables 3-9, wherein measurement of the constituents of the Signature Panel enables measurement of the biological condition of the rodent subject, and measurement of the constituents of the human panel enables measurement of the human biological condition;
deriving from the rodent sample a first profile data set including a plurality of members, each member being a quantitative measure of the amount of a distinct RNA constituent in the Signature Panel; and
producing a calibrated profile data set for the Signature Panel, wherein each member of the calibrated profile data set is a function of a corresponding member of the first profile data set and a corresponding member of a rodent baseline profile data set for the Signature Panel, wherein each member of the rodent baseline data set is a normative measure, determined with respect to a relevant population of rodents, of the amount of one of the constituents in the Signature Panel, the calibrated profile data set providing an assessment of the effect of the agent on the human biological condition,
wherein the measures for each constituent are performed under measurement conditions that are substantially repeatable.

3. A method for assessing the effect of an agent on a human biological condition of interest according to claim 2, wherein amplification is used to measure the amount of RNA of all of the constituents of the Signature Panel, and the efficiencies of amplification for all constituents are substantially similar.

4. A method for assessing the effect of an agent on a human biological condition according to claim 2, wherein the biological condition is arthritis.

5. The method according to claim 4 wherein, the biological condition is rheumatoid arthritis.

6. A method for assessing the effect of an agent on a human biological condition of interest according to claim 2, wherein the Signature Panel comprises a plurality of constituents selected from the group consisting of CASP3, CD14, CSPG2, HSPA1A, ICAM1, IL1B, IL1RN, MEF2C, MMP9, SERPINE1, TGFB1, and TLR2.

7. The method according to claim 2, wherein measurement conditions are repeatable such that measure for each constituent has a coefficient of variation, on repeated derivation of such measure from the sample, that is less than approximately 10%.

8. The method according to claim 2, wherein measurement conditions are repeatable such that measure for each constituent has a coefficient of variation, on repeated derivation of such measure from the sample, that is less than approximately 5%.

9. The method according to claim 2, wherein measurement conditions are repeatable such that measure for each constituent has a coefficient of variation, on repeated derivation of such measure from the sample, that is less than approximately 2%.

10. The method according to claim 3, wherein efficiencies of amplification, expressed as a percent, for all constituents differ by no more than 10%, preferably by no more than 5%, more preferably by no more than 3%, even more preferably by no more than 1%.

11. A rodent Signature Gene Expression Panel consisting of the constituents CASP3, CD14, CSPG2, HSPA1A, ICAM1, IL1B, IL1RN, MEF2C, MMP9, SERPINE1, TGFB1, and TLR2.

12. Use of a rodent Signature Gene Expression Panel consisting of constituents from any of Table **3-9** in assessing the effect of an agent on a human biological condition.

## Patentansprüche

1. Verfahren zum Identifizieren einer Nagetier-Signaturgenexpressionsgruppe zur Verwendung zur Beurteilung eines Agens auf einen biologischen Zustand von Interesse bei einem Menschen, wobei das Verfahren aufweist:
Identifizieren einer Genexpressionsgruppe für Menschen, bezüglich welcher Bestandteil-Expressionsniveaus den biologischen Zustand von Interesse anzeigen;
Beurteilen, in einer Nagetierpopulation, die Bestandteil-Gene der identifizierten Genexpressionsgruppe, um zu bestimmen, welche Bestandteile zu den Expressionen beim Menschen analoge Eigenschaften zeigen, wenn sie unter Bedingungen gemessen werden, die im Wesentlichen wiederholbar sind, und die daher den biologischen Zustand von Interesse sowohl bei Menschen als auch bei Nagetieren anzeigen, wobei ein Satz von Bestandteilen, der so als anzeigend bestimmt wurde, die Signaturgruppe bildet, und wobei die Signaturgruppe aus einer Mehrzahl von Bestandteilen aus einer der Tabellen 3 bis 9 besteht.

2. Verfahren zum Beurteilen der Wirkung eines Agens auf einen biologischen Zustand von Interesse bei einem Menschen, auf der Basis einer Probe von einem Nagetier-Individuum, dem das Agens verabreicht wurde, wobei die Probe eine Quelle von RNAs bereitstellt, wobei das Verfahren aufweist:
Bestimmen einer Signaturgruppe für Nagetiere, deren Bestandteile Bestandteilen einer menschlichen Genexpressionsgruppe entsprechen, und die zu Genexpressionen beim Menschen analoge Eigenschaften zeigen, wenn sie unter Bedingungen gemessen werden, die im Wesentlichen wiederholbar sind, und wobei die Signaturgruppe aus einer Mehrzahl von Bestandteilen aus einer der Tabellen 3 bis 9 besteht, wobei die Messung der Bestandteile der Signaturgruppe eine Messung des biologischen Zustands des Nagetier-Individuums ermöglicht, und eine Messung der Bestandteile der menschlichen Gruppe eine Messung des biologischen Zustands eines Menschen ermöglicht;
Gewinnen, aus der Nagetier-Probe, eines ersten Profildatensatzes, der eine Mehrzahl von Elementen enthält, wobei jedes Element ein quantitatives Maß für die Menge eines bestimmten RNA-Bestandteils in der Signaturgruppe ist; und
Erzeugen eines kalibrierten Profildatensatzes für die Signaturgruppe, wobei jedes Element des kalibrierten Profildatensatzes eine Funktion eines entsprechenden Elements des ersten Profildatensatzes und eines entsprechenden Elements eines Nagetier-Bezugslinien-Profildatensatzes für die Signaturgruppe ist, wobei jedes Element des Nagetier-Bezugslinien-Datensatzes ein normatives Maß für die Menge eines der Bestandteile in der Signaturgruppe ist, das bezüglich einer relevanten Population von Nagetieren bestimmt wurde, wobei der kalibrierte Profildatensatz eine Beurteilung der Wirkung des Agens auf den biologischen Zustand eines Menschen ergibt,
wobei die Messungen für jeden Bestandteil unter Messbedingungen durchgeführt werden, die im Wesentlichen wiederholbar sind.

3. Verfahren zur Beurteilung der Wirkung eines Agens auf einen biologischen Zustand von Interesse bei einem Menschen nach Anspruch 2, bei der zur Messung der Menge an RNA aller der Bestandteile der Signaturgruppe Amplifikation verwendet wird, und die Effizienz der Amplifikation für alle Bestandteile im Wesentlichen gleich ist.

4. Verfahren zur Beurteilung der Wirkung eines Agens auf einen biologischen Zustand bei einem Menschen nach Anspruch 2, bei dem der biologische Zustand Arthritis ist.

5. Verfahren nach Anspruch 4, bei dem der biologische Zustand rheumatoide Arthritis ist.

6. Verfahren zur Beurteilung der Wirkung eines Agens auf einen biologischen Zustand von Interesse bei einem Menschen nach Anspruch 2, bei dem die Signaturgruppe eine Mehrzahl von Bestandteilen aufweist, die ausgewählt werden aus der Gruppe, die aus CASP3, DC14, CSPG2, HSPA1A, ICAM1, IL1B, IL1RN, MEF2C, MMP9, SERPINE1, TGFB1, und TLR2 besteht.

7. Verfahren nach Anspruch 2, bei dem die Messbedingungen wiederholbar sind, so dass eine Messung für jeden Bestandteil, bei wiederholter Gewinnung einer derartigen Messung aus der Probe, einen Variationskoeffizienten hat, der geringer als näherungsweise 10% ist.

8. Verfahren nach Anspruch 2, bei dem die Messbedingungen wiederholbar sind, so dass eine Messung für jeden Bestandteil, bei wiederholter Gewinnung einer derartigen Messung aus der Probe, einen Variationskoeffizienten hat, der geringer als näherungsweise 5% ist.

9. Verfahren nach Anspruch 2, bei dem die Messbedingungen wiederholbar sind, so dass eine Messung für jeden Bestandteil, bei wiederholter Gewinnung einer derartigen Messung aus der Probe, einen Variationskoeffizienten hat, der geringer als näherungsweise 2% ist.

10. Verfahren nach Anspruch 3, bei dem sich die Effizienz der Amplifikation, ausgedrückt als Prozentsatz, für alle Bestandteile um nicht mehr als 10%, bevorzugt um nicht mehr als 5%, bevorzugter um nicht mehr als 3%, noch bevorzugter um nicht mehr als 1%, unterscheidet.

11. Nagetier-Signaturgenexpressionsgruppe, bestehend aus den Bestandteilen CASP3, CD14, CSPG2, HSPA1A, ICAM1, IL1B, IL1RN, MEF2C, MMP9, SERPINE1, TGFB1, und TLR2.

12. Verwendung einer Nagetier-Signaturgenexpressionsgruppe, die aus Bestandteilen aus einer der Tabellen 3 bis 9 besteht, zur Beurteilung der Wirkung eines Agens auf einen biologischen Zustand eines Menschen.

## Revendications

1. Procédé pour identifier un panel d'expression de gènes de signature de rongeur pour utilisation dans la détermination d'un agent sur un état biologique humain présentant un intérêt, lequel procédé comprend :
l'identification d'un panel d'expression de gènes pour humains en rapport avec lequel des niveaux d'expression de constituants sont indicatifs de l'état biologique présentant un intérêt ;
la détermination, dans une population de rongeurs, des gènes constitutifs du panel d'expression de gènes identifié pour déterminer quels constituants présentent des caractéristiques analogues à des expressions de gènes chez les humains, quand on les mesure dans des conditions qui sont sensiblement répétables, et par conséquent sont indicatifs de l'état biologique présentant un intérêt tant chez les humains que chez les rongeurs,
dans lequel un ensemble de constituants ainsi déterminés comme étant indicatifs constitue le panel de signature, et dans lequel le panel de signature est constitué d'une pluralité de constituants de n'importe lesquels des Tableaux 3 à 9.

2. Procédé pour déterminer l'effet d'un agent sur un état biologique humain présentant un intérêt, sur la base d'un échantillon provenant d'un sujet rongeur auquel l'agent a été administré, l'échantillon apportant une source d'ARN, lequel procédé comprend :
la détermination d'un panel de signatures pour rongeurs, dont les constituants correspondent à des constituants d'un panel d'expression de gènes humains et présentent des caractéristiques analogues à des expressions de gènes chez les humains, quand on les mesure dans des conditions qui sont sensiblement répétables, le panel de signature étant constitué d'une pluralité de constituants de n'importe lesquels des Tableaux 3 à 9, la mesure des constituants du panel de signature permettant une mesure de l'état biologique du sujet rongeur, et la mesure des constituants du panel humain permettant une mesure de l'état biologique humain ;
la dérivation, à partir de l'échantillon de rongeurs, d'un premier ensemble de données de profil comprenant une pluralité de membres, chaque membre étant une mesure quantitative de la quantité d'un constituant ARN distinct dans le panel de signature ; et
la production d'un ensemble de données de profil étalonné pour le panel de signature, chaque membre de l'ensemble de données de profil étalonné étant fonction d'un membre correspondant du premier ensemble de données de profil et d'un membre correspondant d'un ensemble de données de profil de base de rongeurs pour le panel de signature, chaque membre de l'ensemble de données de base de rongeurs étant une mesure normative, déterminée par rapport à une population pertinente de rongeurs, de la quantité de l'un des constituants dans le panel de signature, l'ensemble de données de profil étalonné permettant une détermination de l'effet de l'agent sur l'état biologique humain,
dans lequel les mesures pour chaque constituant sont effectuées dans des conditions de mesure qui sont sensiblement répétables.

3. Procédé pour déterminer l'effet d'un agent sur un état biologique humain selon la revendication 2, dans lequel on utilise une amplification pour mesurer la quantité d'ARN de tous les constituants du panel de signature, et les efficacités d'amplification pour tous les constituants sont sensiblement similaires.

4. Procédé pour déterminer l'effet d'un agent sur un état biologique humain selon la revendication 2, dans lequel l'état biologique est l'arthrite.

5. Procédé selon la revendication 4, dans lequel l'état biologique est la polyarthrite rhumatoïde.

6. Procédé pour déterminer l'effet d'un agent sur un état biologique humain selon la revendication 2, dans lequel le panel de signature comprend une pluralité de constituants choisis dans l'ensemble constitué par CASP3, CD14, CSPG2, HSPA1A, ICAM1, IL1B, IL1RN, MEF2C, MMP9, SERPINE1, TGFB1, et TLR2.

7. Procédé selon la revendication 2, dans lequel les conditions de mesure sont répétables au sens où la mesure pour chaque constituant comporte un coefficient de variation, sur une dérivation répétée de cette mesure à partir de l'échantillon, qui est inférieur à environ 10 %.

8. Procédé selon la revendication 2, dans lequel les conditions de mesure sont répétables au sens où la mesure pour chaque constituant comporte un coefficient de variation, sur une dérivation répétée de cette mesure à partir de l'échantillon, qui est inférieur à environ 5 %.

9. Procédé selon la revendication 2, dans lequel les conditions de mesure sont répétables au sens où la mesure pour chaque constituant comporte un coefficient de variation, sur une dérivation répétée de cette mesure à partir de l'échantillon, qui est inférieur à environ 2 %.

10. Procédé selon la revendication 3, dans lequel les efficacités d'amplification, exprimées en pourcentages, pour tous les constituants diffèrent d'au plus 10 %, de préférence d'au plus 5 %, plus préférablement d'au plus 3 %, encore plus préférablement d'au plus 1 %.

11. Panel d'expression de gènes de signature de rongeur constitué des constituants CASP3, CD14, CSPG2, HSPA1A, ICAM1, IL1B, IL1RN, MEF2C, MMP9, SERPINE1, TGFB1, et TLR2.

12. Utilisation d'un panel d'expression de gènes de signature de rongeur constitué de constituants de n'importe lesquels des Tableaux 3 à 9 dans la détermination de l'effet d'un agent sur un état biologique humain.
